Europäisches Patentamt

European Patent Office

Office européen des brevets

(19)

(11) **EP 0 483 143 B2**

(12) NEW EUROPEAN PATENT SPECIFICATION

(45) Date of publication and mention
of the opposition decision:
**09.04.1997 Bulletin 1997/15**

(45) Mention of the grant of the patent:
**01.06.1994 Bulletin 1994/22**

(21) Application number: 89912702.1

(22) Date of filing: 30.10.1989

(51) Int. Cl.[6]: **B01D 63/02**, B01D 63/04,
B01D 63/08

(86) International application number:
PCT/US89/04847

(87) International publication number:
WO 90/05018 (17.05.1990 Gazette 1990/11)

(54) **MEMBRANE AFFINITY APPARATUS AND PURIFICATION METHODS RELATED THERETO**

MEMBRANAFFINITÄTSVORRICHTUNG UND REINIGUNGSVERFAHREN

APPAREIL DE SEPARATION PAR AFFINITE A L'AIDE D'UNE MEMBRANE

(84) Designated Contracting States:
**AT BE CH DE FR GB IT LI LU NL SE**

(30) Priority: 31.10.1988 US 265061
26.10.1989 US 428263

(43) Date of publication of application:
**06.05.1992 Bulletin 1992/19**

(73) Proprietor: **SEPRACOR, INC.**
**Marlborough, MA 01752 (US)**

(72) Inventors:
• **GOFFE, Randal, A.**
**Medway, MA 02053 (US)**
• **ZALE, Stephen, E.**
**Marlborough, MA 01752 (US)**
• **O'CONNOR, James, L.**
**Chelmsford, MA 01842 (US)**

• **KESSLER, Stephen, B.**
**Princeton, MA 01541 (US)**
• **COHEN, Charles, M.**
**Medway, MA 02053 (US)**

(74) Representative: **Hirsch, Marc-Roger**
**Cabinet Hirsch**
**34 rue de Bassano**
**75008 Paris (FR)**

(56) References cited:
EP-A- 249 932       EP-A- 0 095 554
EP-A- 0 173 500     WO-A-88/06899
WO-A-89/05876       JP-A- 6 190 672
US-A- 3 993 560     US-A- 4 474 690

Remarks:
The file contains technical information submitted
after the application was filed and not included in
this specification

EP 0 483 143 B2

**Description**

1.0. Field of the Invention

This invention relates to an apparatus for carrying out affinity separation comprising:

(a) a porous hollow fiber membrane in association with a ligand;
(b) means for enclosing said porous hollow fiber membrane;
(c) means for providing a first fluid in intimate contact with one side of said enclosed porous hollow fiber membrane; and
(d) exit means for directing into a container any fluid present on the side of said enclosed porous hollow fiber membrane opposite that side to which said first fluid is first provided according to the means recited in element (c).

It also concerns a method for carrying out affinity purification of a ligate in a membrane system comprising:

(a) providing a ligate-containing feed liquid to a porous hollow fiber membrane associated with a ligand to said ligate, said feed liquid being under a pressure sufficient to cause a portion of said feed liquid to flow tangentially across an exterior surface of said membrane and the remainder of said fluid being caused to flow into and through said porous membrane whereby said ligate present in said feed liquid binds to said ligand and is thereby separated from said feed liquid;
(b) washing said membrane with a buffer solution;
(c) providing an elution solution to one side of said membrane under a pressure sufficient to cause said elution solution to flow into and through said membrane and to effect the disassociation of any ligate-ligand bonds formed in step (a), whereby any ligate bound to said ligand is eluted with said elution solution; and
(d) separating said ligate in a purified form from said elution solution.

2.0. Background of the Invention

2.1. Affinity Separations

Of the separation technologies available today, those based on affinity interactions are ever more popular - particularly at the laboratory scale. Affinity separation has become the preferred method for purifying proteins and other biomolecules from complex, biologically derived fluids. Affinity Chromatography and Biological Recognition, 1983. I.M. Chaiken, M. Wilchek, and I. Parikh (eds.). Academic Press, New York. Hill, E.A., and Hirtenstein, M.D. 1983. Affinity chromatography: its application to industrial scale processes, pp. 31-66 in Advances in Biotechnological Processes. Alan R. Liss, Inc., New York. The key to the method's attractiveness is its unequaled degree of selectivity.

Affinity separations, as they are currently practiced, typically involve the following steps: A solution containing the compound of interest is passed through a column containing a highly specific ligand immobilized on a solid support. As the fluid passes through the column, the desired component binds selectively - and reversibly - to the ligand; most impurities pass unhindered. Any residual impurities are removed by flushing the column with an appropriate buffer solution. The compound, now purified but still bound, is then recovered by passing through the column a solution that disrupts the ligand-binding interaction - by changing ionic strength or pH, for instance.

Many types of molecules can serve as ligands, including antibodies, antigens, enzyme inhibitors, isolated receptors, and more recently, cloned receptors. Bailon, P., Weber, D.V., Keeney, R.F., Fredericks, J.E., Smith, C., Familletti, P.C., and Smart, J.E. 1987, Receptor-affinity chromatography: a one-step purification for recombinant interleukin-2, Bio/technology 5:1195. In contrast, however, the choice of materials to support the ligand has been limited to either agarose gel beads or silica particles. Although both of these materials are quite suitable for laboratory-scale affinity separations, they do not scale-up well. The intrinsic compressibility of agarose gel beads poses severe limitations for engineering efficient process-scale separation systems. Arnold, F.H., Chalmers, J.J., Saunders, M.S. Croughan, M.S., Blanch, H.W., and Wilke, C.R. 1985; A rational approach to the scale-up of affinity chromatography, pp. 113-122 in Purification of Fermentation Products, D. LeRoith, J. Shiloach, and T.J. Leahy (eds.), American Chemical Society, Washington, D.C.

Compressibility may be more than a limitation: it has even been considered a major liability if used in process-scale affinity systems. Clonis, Y.D. 1987, Large-scale affinity chromatography, Bio/Technology 5:1290. The compression and associated tight packing of an agarose gel bed under typical operating conditions can often seriously compromise the throughput of such systems. Silica supports, which have greater structural rigidity, have provided an alternative to agarose gels. Indeed, silica does minimize compression and allows for the high throughputs necessary for a commercial process system. However, high throughputs are realized only at the expense of high operating pressures. And high operating pressures mean increased costs for capital equipment.

## 2.2. Factors for Process Design

Realizing the intrinsic material constraints of both agarose gel beads and silica particles, design engineers turned instead to manipulating bed geometry to circumvent the through-put/pressure tradeoff.

Efforts to maximize throughput have resulted in a trend towards shorter, wider beds since, for a given pressure drop across the bed, the throughput is inversely proportional to the bed height. A number of column configurations are now on the market: these range from stacked configurations (a number of short beds are connected in series-parallel combinations) to radial flow arrangements (a short, wide bed is curled up end-to-end upon itself).

The "ideal" column geometry would have an infinitely short bed height (to minimize operating pressures and maximize throughput) and an infinite width (to maximize ligand loading and binding capacity). In reality, a substantially isotropic microporous hollow fiber membrane configuration approaches this ideal quite closely with "bed heights" in the 300-$\mu$m range and large internal surface areas.

However, a key parameter in designing processes for affinity separations and one that has been all but overlooked is ligand utilization. Although some scientists use "ligand loading capacity" to measure the efficiency of an affinity column, in reality it is ligand utilization that determines efficiency.

If the requirements of the system are such that an extended residence time is necessary for a high degree of solute/ligand binding, then the only way this extended residence time can be accomplished, without sacrificing throughput, is by increasing the size of the device. A bigger device requires more ligand. This becomes a particularly important consideration when the ligand is relatively expensive (as for a monoclonal antibody or a receptor); the price of the ligand now becomes a substantial portion of overall purification costs. If residence time can be reduced, the ligand will be used at maximum efficiency.

## 2.3. Maximizing Mass Transfer

To maximize ligand use within an affinity device, it is necessary to maximize mass transfer. In packed bed affinity columns, mass transfer is limited by the time it takes for solute molecules to diffuse through the interstitial spaces to the ligand itself which resides on the surface or within the pores of gel beads and silica particles. Assuming rapid binding kinetics, the ligand would be used efficiently only if the average time for a solute molecule to diffuse to the ligand ($t_D$) is substantially shorter than its column residence time ($t_c$) (Relation 1). From this, we can define a form of Peclet number, $P_e$. Kessler, S.B., Zale, S.E., and Bratzler, R.L. in 1987, Affinity device designs for process scale applications, presented at the Society for Industrial Microbiology Annual Meeting, Baltimore, MD. Boucher, D.F. and Alves, G.E. 1959, Dimensionless numbers, Chem. Eng. Progress 55:55:

$$t_D \ll t_c \qquad\qquad \text{Relation 1}$$

$$P_e = t_D/t_c = (L_D{}^2/D)/(V_o/Q) \qquad\qquad \text{Equation 1}$$

Equation 1 (where D is the diffusion coefficient of the solute of interest) can be used to indicate mass transfer efficiency; the $P_e$ values for high efficiencies are significantly less than unity.

Mass transfer efficiency is highly dependent upon diffusional path length ($L_D$). In a packed bed affinity column, this value is necessarily determined by the mean particle radius, hence the trend towards reduced particle size in conventional affinity supports. Once again, however, there is a trade-off. Improved mass transfer is attained only at the expense of elevated operating pressures.

Membrane-based separation systems, however, largely alleviate the mass transfer limitations seen with conventional technology. Because solute molecules are convected through the membrane support past the ligand, rather than having to diffuse to a bead or particle to reach the ligand, diffusional path lengths are minimized and mass transfer efficiency increases significantly. However, even in the case of membranes there is a conflict between high capacity and good mass transfer characteristics which necessitate careful membrane design.

In U.S. Patent 4,163,714 issued August 7, 1979, there is described a pressure driven membrane affinity system. In this system the assorted ligate is both absorbed onto and disassociated from the membrane with the feed liquid, wash and elution solution all being provided to the same surface of the membrane. This process, however, results in the ligate being gradually associated in a somewhat inefficient manner, thereby making it difficult to separate the ligate from the elution solution.

If the rate of ligand/solute association in a particular affinity separation happens to be slower than the rate of solute diffusion, it is conceivable that improving the diffusion characteristics would have no effect on the process efficiency. If one examines the association kinetics of a Sepharose-bound antigen ligand as a model, it is evident that the characteristic reaction time for antibody/antigen association ($t_R$) depends on the concentration of binding sites within the Sepharose bead ($q_m$). The reaction time can be calculated using Equation 2, where $k_1$ is the second order association rate constant and h is the fractional saturation of the adsorbent.

$$t_R = 1/k_1 \cdot q_m (1 - \theta) \qquad \text{Equation 2}$$

Olsen and Yarmush (Olson, W.C., and Yarmush, M.L. 1987, _Electrophoretic elution from monoclonal antibody immunoadsorbents: A theoretical and experimental investigation of controlling parameters_, Biotech. Progress 3:177) have calculated that bovine serum albumin (BSA) binds to monoclonal antibody-coated Sepharose beads on the order of five seconds. Conversely, the average time required for BSA to diffuse to the bead's surface is much longer - 41 seconds. This time is given by $L_D^2/D$ (see Equation 1), where $L_D$ is the mean bead radius ($5 \times 10^{-3}$ cm) and D is the diffusion coefficient for BSA ($6 \times 10^{-7}$ cm$^2$/s). The average diffusion time is almost an order of magnitude greater than that for solute/ligand association. Thus, conventional affinity separations are diffusion-limited for this system: for large-scale processes, this is inefficient.

### 2.4. Membrane-Based Affinity Supports

In the search for superior affinity substrates it was inevitable that membranes would emerge as an alternative to packed columns. Until now, however, the advancement of membrane-based affinity purification technology has been limited by the availability of systematically designed membranes for this purpose. Consequently, conventional flat sheet filters have been investigated for use as affinity separators by Gregor (U.S.-A-4,163,714) and Degan et al. (U.S.-A-4,693,985).

Gregor (U.S.-A-4,163,714) claims ultrafilters (UF) with an average pore size of about 15 to 200 Angstrom. Current technology for producing UF membranes invariably creates a skinned anisotropic structure. While the relatively small pore size is advantageous due to a high internal surface area, protein sieving during the binding (or loading) step in an affinity process would severely limit the utility of such membranes. Fouling and plugging of the membrane occurs very soon after a pressure is applied to the ligate containing fluid.

One method which has been employed in cleaning and unplugging ultrafilters is backflushing (or back-washing). This is a process whereby filtrate is forced back across the filter in the reverse direction. After a few years of using backflushing the frequency of its use as dramatically declined in recent years, due in part to catastrophic membrane failure linked with this practice. Backflushing (often at higher pressures than those at which the UF step was carried out), frequently results in:

- delamination of the skin region of the membrane from the matrix region;
- ruptures (i.e. splits and tears) in the entire membrane wall;
- complete and irreversible plugging, as trapped material is unable to pass through the anisotropic matrix in the reverse direction through pores of ever decreasing size.

Hollow fiber membranes are particularly susceptible to damage during backflushing (and the stresses of start-up/shut-down cycles) because they are self supporting. Flat sheet membranes usually have porous (hydrophobic) backing material to provide mechanical support.

U.S.-A-4,693,985 describes flat sheet polyamide membranes for affinity applications. These are microporous, skinless membranes with pore diameter of >0.1 to <0.45 μm. They represent a major improvement over UF flat sheet membranes, but are still limited by being difficult to configure in a device (as they are not self supporting). Also, high surface area/low dead volume devices become increasingly difficult to design. In U.S.-A-4,693,985 pleated filter is described as the best available construct as process scale increases. In U.S.-A-4,693,985 the configuration for packaging flat sheet membrane is a high-surface-area configuration.

The ideal membrane configuration for downstream processing is a hollow fiber for the following reasons:

- the high surface-area-to-dead volume which can be achieved per device; and
- there are fluid management advantages when feed streams are delivered down the lumen of a fiber.

Stimpson, D.E. ACS Polymer Preprints, Vol. 27, No. 2, p. 424 (Sept. 1986) studied anisotropic hollow fiber membranes over a wide pore size range as affinity supports and concluded that pores in the size range of about 0.1 mm are preferred for achieving the best compromise between surface area and pore size.

WO-A-8806899 discloses an apparatus for carrying out blood purification and concerns a device for simultaneous diffusion hemodialysis and enzyme catalysed conversion of heparine or low molecular weight derivatives thereof, in whole blood using immobilized enzymes such as heparinase. In a preferred embodiment, the enzyme is immobilized on the lumen exterior surface of cellulose hollow fibers. The method and apparatus of this patent employ transmembrane diffusion of low molecular weight components present in whole blood.

## 2.5. Prior Methods For The Production of Microporous Membrane

In the specialized area of membrane art, the current methods for producing microporous membranes generally result in skinned anisotropic structures characterized by wide variations in pore sizes from the outer to the inner portion of the membrane. In particular, the production of isotropic hollow fiber membranes has been hampered by prevailing biases in the art and by existing extrusion methods, over and above the general manufacturing techniques.

In the first place, materials or polymers used for manufacturing membranes have generally been classified, as already stated above, into two general groups: reactive or hydrophilic versus inert (See, for example, Cabasso, I. in "Membranes," Encyclopedia of Polymer Science and Eng. 1987, 9, 509-579, by Wiley Interscience Publication; Kesting R.E., Synthetic Polymeric Membranes 1985, 2d Ed., Wiley; Pusch, W. and Walch, A., Angew. Chem. Int. Ed. Engl. 1982, 21(9), 660-685). Examples of the former group are either intrinsically hydrophilic or can be readily modified to achieve hydrophilicity. High hydrophilicity minimizes the nonspecific binding of proteins to the polymer surface. The main drawback with intrinsically hydrophilic membranes, especially those made from materials such as cellulose, is their limited mechanical and thermal properties. On the other hand, membranes belonging to the latter "inert" group, while possessing superior physical, thermal and chemical resistance properties, are extremely hydrophobic and are thus prone to nonspecific binding of proteins and membrane fouling or plugging.

As a reactive/hydrophilic membrane material, cellulose has been widely used in many of its forms but has some severe drawbacks. These drawbacks include limited pH and chemical resistance (e.g., to chlorine-containing sanitizing agents) and a general lack of requisite physical properties in many applications (See, Kesting, Syn. Polym. Memb., supra). Polysulfone (PS) is the most widely used polymer type in ultrafiltration (UF) membranes by virtue of its relative versatility, both in terms of physical/chemical properties and processability to produce a wide variety of structures and pore sizes (i.e., with molecular weight cutoffs, MWc, from about 2,000 kD to about 1 kD).

Polysulfones have only become an important polymer for the construction of microfiltration (MF) membranes in recent years. Such MF membranes are becoming more numerous in flat sheet form but are still fairly rare in hollow fiber form. Generally speaking, however, polysulfone membranes tend to foul readily and methods for covalently modifying these membrane surfaces have not been developed. Furthermore, these membranes are invariably of the anisotropic variety.

Traditionally, workers in the art have to take into account the pore size range of interest in selecting the membrane polymer. It is believed that certain polymers are more readily processed to make membranes in certain pore-size ranges than others (See, Kesting, Syn. Polym. Memb., supra). Workers in the field such as Strathmann, et al., Desalination 1977, 21, 241-255 and Desalination 1975, 16, 179-203; Tanny, et al., J. Appl. Polym. Sci. 1974, 18, 2149-2163; Koenhen, D.M., et al., J. App. Polym. Sci. 1977, 21, 199-215; Broens, L., et al., Desalination 1977, 22, 205-219; Altena, F.W. and Smolders, C.A., J. Polym. Sci.: Polymer Symposium 1981, 69, 1-10; Broens, L., et al., Desalination 1980, 32, 33-45; Bokhorst, H. et al., Desalination 1981, 38, 349-360; Wijmans, J.G., et al., J. Memb. Sci. 1983, 14, 263-274; and Kesting have headed efforts toward a greater understanding of the mechanism of membrane formation and the ways of manipulating structural properties. It has generally been accepted in the field of membrane processing that many key manufacturing parameters have to be changed and tediously reoptimized in going from a flat sheet formulation to a hollow fiber product. Progress, has thus been slow, particularly with respect to the production of isotropic microporous hollow fiber membranes.

Others have pursued the use of blends consisting of hydrophilic and hydrophobic polymers as dopes for preparing membranes (See, Cabasso, I. Encyclopedia of Polymer Science and Eng., supra; Pusch, W. and Walch, A., Angew. Chem. Int. Ed. Engl., supra). Their primary goal has been to use hydrophilic polymers as processing aids; i.e., the hydrophilic polymers are used to increase the viscosity of the dope. Extraction steps used to remove the hydrophilic component, during and after the coagulation process, enhances both the pore density and range of pore sizes attainable. Consequently, these references generally avoid very high molecular weight hydrophilic polymers as blend components because these polymers have a greater tendency to be entrapped in the membrane matrix. Furthermore, the membrane technology literature teaches that, as the molecular weight of a hydrophilic additive increases, especially to the 100,000 range and above, the pore size obtained in the final membrane decreases dramatically (See, Cabasso, I. et al., J. Appl. Polym. Sci. 1976, 20, 2377-2394; Nguyen, Q.T. et al., J. Mem Sci. 1985, 22, 245-255). For example, Cabasso has shown that by increasing the molecular weight (MW) of polyvinylpyrrolidone (PVP) from 10,000 to 40,000 in an experimental PS/PVP blend, the water permeability (Lp) of the resulting hollow fiber is reduced by a factor of five. Furthermore, the initial modulus and tensile strength also suffer. These results suggest that the PVP is retained in the final membrane as the molecular weight is increased. Apparently, the phenomenon of chain entanglement becomes more important as the molecular weight of a water-soluble polymer additive in the blend increases. Thus the high molecular weight additive is less readily extracted and the density of the final membrane increases, preventing the easy passage of water.

A hollow fiber manufacturing process has also been described (See, Cabasso, I. et al., J. Appl. Polym. Sci. supra) which employs optically clear (i.e., single phase) dopes made from PS blended with PVP or polyethylene glycol (PEG) (MW = 600) dissolved in either dimethylformamide or dimethylacetamide. This reference emphasizes that these dopes do not exhibit any cloud point behavior, not even, the typical upper critical solution temperature (UCST) observed when nonsolvent is titrated into a polymer/solvent mixture. Instead, these clear dopes, when contacted with nonsolvent, become phase separated with the inward diffusion of nonsolvent. Researchers speculate that the size of the resulting solvent/PVP-rich domains are probably dictated by the thermodynamic phase relationships and by the kinetics of the phase separation (See, Cabasso, I. et al., J. Appl. Polym. Sci. 1977, 21, 165-180). These observations are essentially consistent with the findings reported by others (See, Kesting, Syn. Polym. Memb., supra; Kamide, K. and Manabe, S., Material Science of Synthetic Membranes, ACS Symposium Series 1985, 269 197-228, Lloyd, D.R., Ed.).

Where very high molecular weight hydrophilic polymers (e.g., poly(ethylene oxide) (PEO), at 4 to 5 million MW) have been blended with polysulfone-type polymers, the intent has been to take advantage of the compatibility between the blended polymers and to retain the hydrophilic polymer in a homogeneously blended transparent film (See, U.S.-A-4,387,187 and EP-A-37,181 to Newton and assigned to Imperial Chemical Industries, Ltd.). As disclosed in U.S. Patent No. 4,387,187, such PES/PEO films or semipermeable membranes are prepared mainly by solvent evaporation, with a leaching step to remove remaining solvent from the already formed film. The degree of porosity attained in such a dense membrane is expected to exclude the permeation of molecules much larger than about 1,000 molecular weight. However, due to the retention of PEO in the final structure there would be some hydrophilicity imparted to the membrane.

When using hydrophilic polymers as processing aids the limitation encountered is that of compatibility. In general, low molecular weight polymers can be loaded into dopes at much higher concentrations (See, Japanese Patent No. 57,035,906). This Japanese patent teaches one how to achieve the maximum possible loading of PEG into a homogeneous dope for membrane casting as a function of molecular weight. PEG molecules above 100,000 MW is specifically excluded.

Similarly, Klein and Smith (U.S.-A-4,051,300) teach the use of low molecular weight PVP (average molecular weight of at least 2,000) in a blend solution with polysulfone to achieve high dope viscosities for hollow fiber manufacture. The weight ratio of polysulfone to PVP is specified to be no less than 0.5 and no greater than 55. Thus, the relatively low molecular weight hydrophilic polymer additive (so-called, "non-solvent" by Klein and Smith) is used in sufficient quantities to serve as a processing aid. The amount of processing aid is restricted to ensure that:

(i) the dope does not exhibit a phase boundary under normal process conditions, e.g., temperature (Cabasso, I. et al., J. Appl. Polym. Sci. supra); and
(ii) PVP is not retained in the hollow fiber to reduce either the void volume (or rather the porosity) of the final membrane, or the hydraulic permeability (Lp) and pore size.

Membrane forming PS/PVP dopes of Cabasso (Cabasso, I. et al., J. Appl. Polym. Sci. supra) and Klein and Smith (U.S.-A-4,051,300) require contact with a nonsolvent in either the vapor or liquid phase to undergo phase separation. Under these circumstances, the relative rates of diffusion of nonsolvent into the dope and solvent out of the dope control the process of phase separation in these systems. The nonsolvent employed to induce phase separation causes precipitation of the polysulfone while dissolving and extracting the PVP from the polysulfone fiber as it is being formed.

Relying solely on quenching a dope solution with a nonsolvent frequently gives rise to the formation of a skinned highly anisotropic structure due in part to the limitations imposed by inefficient or slow mass transfer. Greater rates of diffusion of nonsolvent molecules through the dope composition may sometimes be achieved by dissolving smaller amounts of solids in the dope. Quenching these low solids dopes in solvent/non-solvent mixtures helps to overcome skin formation and anisotropy. However, the resulting membranes, though more isotropic, are frequently weak and are not self-supporting. This slow diffusion process of the prior art gives smaller pores near the membrane surface which first comes into contact with the nonsolvent and progressively larger pores deeper into the membrane matrix.

In contrast to the nonsolvent induced liquid/solid phase separation for preparing essentially anisotropic microporous membranes, Castro (U.S. Pat. No. 4,247,498) has exploited thermal phase inversion (i.e., liquid/liquid phase separation brought about by temperature changes) in the preparation of isotropic microporous membranes. Thermal phase inversion, as it is currently practiced, requires a polymer melt and a compatible liquid to give a homogeneous solution in which the polymer is solubilized in the poor solvent. Subsequent cooling of these melts results in the precipitation of the polymer. The structure is thus "frozen" by the cooling process.

Different methods for spinning fibers are known in the art. These include dry-wet spinning, in which there exists an air gap between the extrusion device or spinnerette and the quench bath, and wet-jet spinning (See, Cabasso, I. Encyclopedia of Chem. Tech. 3rd Edition, Vol. 12, p. 501). In wet-jet spinning the spinnerette is submerged in the quench bath so that there is a zero air gap. When powerful solvents are employed in the quench bath the plasticizing effect on newly formed fiber can limit spinnability.

2.6. Spinnerette Assemblies Of The Prior Art

Present spinnerette assemblies for hollow fiber manufacturing, are wholly inadequate and inflexible for the production of substantially isotropic microporous membranes. The extrusion dies currently in use do not provide the degree of control over the pore structure and pore-size distribution of the resulting microporous hollow fibers that one would wish to have. Typical tube-in-orifice spinnerettes are described in U.S. Patents Nos. 4,198,363 (Noel, G. et al.) and 4,229,154 (Chaban and Hawkins); in Borneman, Z. et al. Proceedings, 4th British Oxygen Company Conference, Sept. 1986, p. 145-157; and in Aptel, P. et al. J. Memb. Sci. 1985, 22, 199-215. Spinnerette face plate configurations are further disclosed in an article by Cabasso in "Hollow Fiber Membranes, "Encyclopedia of Chem. Tech., 3rd Edition, Vol. 12, p. 499, Kirth-Othmer, Eds.

Numerous other spinnerette assemblies or extrusion dies are described elsewhere. Among these are U.S. Patent Nos. 4,370,114 (for the production of multi-cored filaments), 1,541,528 (a device for extruding tubing, not hollow fibers), 2,574,555 (double-annular face plate but apparently no central hollow bore), and 3,321,803 (die for coating a metallic pipe). Still other devices are disclosed in U.S. Patent No. 3,121,254 (mentions inert gas in hollow bore), 3,357,051 (for extrusion of double-walled tubes), 3,690,806 (device with internal components useful for reverse-flow and adjustable chock applications), and 3,716,317 (device for spinning filaments from two polymer streams).

A very recent spinnerette assembly, described in U.S. Patent No. 4,493,629 (the '629 patent), is a modular unit designed for the co-extrusion of three fluids during hollow fiber manufacture and features a tangential entry port. There are two key factors which make this prior device poorly suited for manufacturing substantially isotropic structures:

(1) The '629 patent describes a spinnerette with only one annulus emerging from the face of the spinnerette (i.e., the surface of the device from which the extruded fiber emerges). Thus, fluids within the body of the device will have a tendency to mix before emerging from the spinnerette as a hollow fiber. The extent of mixing is a function of the relative viscosities and relative flow rates. Therefore, two of the three streams entering the device cannot be varied independent of each other over a significant range of flow.

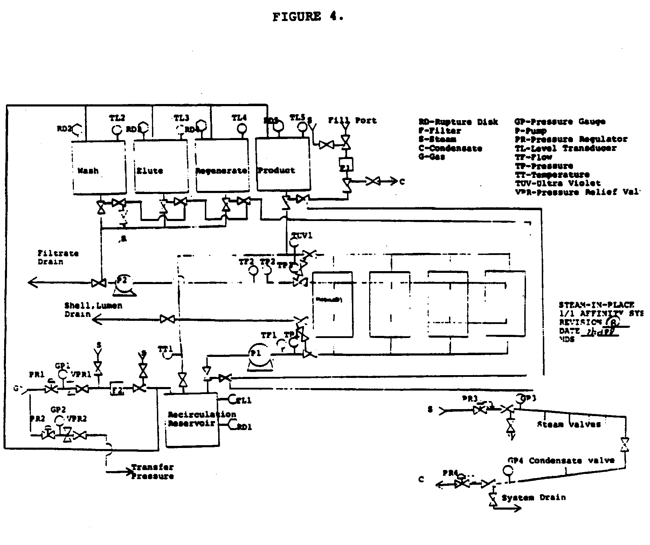

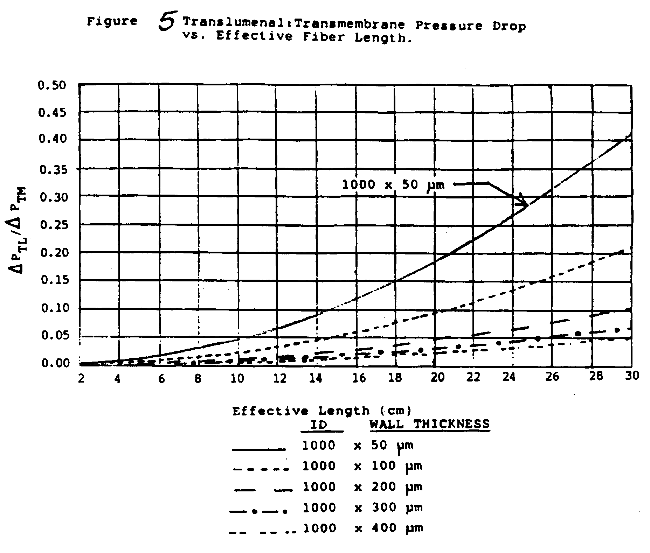

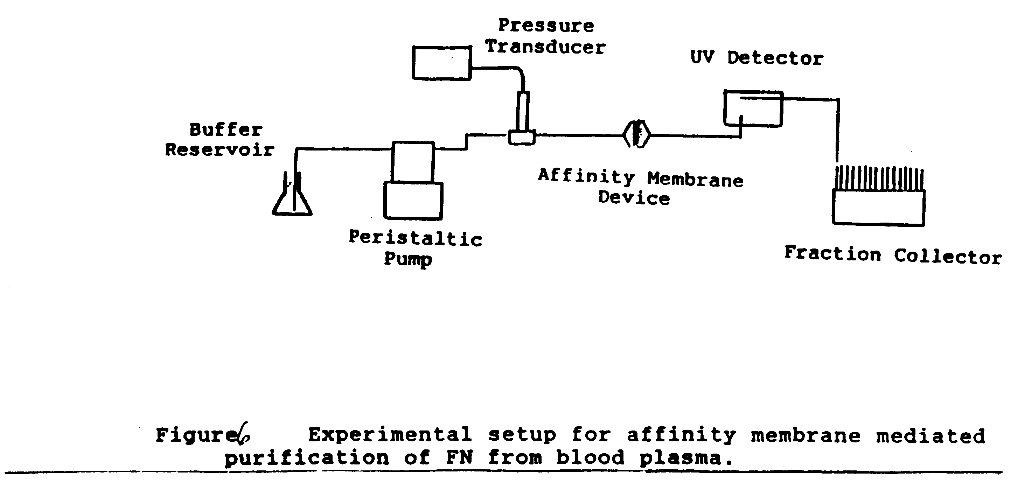

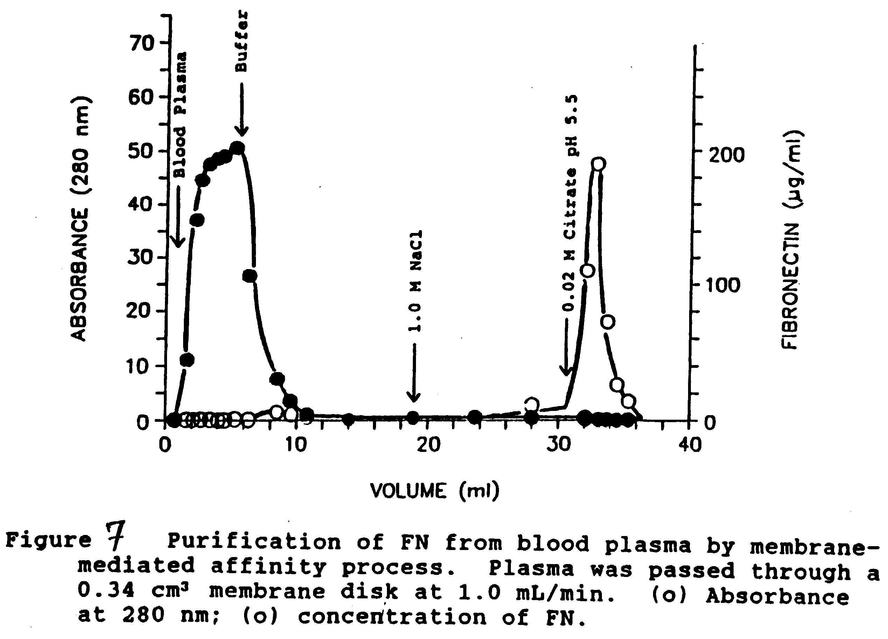

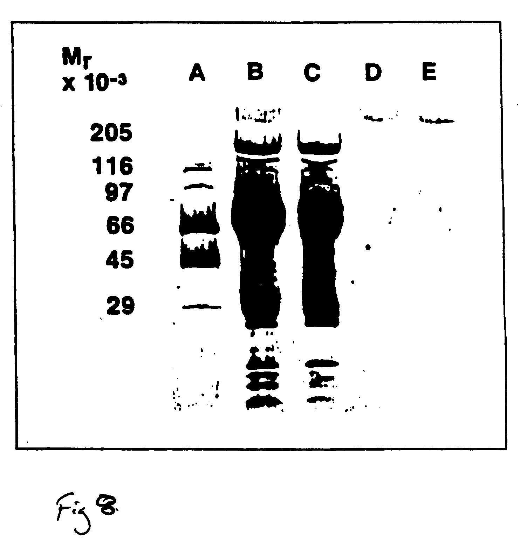

(2) The plurality of ports in the annular spacer described in Figures 4 and 5 of the '629 patent is cumbersome. Moreover, the uniformity of the overall flow, via this divided flow-path, is a direct function of the dimensions of each and every port. Problems arise when delicate control of low, and/or, vastly different flow rates are required. Similarly, difficulties are encountered when there are significant differences in viscosity for the two fluid streams which are forced to emerge through the single annular space.

It is, therefore, an object of the present invention to provide a membrane-based affinity system which improves the efficiencies of mass transfer and ligand use and which is amenable to scale-up.

It is an additional object of the present invention to provide an apparatus which has a processing capability equivalent to that of a conventional, agarose-based affinity system.

It is a further object of this invention to provide an apparatus whose high throughput flow rate and efficient ligand use permit rapid bind/elute cycle times.

It is also an object of this invention to provide a membrane apparatus whose small volume and preferred mode of elution permits product concentration.

3.0. Summary of the Invention

The apparatus of this invention is a cross flow, hollow fiber affinity membrane system for separation of high value products, such as therapeutic proteins. The central feature of the system is a substantially isotropic microporous hollow fiber membrane designed so as to optimize loading capacity and low dead volume while achieving high mass transfer rates. A large scale system having one 600 ml module is designed to process up to 10,000 liters of cell cultures harvests per week. Multiple affinity systems or modules can be run in parallel in order to process even larger quantities of feed material.

The adsorption (or loading) is accomplished by recycling the fluid through the fiber lumen at high recycle rates. A fraction (typically 20%) of the recycle flow permeates the hollow fiber membrane. A highly specific ligand is immobilized within the membrane's porous structure. As the fluid passes through the membrane, the desired component i.e. ligate or target molecule binds selectively - and reversibly - to the ligand; most impurities pass unhindered.

After the adsorption of the ligate, the fibers are washed with a buffer solution, to remove all nonbound impurities. This is accomplished by first washing the shell and then flushing backwards through the lumen. The spent wash buffer is routed to drain.

The wash is followed by product elution. A buffer which inhibits or competes with the attraction of the ligand to the product is preferentially, but not limited to being washed through the shell and then backwards through the lumen. Optionally, elution can be accomplished in the same manner as the loading step. The presence of product is indicated by a UV adsorption peak; the product is directed to a holding reservoir.

When the elution is complete, the membrane is regenerated by a third buffer that restores the membrane to the initial conditions. This is also conducted through the shell, followed by flushing backwards through the lumen. The system is now ready to repeat the cycle.

The apparatus is designed to meet criteria such as high volumetric throughput, high reliability, ease of scale-up, high selectivity and high product yield. The high volumetric throughput is accomplished by high filtrate flow rates enabled by the unique low pressure drop characteristic of the membrane process, but more particularly, by the substantially isotropic, $\mu$m pore size, microporous hollow fiber membrane of this invention.

Generally stated, this invention comprises an apparatus for carrying out an affinity separation process such as defined above characterised in that at least part of said fluid leaving from means (d) has originated from said first fluid of means (c), and further characterized in that said membrane has a substantially isotropic microporous structure in all directions throughout the membrane, with pores large enough to permit the convective flow of macromolecule-containing solutions across the membrane.

In another embodiment of the present invention, the apparatus is characterized in that said membrane has a mean pore sized of at least 0.20 $\mu$m.

In a further embodiment of the present invention, the apparatus is characterized in that said membrane is at least about 200 $\mu$m in thickness.

Generally stated, the membrane of this apparatus may have its interfacial surface derivatized by:

(a) contacting said membrane with a solution comprising a nonsolubilizing solvent and a linker moiety capable of covalently bridging said membrane to a ligand, which ligand has a plurality of functional groups and is capable of exhibiting the desired interfacial characteristics, for a length of time sufficient to form a covalent bond between a chain end of the polymer comprising said membrane and said linker moiety to form a pendant linker moiety;

(b) contacting the membrane of step (a) with a solution comprising a nonsolubilizing solvent and said ligand, for a length of time sufficient to form a covalent bond between a functional group of said ligand and said pendant linker moiety, to provide a product membrane with derivatized interfacial characteristics.

The above apparatus may further comprise a second exit means for directing said first fluid into a second container. In addition, said apparatus may further comprise a second means for providing a second fluid into said enclosure means on the opposite side of said membrane than said first fluid is provided. Furthermore, this second means for providing may be a reversible pump capable of withdrawing a fluid or regulating the exit of fluid from said enclosure means. In addition, the exit means of element (d) may be closed. The membrane may further comprise (a) polyethersulfone as the primary hydrophobic polymer component having functionalizable phenolic chain ends; (b) hydroxyalkylcellulose having hydroxyl functional groups; and (c) a linker moiety selected from the group consisting of ethylene glycol diglycidyl ether, 1,4-butanediol diglycidyl ether, epichlorohydrin, and chloroacetic acid, which linker moiety is able to covalently bridge a phenolic chain end of said polyethersulfone with at least one hydroxyl group of said hydroxyalkylcellulose.

Furthermore, this invention comprises the following method for carrying out affinity purification of a ligate in a membrane system:

(a) providing a ligate containing feed liquid to a porous hollow fiber membrane associated with a ligand to said ligate, said feed liquid being under a pressure sufficient to cause a portion of said feed liquid to flow tangentially across an exterior surface of said membrane and to cause the remainder of said fluid to flow into and through said porous membrane whereby said gate present in said feed liquid binds to said ligand and is thereby separated from said feed liquid;

(b) washing said membrane with a buffer solution;

(c) providing an elution solution to one side of said membrane under sufficient pressure to cause said elution solution to flow into and through said membrane and to cause the disassociation of any ligate-ligand bonds formed in step (s) whereby any ligate ligate bound to said ligand is eluted with said elution solution; and

(d) separating said ligate in a purified form from said elution solution,

characterized in that said membrane has a substantially isotropic microporous structure in all directions throughout the membrane with pores large enough to permit the convective flow of macromolecule-containing solutions across the membrane.

In another embodiment of the present invention, the method is characterized in that said elution solution is provided to the opposite side of said membrane than where the feed liquid is provided, under sufficient pressure to cause said elution solution to flow into and through said membrane.

In a further embodiment of the present invention, the method is characterized in that said buffer solution is provided to the opposite side of said membrane than where the feed liquid is provided, under sufficient pressure to cause said solution to flow into and through said membrane.

This invention also relates to the methods being used to carry out affinity purification of Factor VIII with a monoclonal antibody to Factor VIII; fibronectin with porcine gelatin; Factor IX with monoclonal antibody to Factor IX; and IgG with Protein A.

The method may be used with various types of ligands in addition to antibodies, including various natural or synthetic binding proteins.

A preferred ligand or macromolecule useful in the present invention is a single chain multifunctional biosynthetic protein expressed from a single gene derived by recombinant DNA techniques. The protein comprises a biosynthetic antibody binding site (BABS) capable of functioning as a ligand, comprising at least one protein domain capable of binding to a preselected antigenic determinant, a spacer segment, and an effector region. The amino acid sequence of the BABS domain is homologous to at least a portion of the sequence of a variable region of an immunoglobulin molecule capable of binding the preselected antigenic determinant. The effector protein has an amino acid sequence capable of selective covalent binding to a linker moiety, another macromolecule or ligand, or to the derivatized interfacial area of the membrane polymer itself.

In another aspect, this multifunctional biosynthetic protein may have a binding site comprising two domains connected by a polypeptide connector region. The amino acid sequence of each of the domains comprises a set of complementarity determining regions (CDRs) interposed between a set of framework regions (FRs), each of which is respectively homologous with at least a portion of the CDRs and FRs from an immunoglobulin molecule. The two domains together define a hybrid synthetic binding site having specificty for a preselected antigen, determined by the selected CDRs, and mimicking the structure of $F_v$.

The polypeptide connector region comprises plural, peptide-bonded amino acids defining a polypeptide of a length sufficient to span the distance between the C terminal end of one of the domains and N terminal end of the other when the binding protein assumes a conformation suitable for binding. The connector region preferably comprises amino acids which together assume an unstructured polypeptide configuration in aqueous solution. The binding protein is capable of binding to a preselected antigenic site, determined by the collective tertiary structure of the sets of CDRs held in proper conformation by the sets of FRs. The binding protein may have a specificity substantially identical to the binding specificity of the immunoglobulin molecule used as a template for the design of the CDR regions.

In preferred aspects, the polypeptide sequence incorporated as a spacer in the multifunctional protein is disposed between the binding site and the second polypeptide. The preferred polypeptide spacers are cysteine-free and comprise amino acids which together assume an unstructured configuration in aqueous solution. The function of the spacer is to promote unhindered binding activity of the BABS domain after immobilization onto the membrane.

The process and apparatus of the present invention utilize a porous membrane which is associated with a preselected ligand. Such a membrane is manufactured from a suitable polymer and is substantially isotropic in the size distribution of its pores. Moreover the surface characteristics of said membrane is preferably modified to accommodate better the associated ligand. Most preferably, such surface modification process takes advantage of the functionalizable chain ends of the polymer. The process is carried out under heterogeneous conditions preferably on a preformed membrane (e.g., hollow fibers). Thus, by allowing a covalent bond to form between the functionalizable polymer chain end which is available at the polymer surface and a linker molecule which is capable of serving as a covalent bridge, a ligand or a macromolecule, which is capable of altering the interfacial or surface properties of the polymer, may then be introduced over substantially all the interfacial boundaries of the polymer. The process disclosed is particularly effective for the modification of surfaces of articles manufactured from hydrophobic polymers, although materials made from any polymer with functionalizable chain ends are equally susceptible to modification by the same methods.

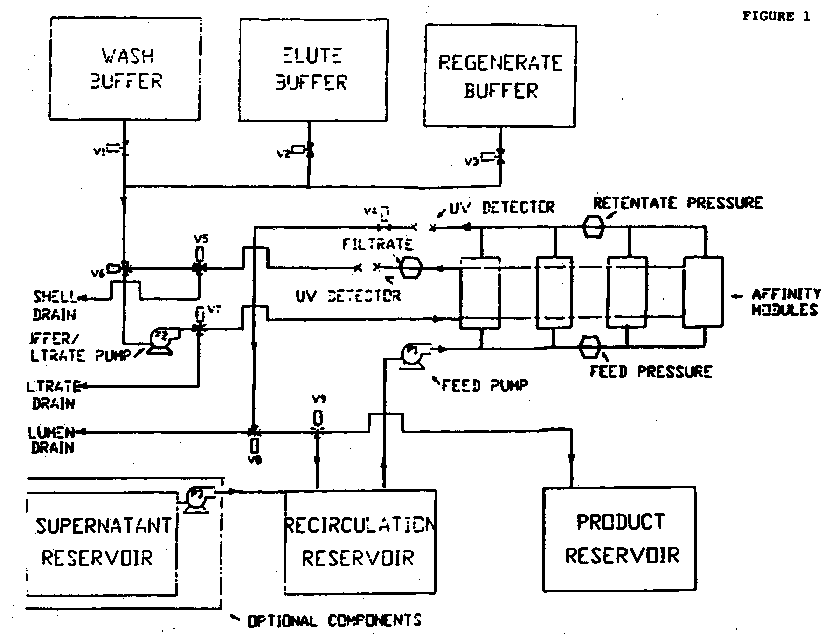

The present invention involves the surface modification of membranes comprised of hydrophobic polymeric materials. Thus a membrane produced from polyethersulfone, a hydrophobic engineering material with desirable processing characteristics and useful bulk properties but which undesirably binds proteins nonspecifically by adsorption, can be derivatized or modified covalently by utilizing the phenolic end group present in each polymer chain. In those cases where the polymer end groups are less reactive, these chain ends may be converted to more reactive functional groups by a suitable reagent (See, for example, the conversion of terminal chloride groups to terminal hydroxyl groups in FIG. 1). A useful proportion of these groups are exposed at the membrane surface and by bringing the membrane in contact with a solution containing a linker moiety, which linker moiety is capable of forming a covalent bond with the polymer chain end group and at least one functional group of a macromolecular or ligand species, the polymer surface is rendered susceptible to modification by the subsequent introduction of said macromolecule or ligand selected for its ability to alter the surface properties of the bulk polymer. Subsequent layers of a variety of macromolecular or ligand species may then be covalently introduced by repeating the overall process although the use of the linker moiety in these additional layers is not always necessary.

A preferred linker moiety is a diepoxide, an epoxyhalide, or a dihalide and the macromolecule or ligand species may be a hydrophilic or hydrophobic synthetic or natural polymeric substance or may even be a low molecular weight compound. Biologically active proteins, polypetides, and polynucleotides may also be covalently bound to the polymer surface in like fashion.

The present invention also describes the unique characteristics of a four-component dope composition which exhibits thermal phase inversion boundaries at a so-called lower critical solution temperature (LCST) as well as at an upper critical solution temperature (UCST). These properties are exploited by a manufacturing process that employs a temperature-regulated nonsolvent quench bath which serves to initiate the temperature-dependent phase inversion phenomenon as well as freezing or precipitating out and preserving the resultant microporous structure.

In conjunction with the procedure disclosed for the production of isotropic microporous hollow fiber membranes, the present invention further describes an improved spinnerette assembly comprised of two independent concentric annuli surrounding a central bore which optionally contains therein a removable hollow pin. This improved spinnerette, which can be maintained at a desired temperature with the aid of means for external heating, is designed to accommodate three separate entry ports for controlling the flow of three separate fluids: namely, a dope composition, an intraannular fluid, and an extraannular fluid. The design of this improved spinnerette is quite simple and economical and has no need for tangential entry ports.

The ability to deliver the extraannular fluid over the outer surface of an extruded hollow fiber permits, among other things, the production of hollow fiber membranes with a substantially isotropic microporous structure in all directions throughout the membrane. As disclosed further below, other membrane structures (e.g., skinned, double-skinned, anisotropic) are also possible by the methods of the present invention.

## 4.0. Brief Description of the Figures

FIG. 1 illustrates the preferred small-scale membrane affinity apparatus operated in a non sterile matter including pump means, valve means, piping means and solutions along with indications of material flow directions through the apparatus.

FIG. 2 illustrates the preferred large-scale membrane affinity apparatus including pump means, valve means, piping means and solutions along with indications of material flow directions through the apparatus.

FIG. 3. illustrates membrane-mediated affinity separation steps where the membrane is in the form of a hollow-fiber membrane.

FIG. 4. large scale system operated in a sterile manner where system is under positive pressure at all times, buffers are transported into reservoirs through sterilizing filters and apparatus can be steamed in place sterilized prior to operation.

FIG. 5. illustrates calculated ratios of translumenal-to-transmembrane pressure drops as a function of effective fiber length.

FIG. 6. illustrates an experimental apparatus used in the flat-sheet affinity membrane purification of fibronectin from blood plasma and determination of capture efficiency of human IgG from a dilute solution.

FIG. 7. illustrates results of the purification of fibronectin from blood plasma with a flat-sheet affinity membrane.

FIG. 8. shows an SDS PAGE gel used in determination of fibronectin purity and extend of depletion from affinity membrane-treated blood plasma.

FIG. 9. shows experimental results of cycling a protein-A activated hollow-fiber affinity membrane module used in the purification of a murine monoclonal antibody from a cell culture supernatant.

FIG. 10. shows a phase diagram for a four component PES/PE dope composition.

FIG. 11. shows a schematic diagram of a spinnerette assembly.

FIG. 12. shows an alternative embodiment of the double annular co-extrusion spinnerette assembly.

FIG. 13. shows a plot of hydraulic permeability versus extraannular flow rate employed in fiber manufacture.

FIG. 14 is a schematic representation of a model assembled DNA sequence encoding a multifunctional biosynthetic protein comprising a leader peptide (used to aid expression and thereafter cleaved), a binding site, a spacer, and an effector molecule attached as a trailer sequence.

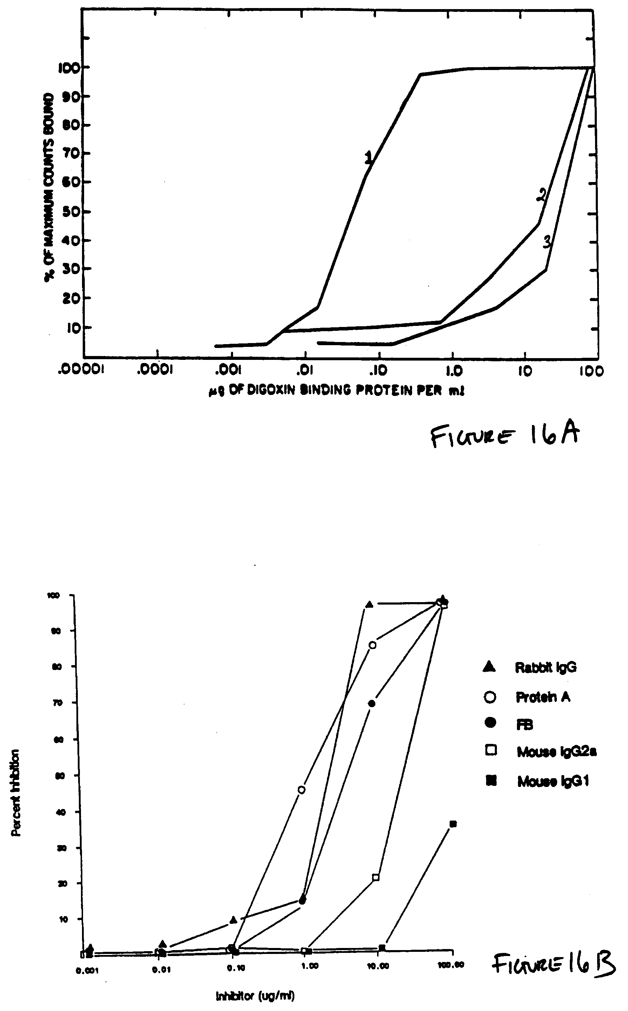

FIG. 15 describes a multifunctional protein (and the DNA encoding it) comprising a single chain BABS with the specificity of murine monoclonal 26-10, linked through a spacer to the FB fragment of protein A, here fused as a leader, and constituting a binding site for Fc. The spacer comprises the 11 C-terminal amino acids of the FB followed by Asp-Pro (a dilute acid cleavage site). The single chain BABS comprises sequences mimicking the $V_H$ and $V_L$. The $V_L$ construct is altered at residue 4 where valine replaces methionine present in the parent 26-10 sequence. The amino acid sequence of the expression product starts after the GAATTC sequence, which codes for an EcoRI splice site, translated as Glu-Phe on the drawing. This construct contains binding sites for both Fc and digoxin. Its structure may be summarized as;

FB-Asp-Pro-$V_H$-(Gly$_4$-Ser)$_3$-$V_L$,

where (Gly$_4$-Ser)$_3$ is a polypeptide connector region. It may be used in the apparatus and process of the invention for affinity chromatography purification of digoxin.

FIG. 16A is a graph of percent of maximum counts bound of radioiodinated digoxin versus concentration of binding protein adsorbed to the plate comparing the binding of native 26-10 (curve 1) and the construct of Figure 15 renatured using two different procedures (curves 2 and 3). Figure 16B is a graph demonstrating the bifunctionality of the FB-(26-10) BABS adhered to microtiter plates through the specific binding of the binding site to the digoxin-BSA coat on the plate. Figure 16B shows the percent inhibition of $^{125}$I-rabbit-IgG binding to the FB domain of the FB BABS by the addition of IgG, protein A, FB, murine IgG2a, and murine IgG1.

## 5.0. Detailed Description of the Invention

### 5.1. General Apparatus Description

Generally stated, this invention comprises an apparatus capable of carrying out an affinity separation process for removal of target molecules from a feed stream. A ligand specific to the target molecule or ligate is anchored to a membrane in the apparatus. The apparatus capacity for the target molecule is defined by the membrane geometry, membrane characteristics and its associated ligand's binding efficiency. The apparatus preferably has separate compartments on either side of the membrane defining a feed side and filtrate side chamber. A preferred process for carrying out affinity separation in the apparatus begins by flowing a first fluid containing the target or ligate molecule through the feed chamber of the membrane device. A specified volume of the feed perfuses the membrane at a flow rate defined by the membrane device's capacity for the target molecule and regulated by the filtrate pump. The process to recover the membrane bound target molecule continues by washing the device's filtrate chamber with a buffer solution, followed by washing the membrane structure and feed chamber. Eluant is used to flush the filtrate chamber, followed by elution of the target molecule from the membrane structure, preferably into the feed chamber and out the device. The membrane device is then equilibrated with a regeneration buffer by first flushing the filtrate chamber, and then flushing the membrane and feed chamber.

Preferably, the apparatus automatically performs the separation process with repeated rapid cycles. The apparatus membrane comprises substantially isotropic microporous membrane material on to which a ligand, specific to the target molecules, is anchored. The apparatus contains pumps and valves controlled by a microprocessor and, in its operation, is programmed to cycle through a membrane loading step with the feed stream containing the target molecule such as a protein, a washing step to remove remaining contaminants, an elution step to recover the target molecule and a membrane regeneration step to complete the cycle. Fluid containing the target molecule is recirculated with a feed pump from a reservoir and through the feed chamber of the apparatus. There, a fraction of the feed perfuses the membrane, regulated by a filtrate pump at a flow rate consistent with the device geometry and membrane characteristics, permitting the target molecule to be captured by the membrane. Eluant is used to flush the filtrate chamber, followed by elution of the target molecule from the membrane bound ligand. The membrane device is then equilibrated with a regeneration buffer by first flushing the filtrate chamber, and then flushing the membrane and feed chamber.

The ease of scale-up is again enabled by the unique membrane design. The system scales directly with membrane volume and ligand content. Operation is simplified by maximizing the automation of the process.

### 5.2. General Description of Affinity Membrane Process for Protein Purification

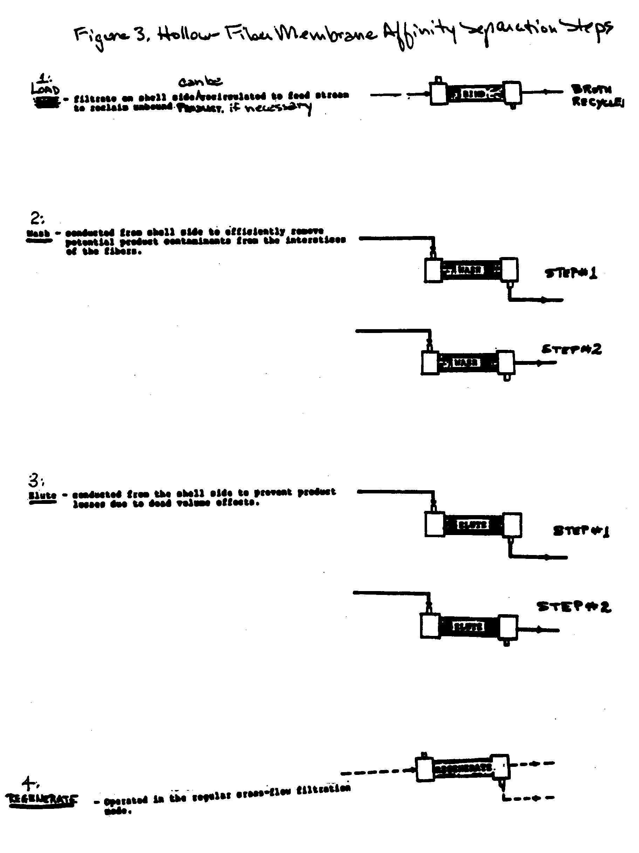

An affinity membrane-mediated protein purification process takes in four basic steps (Figure 3). In a actual hollow fiber affinity membrane device, as many as hundreds or thousands of fibers form a long cluster inside the module or container. (1) Feed solution containing the target protein is brought into contact with the immobilised affinity ligand by forcing the feed solution through the pores of the membrane. During passage of the feed through the membrane, target protein preferentially absorbs to the bound ligand on the pore walls. Loading can be carried out in a single-pass fashion, wherein the filtrate, depleted of target protein, is discarded; or in a multiple pass format, wherein the filtrate stream is contacted with the affinity membrane more than once. The loading step is carried out in the cross-flow mode, wherein the feed solution is recirculated on one side of the membrane and a portion is passed through the membrane. (2) One or more wash steps to remove nonspecifically adsorbed proteins. This step can be carried out either in the forward direction (i.e., with flow in the same direction as the feed solution was convected through the walls of the membrane) or in the reverse direction (i.e., backflush). (3) An eluant is passed through the membrane, thereby effecting release of the target protein from the immobilized affinity ligand. The target protein present in the eluate is recovered, generally in purified, concentrated form. (4) A regeneration solution is employed to remove residual eluant, thereby returning the membrane to an environment conducive to binding of the target protein to the immobilized ligand in a subsequent cycle. As with the wash step, steps (3) and (4) can be conducted with fluid flow in either the forward or reverse direction.

The affinity membrane device is a hollow fiber module, which is configured in an automated, microprocessor-controlled system comprising feed and filtrate-side pumps and a series of valves to control the rate and direction of fluid flow. In a typical purification cycle, loading is carried out in the cross-flow mode, with a recirculation rate of 30-100 device volumes per minute and a filtration rate of 1 to 20 device volumes per minute. The time required for this step is dependent on the target protein binding capacity of the device, the titer of the target protein in the feed solution and the filtration rate. Typical loading times range from one to twenty minutes.

Each wash step is typically performed in two stages, the first to flush residual filtrate from the shell side of the device and the second to wash the membrane in the direction of shell to lumen (i.e., backflush) in order to remove nonspecifically adsorbed proteins and to dislodge particulates in the lumen side of the membrane wall. Times for each wash step range from 15 seconds to five minutes and are typically 15 and 60 seconds for shell and lumen washes respectively; flow rates are typically 1-20 device volumes per minute. Wash buffers are generally sent to drain after passage through the membrane device.

Elution is performed in a fashion similar to the wash steps, i.e., in two stages, except that the eluted product is directed into a product reservoir. The flow rate in the elution step is typically 1-20 device volumes per minute and the duration is typically 1-10 minutes.

Regeneration is performed in the same fashion as the wash step, i.e. in two stages--shell and lumen regeneration, with the spent regeneration buffer generally flowing to drain. Times and flow rates of the regeneration step generally fall in the same range as the wash steps.

Typical cycle times for the affinity purification process outlined above range from ten to sixty minutes.

In a further embodiment of an affinity membrane process, the affinity membrane is protein A and the target protein is a murine monoclonal antibody, which is contained in impure form in cell culture supernatant, mouse ascites fluid or a fluid derived from cell culture supernatant or ascites fluid. The protein A containing affinity device is first loaded with monoclonal antibody. Washing is conducted using a buffered solution at near neutral pH. Typical buffers employed for washing include PBS pH 8.0 and 1.5 M glycine, 3 M NaCl pH 8.9. Product elution is effected using an acidic buffered solution such as 0.1 M citrate pH 3.0. Regeneration buffers are typically similar to those used for wash step.

## 5.3. Modified Membranes Utilized in Apparatus and Method of Their Manufacture

The present membrane modification process utilizes the functionalizable chain ends present in practically all polymeric materials. The instant process provides that treatment of suitable hydrophobic polymer samples, under heterogeneous conditions with linker moieties capable of forming a covalent bond with the hydrophobic polymer end groups, allows for the modification of the surface properties of the polymer while preserving desirable bulk properties. Using the methods of the invention, the surface properties of any article manufactured from the subject polymer may be modified while preserving the shape and microstructure of the manufactured article. Thus, bulk polymers with functionalizable end groups may be derivatized or modified under heterogeneous conditions whether the polymer is in powdered form, in the form of an extruded fiber, a microporous membrane, a solid strip, molded into a pipe, or incorporated into an artificial organ, skin, or prosthetic device. Such an article may be manufactured by techniques well-known in the art. Examples of these manufacturing methods include but are not limited to, injection, compression, and blow molding, blowing, calendaring, casting, coating, forming, lamination, or extrusion methods.

Furthermore, a process for the production of substantially isotropic microporous membranes is disclosed, which process takes advantage of the special properties of a unique four-component dope composition and an improved double annular multi-port spinnerette assembly.

### 5.3.1. Membrane and Module Design Considerations

Development of membrane-based affinity purification device to meet the needs of downstream processing requires an integrated approach to hollow fiber development and module design.

The most important membrane and device properties to be taken into account in designing a hollow fiber affinity device are:

(i) translumenal pressure drop ($\Delta P_{TL}$) as a function of module length;
(ii) $\Delta P_{TL}$ relative to transmembrane pressure drop $\Delta P_{TM}$; and
(iii) wall thickness and specific surface area in a given volume of membrane wall.

Translumenal pressure drop can clearly be minimized by producing hollow fibers with progressively larger internal diameter (ID). The following disadvantages are associated with this approach:

1. Fewer fibers can be contained in a given shell volume; therefore ligand loading capacity of the device would suffer.
2. Larger shell-side volume should be employed for containment of the increased number of fibers required to maintain the pre-determined ligand loading capacity, thus increasing both shell-side and lumen-side dead volumes.
3. Fibers with low wall thickness to outside diameter ratio (t/OD) are prone to physical damage (for example, fiber-colapse during backflushing), which would be the case for large ID thin-walled fibers.
4. Prior art for hollow fiber spinning technology (based on a non-solvent coagulation approach) cannot produce fibers with wall thicknesses much in excess of 100 μm without incurring severe resistances, both in the matrix and on the outer surface of the fiber.

The concept of a thick walled fiber with a large ID has important implications to module design. For example, Figure 5 illustrates the trend in the ratio of translumenal to transmembrane pressure drop as a function of fiber length at various wall thicknesses for a fiber ID of 1,000 μm. Decrease in the $\Delta P_{TL}/\Delta P_{TM}$ ratio with increased wall thickness signifies the improvement in uniformity of transmembrane flow along the length of the module.

Another benefit of thick walled fibers is the fact that for a given total membrane volume and packing density, dead volume decreases with increasing wall thickness.

The non-linear decrease in dead volume as fiber wall thickness is increased approaches a point of diminishing returns above 300 $\mu$m (e.g., at 1,000 $\mu$m ID). The net result of these considerations in the module design exercise is a device in which shell-side volume is only a factor of two greater than lumen-side volume.

The morphology and isotropy of microporous membranes are critical to the performance of hollow fibers employed in affinity purification. Pore sizes in the 0.22 $\mu$m to several $\mu$m range are not expected to result in sieving of protein molecules. Membranes in this pore size range are by convention classified as microfilters (MF) based on their ability to reject particulate matter.

Any significant pore size distribution (i.e., anisotropy, or asymmetry) within the membrane wall can cause entrapment of material and hence plugging. Because a viable affinity hollow fiber may be required to operate reproducibly for several hundred cycles in order to be cost-effective, membrane plugging should be avoided. Furthermore, structural (and chemical) integrity of the membrane should be such that catastrophic failure does not occur during repeated cycling.

Besides reducing the likelihood of plugging, isotropy enables fluids to flow freely and unhindered in either direction across the membrane. Due to their uniform resistance, membranes of this type would present less hindrance to flow in a backflush mode of operation than a skinned membrane. Ultrafilters are always skinned and would be very short lived if employed in this manner.

As membrane pore size increases (i.e., from UF to MF type membranes), internal surface area inevitably decreases. However, microfilters have the advantage of high volumetric flux at low transmembrane pressure, and a much lower probability of fouling and/or plugging. For these reasons, a MF-type, isotropic microporous hollow fiber is a highly desirable membrane for affinity purification applications.

5.3.2. <u>Modification Of Hydrophobic Polymer Surfaces</u>

Almost all known polymers have at least one functionalizable end group which is originally present in the monomer precursor or is introduced via the polymerization process. Thus, poly(ethylene oxide) has terminal hydroxyl groups, polyethersulfones have a halide at one end and a substituted phenol at the other, polyimides have terminal carboxyl and amino groups, and polyesters have terminal carboxyl and hydroxyl groups, to name a few polymers. Moreover, polymers prepared by free radical polymerization contain a functionalized initiator fragment at some of the polymer chain ends. For example, a vinyl halide, polymerized in the presence of azobis(isobutyronitrile), would contain a tertiary nitrile group in some of the chain ends. The proportion of polymer chains bearing the initiator fragment may be adjusted by varying the composition of the starting monomer/initiator mixture or the polymerization conditions.

The present modification process is directed to the functionalizable groups inherently present at the polymer chain ends. Furthermore, the present invention finds its most significant utility in derivatizing hydrophobic engineering homopolymers, copolymers, or blends having relatively inert monomer units in the polymer backbone. These types of polymers are generally prepared by step, radical chain, ionic chain, ring opening, or Ziegler-Natta polymerization and are generally regarded as being completely inert and not amenable to derivatization or modification by the mild conditions disclosed in this invention. Examples of these polymers include, but are not limited to polysulfones, polyethersulfones, polyimides, poly(arylene oxide), polyarylene sulfide, polyquinoxaline, polysilane, polysiloxane, polyurethanes, poly(etheretherketones), polycarbonates, polyesters, poly(vinyl halides), and poly(vinylidene polyhalides), derivatives, blends, mixtures, or copolymers thereof.

Further, although only one functionalizable end group need be present in a polymer chain, the number of available groups may be increased by chemically converting inherently less reactive or less useful end groups to more useful functionalities. For instance, terminal nitrile groups, introduced by the free radical polymerization using nitrile-containing initiators, may be reduced to amines using any of a wide variety of reducing agents available to the practitioner. Metal hydride reagents can serve this purpose, for example. Aromatic halide groups of polyarylsulfones can be converted to aryloxy groups by treatment with aqueous base. Also, isocyanate groups of polyurethanes may be converted to amines. In this manner, the number of useful terminal groups may be increased without affecting the integrity of the polymer backbone.

In addition, it has also been discovered that preconditioning polymer samples or articles manufactured therefrom by washing or heating the samples in aqueous or nonsolubilizing organic solvents increases the efficiency of the subsequent derivatization steps. Not seeking to be limited by theory, it is believed that the polymer interface or the surface of the manufactured article may be contaminated with foreign materials or processing aids thus shielding the functionalizable end groups. Washing the polymer samples may simply provide a means for mechanically stripping away these contaminants and exposing more of the polymer chain ends present at the surface or interfacial boundaries. Preferred organic solvents include, but are not limited to, acetonitrile and isopropanol. Aqueous solutions of these solvents may also be used. In one embodiment which does not form part of the invention, flat sheet microporous membranes, comprising polyethersulfone, (PES) as the primary or bulk polymer component, are immersed overnight at room temperature in a basic aqueous solution containing a diepoxide linker moiety. Optionally, the membrane samples may be preconditioned by heating them in aqueous solutions or washing them in acetonitrile or isopropanol. The substituted phenol groups of the PES chain ends exposed at the membrane surface are deprotonated by the base giving a nucleophilic phenoxide group. This nucleophile attacks an epoxide group of the linker moiety forming a covalently bound (i.e., ether bond) linker moiety. Because the covalently bound linker moieties are capable of forming at least one other covalent bond (via e.g., a second epoxide group) with another chemical entity, any molecule, macromolecule, or ligand, may then be covalently bound to the membrane surface. The covalently bound macromolecule is thus held very strongly and cannot be removed by washing or other mechanical means.

It is understood that the linker moiety serves to covalently bridge available functionalizable polymer chain ends with functional groups present on the macromolecule of choice. Thus the linking agent may preferably take the form of any polyfunctional organic molecule such as aliphatic or aromatic compounds bearing epoxide, carbonyl, carboxyl, amino, halo, hydroxyl, sulfonyl halides, acyl halides, isocyanate or combinations of these or other functional groups so long as the linker moiety is stable, compatible, and able to form covalent bonds with the bulk polymer and macromolecular or ligand species. The linker moiety may even incorporate inorganic functionality such as silicon, boron, aluminum, tin, phosphorous, sulfur, or nitrogen groups. It is within the scope of the present invention that other variations incorporating silicates, aluminates, borates, stannates, phosphates, or sulfonates, for instance, may also be used as the primary bridging group. However, ethylene glycol diglycidyl ether (EGDGE), 1,4-butanediol diglycidyl ether, epichlorohydrin, aliphatic dihalides, diacids, diacid halides, disulfonyl halides, and triazines are preferred embodiments of the linker moiety.

As mentioned above, the macromolecular or ligand species selected to modify the membrane surface or, more generally, any hydrophobic polymer surface, should preferably be capable of altering the surface properties of the hydrophobic polymer, membrane, or manufactured article and have at least one functional group which is able to form a covalent bond with the linker moiety. In some cases, one type of functionality may suffice. For example, the -OH groups of hydroxyethylcellulose (HEC) convey hydrophilicity to the hydrophobic membrane surface and also form ether bonds with the pendant epoxide groups of the covalently bound EGDGE. Depending upon the end use applications, therefore, the macromolecule can be comprised of molecules of low molecular weight, oligomers of intermediate molecular weight, or polymeric substances of high molecular weight. Preferably, the macromolecule is of high molecular weight and may include, but is not limited to, surfactants, carbohydrates, lectins, polypeptides, polysaccharides, liposomes, proteins, glycoproteins, olignucleotides, synthetic dyes, natural dyes, polynucleotides, derivatives, or mixtures thereof. Molecules or polymers which are capable of bearing a charge, either cationic or anionic, or those bearing nonionizable groups are also useful. FIG. 1 is a schematic depiction of part of the basic processes of the invention.

Preferred macromolecular species include polysilanes, polysiloxanes, hydroxyalkylcellulose, dextran, carboxymethylcellulose, poly(ethylene imine), poly(carboxymethylethylene imine), poly(vinyl alcohol), derivatives, blends, mixtures or copolymers thereof. The macromolecules may also be biologically important molecules and may include, but are not limited to monoclonal antibodies, polyclonal antibodies, antigenic substances, enzymes, carrier proteins, coagulation factors, cofactors, inhibitors, hormones, immunoglobulins, histones, plasmids derivatives, or mixtures thereof.

It should be apparent that the process for covalently binding the macromolecule to the polymer chain ends can be repeated several times. Subsequent applications are likely to utilize functionalizable groups of the first macromolecular layer, however, because these groups are present in much greater concentrations than the remaining unutilized polymer chain ends. Each successive binding thus involves increasing numbers of linker moieties resulting in a stronger bond as well as greater amounts of loading on the membrane surface. For this reason, it may sometimes be more advantageous to "amplify" the surface functionalizable groups present on a membrane surface by first applying one or more layers of a readily available macromolecular species before applying a more valuable ligand species on the top layer. In a case where layers of hydrophilic macromolecules are covalently attached, the nonspecific protein binding of the modified surface is lowered dramatically versus the virgin hydrophobic surface.

The covalent binding of the surface ligand layers need not necessarily involve the intermediacy of a linker moiety although in certain cases, a "linker molecule" is best employed. It is possible, for example, to render certain functional groups of macromolecules already bound to the polymer surface more reactive towards the functional groups of an added ligand by employing activating reagents. These methods which lead to active sites on the macromolecule are well known in the art and include the use of such reagents as dialkylcarbodiimides (for forming amide bonds), diazotization (for coupling aromatic groups), cyanogen bromide (most commonly used for the activation of solid supports), epoxides, sulfonyl chlorides, or other processes, such as the use of 2-fluoro-1-methylpyridinium p-toluensulfonate (FMP), which facilitate the coupling reaction by introducing a superior leaving group. Other nonlimiting reagents which may be used to covalently bind the ligand to the macromolecule or polymer surface include diepoxides, haloepoxides, 2,4,6-trichloro-S-triazines, diacid chlorides, dialdehydes, diisocyanates, or haloalkylcarboxylic acids.

In a specific preferred embodiment of the invention, Protein A molecules may be bound directly by using EGDGE as the linker moiety to the polymer chain ends of a hydrophobic PES membrane. Preferably, the PES membrane is first modified by applying a few layers of hydroxyethylcellulose. Other hydroxyl-containing macromolecules such as dextran, agarose, hydroxypropylcellulose, or poly(vinyl alcohol) may be used with equal efficacy. After the number of hydroxyl groups on the membrane surface is thus "amplified," the membrane is treated with FMP to generate active sites on the membrane surface. The membranes are then exposed to a slightly basic buffered solution of Protein A to effect efficient covalent attachment of this valuable ligand.

Membrane samples with covalently attached Protein A are quite useful, for instance, in the selective binding and isolation of human immunoglobulin G (IgG) from a mixture of serum proteins. This utility is demonstrated in the Examples section of this specification.

It is evident that many types of ligands can be bound to the hydrophobic membrane by the methods of this invention. Natural products and biologically active components as well as synthetic polymeric materials may be used. All the types of molecules listed above as possible macromolecular species, for example, may also comprise the ligand species. Additional non-limiting examples include dyes, enzyme inhibitors, amphoteric, ionizable molecules, hydrophobic long chain aliphatic hydrocarbons, aromatic hydrocarbons, and the like. Silane derivatives may also be useful, not only as simple ligands, but as potential polymerizable species. Examples of these silanes include but are not limited to terminal amino aliphatic hydrocarbon trialkyloxy silanes, such as aminoethyl aminopropyl trimethoxysilane, carboxyl-substituted silanes, long chain aliphatic or aromatic hydrocarbon silanes, and the like. Many types of functional groups may, of course, be present in the silane compounds.

Preferred ligands include natural or recombinant Protein A, Avidin, Biotin, Heparin, animal, plant, bacterial cell surface receptors, antibodies against IgG, IgM, IgA, IgE, tissue plasminogen activator (TPA), human interleukin (IL) proteins, human chorionic gonadotropin (HCG), thyrotropic hormone (TSH), carcinoembryonic antigen (CEA), a-fetoprotein, transforming growth factor (TGF), interferons, and blood coagulation factors such as Factor VIII or IX. In general, ligands capable of binding specific ligates from a sample solution or mixture with a dissociation constant of about $10^{-2}$-$10^{-12}$ $\underline{M}$ are preferred. Those with binding constants less than about $10^{-6}$ $\underline{M}$ are highly preferred. Other preferred ligands and possible substrates or ligates are listed in U.S. Patent No. 4,693,985. The entire disclosure of this reference is incorporated herein by reference.

Still other examples of useful ligands may be easily found in catalogs for products useful in molecular biology research (See, e.g., ligand index in the Pharmacia Affinity and Chromatography Media catalog incorporated, herein by reference). An abbreviated list is illustrative: acetylglucosamine, anti-A lectin, arginine, butylamine, castor bean lectin, Cibacron Blue, coenzyme A, decylamine, diadenosine pentaphosphate, gelatin, hemoglobin, iminodiacetic acid, HMG-CoA, lysine, NADP, oligo(dA, dC, dG, dI, or dT), PHA-L, polyriboguanylic acid, poly(I)poly(C), Procion Red, uridine nucleotides, or conjugates thereof. The only limitation on the ligand species is that it have at least one functional group with which to form a covalent bond with the linker moiety or the active sites on the macromolecule.

A favored type of ligand for use in the invention is a single chain multifunctional biosynthetic protein expressed from a single gene produced by recombinant DNA techniques. These novel biosynthetic, bifunctional proteins comprise biosynthetic antibody binding sites, that is, "BABS", and a biosynthetic polypeptide defining a membrane binding region. One region is capable of selective antigen recognition and preferential antigen binding. Its structure is patterned after and mimics the properties of natural $F_V$ regions from immunoglobulins, or one chain thereof. The other region comprises one or more peptide-bonded additional "effector" protein or polypeptide regions, designed to have one or more functional groups capable of selective covalent binding to a linker moiety, another macromolecule or ligand, or to the derivatized interfacial area of the membrane polymer itself. This second effector protein or polypeptide sequence may act simply to provide distance between the membrane surface and the ligand binding site. Additionally, it may act as a ligand itself as, for example, protein A. The use of a multifunctional biosynthetic protein wherein both the macromolecule and the ligand of interest are attached as a single unit reduces the number of covalent attachment steps required, allows for the attachment of multiple, potentially distinct ligand binding sites, and has other advantages. The smaller molecular size of the BABS as compared with intact antibodies and other natural binding proteins permits a greater surface density of binding sites to be engineered into the affinity device; the effector domain can comprise amino acids which individually enable permanent or reversible covalent bond formation or amino acid regions having a tertiary structure designed to facilitate binding.

5.3.2.1. Design and Manufacture of Single Chain Multifunctional Biosynthetic Protein

The technology required for the recombinant production of single chain binding sites having affinity and specificity for a predetermined antigenic determinant is disclosed in detail in U588/01737 (WO-A-8809344) and in copending U.S. application Serial Number 052,800 filed May 21, 1987. In view of this disclosure, and that of the listed applications, persons skilled in recombinant DNA technology, protein design, and protein chemistry can produce such sites which, when disposed in solution, have high binding constants and excellent specificity. Moreover, because these structures are designed at the DNA level, an essentially limitless combination of binding sites and effector proteins is possible, each of which can be refined as disclosed herein to optimize binding activity at each region of the synthetic protein. The synthetic proteins can be expressed in procaryotes such as E. coli, and thus are less costly to produce than immunoglobulins or fragments thereof which require expression in cultured animal cell lines.

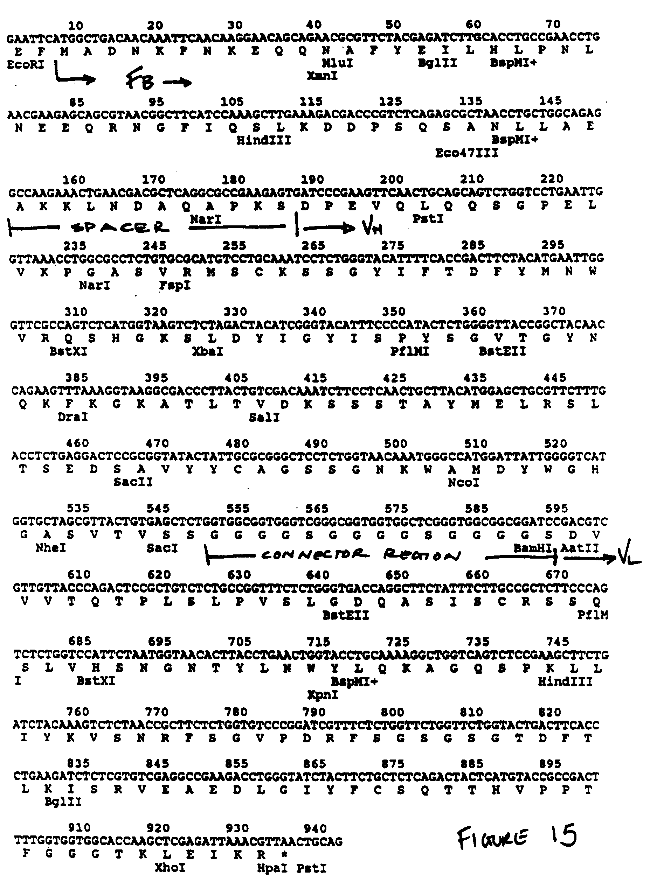

The BABS useful in the present invention are designed at the DNA level. The chimeric or synthetic DNAs are then expressed in a suitable host system, and the expressed proteins are collected and renatured if necessary. A general structure of the DNA encoding the proteins is set forth in Figure 14. As illustrated, it encodes an optional leader sequence used to promote expression in procaryotes having a built-in cleavage site recognizable by a site specific cleavage agent, for example, an endopeptidase, used to remove the leader after expression. This is followed by DNA encoding a $V_H$-like domain, comprising complementarity determining regions (CDRs) and framework regions (FRs), a connector sequence, a $V_L$-like domain, again comprising CDRs and FRs, a spacer, and an effector protein or fragment. Alternatively, the effector protein DNA may precede that of the $V_H$-like domain. Also, the binding site may consist of a single $V_H$ or $V_L$-like domain if lower affinity can be tolerated. After expression, folding, and cleavage of the leader (if any), a bifunctional protein is produced having a binding region whose specificity is determined by the CDRs, and a peptide-linked independently functional effector region.

The design of the BASS sequence is based on the observation that the three subregions of the variable domain of each of the heavy and light chains of native immunoglobulin molecules collectively are responsible for antigen recognition and binding. It is well known that Fv, the minimum antibody fragment which contains a complete antigen recognition and binding site, consists of a dimer of one heavy and one light chain variable domain in noncovalent association. It is in this configuration that the three subregions, called herein "complementarity determining regions" or CDRs, of each variable domain interact to define an antigen binding site on the surface of the $V_H$-$V_L$ dimer. Collectively, the six CDRs confer antigen binding specificity to the antibody. A single variable domain (or half of an Fv comprising only three CDRs specific for an antigen) does have the ability to recognize and bind antigen, although at a lower affinity than an entire binding site (Painter et al. (1972) Biochem. 11:1327-1337).

Each of the CDRs consists of one of the hypervariable regions or loops and of selected amino acids or amino acid sequences disposed in the framework regions or FRs which flank that particular hypervariable region. These FRs serve to hold the CDRs in their proper orientation for antigen binding. It has been discovered that biosynthetic domains mimicking the structure of the two chains of an immunoglobulin binding site may be connected by a polypeptide connector region while closely approaching, retaining, and often improving binding properties.

The two domain, single chain composite polypeptide of the BABS has a structure patterned after tandem $V_H$ and $V_L$ domains, but with the carboxyl terminal of one attached through a connector amino acid sequence to the amino terminal of the other. The connector sequence preferably comprises at least about 14 amino acids, or spans a distance of at least 40 Å in aqueous solution, and comprises residues which together present a hydrophilic, relatively unstructured segment. Each domain comprises a set of amino acid sequences analogous to immunoglobulin CDRs held in appropriate conformation by a set of sequences analogous to the framework regions (FRs) of an Fv fragment of a natural antibody. The CDR and FR polypeptide segments are designed based on sequence analysis of the Fv region of preexisting antibodies or of the DNA encoding them.

Either the amino or carboxyl terminal ends (or both ends) of these chimeric, single chain binding sites are attached to an amino acid sequence designed for attachment to a surface. For example, the synthetic binding site may include a leader and/or trailer sequence defining a polypeptide which provides a functional group designed to link covalently to an activated group on the membrane surface. Preferred effector polypeptide sequences include sequences containing positively or negatively-charged amino acids, or sulfhydryl-containing amino acids, such as methionine. This fused, independently functional section of protein should be distinguished from fused leaders used simply to enhance expression in prokaryotic host cells or yeasts. The multifunctional proteins also should be distinguished from the "conjugates" disclosed in the prior art comprising antibodies which, after expression, are linked chemically to a second moiety.

Connecting the independently functional antigen binding region and the membrane surface binding region of the bifunctional protein is a spacer comprising a short amino acid sequence whose purpose is to separate the functional regions so that the binding region can assume its active tertiary conformation. The spacer can consist of an amino acid sequence present on the end of a functional protein which sequence is not itself required for its function, and/or specific sequences engineered into the protein at the DNA level.

The spacer generally may comprise between 5 and 25 residues. Its optimal length may be determined using constructs of different spacer lengths varying, for example, by units of 5 amino acids. The specific amino acids in the spacer can vary. Cysteines should be avoided to prevent improper folding of the bifunctional protein. Hydrophilic amino acids are preferred. The spacer may be designed to assume a structure, such as a helical structure, but preferably comprises amino acids which do not promote domain formation.

The ability to design the BABS depends on the ability to determine the sequence of the amino acids in the variable region of monoclonal antibodies of interest, or the DNA encoding them. Hybridoma technology enables production of cell lines secreting antibody to essentially any desired substance that produces an immune response. RNA encoding the light and heavy chains of the immunoglobulin can then be obtained from the cytoplasm of the hybridoma. The 5' end portion of the mRNA can be used to prepare cDNA for subsequent sequencing, or the amino acid sequence of the hypervariable and flanking framework regions can be determined by amino acid sequencing of the V region fragments of the H and L chains. Such sequence analysis is now conducted routinely. This knowledge, coupled with observations and deductions of the generalized structure of immunoglobulin Fvs, permits one to design synthetic genes encoding FR and CDR sequences which likely will bind the antigen. These synthetic genes are then prepared using known techniques, or using the technique disclosed below, inserted into a suitable host, and expressed, and the expressed protein is purified. Depending on the host cell, renaturation techniques may be required to attain proper conformation. The various proteins are then tested for binding ability, and one having appropriate affinity is selected for incorporation into a reagent of the type described above. If necessary, point substitutions seeking to optimize binding may be made in the DNA using conventional cassette mutagenesis or other protein engineering methodology such as is disclosed below.

Preparation of the effector polypeptide region of the bifunctional protein also is dependent on knowledge of the amino acid sequence (or corresponding DNA or RNA sequence) of the protein of interest. Such sequences are reported in the literature and available through computerized data banks.

The DNA sequences of the binding site and the second protein domain are fused using conventional linker techniques, or assembled from synthesized oligonucleotides, and then expressed using equally conventional techniques.

The processes for manipulating, amplifying, and recombining DNA which encode amino acid sequences of interest are generally well known in the art, and therefore, not described in detail herein. Methods of identifying and isolating genes encoding antibodies of interest are well understood, and described in the patent and other literature. In general, the methods involve selecting genetic material coding for amino acids which define the proteins of interest, including the CDRs and FRs of interest, according to the genetic code.

Accordingly, the construction of DNAs encoding proteins as disclosed herein can be done using known techniques involving the use of various restriction enzymes which make sequence specific cuts in DNA to produce blunt ends or cohesive ends, DNA ligases, techniques enabling enzymatic addition of sticky ends to blunt-ended DNA, construction of synthetic DNAs by assembly of short or medium length oligonucleotides, cDNA synthesis techniques, and synthetic probes for isolating immunoglobulin or other bioactive protein genes. Various promoter sequences and other regulatory DNA sequences used in achieving expression, and various types of host cells are also known and available. Conventional transfection techniques, and equally conventional techniques for cloning and subcloning DNA are useful in the practice of this invention and known to those skilled in the art. Various types of vectors may be used such as plasmids and viruses including animal viruses and bacteriophages. The vectors may exploit various marker genes which impart to a successfully transfected cell a detectable phenotypic property that can be used to identify which of a family of clones has successfully incorporated the recombinant DNA of the vector.

One method for obtaining DNA encoding the proteins disclosed herein is by assembly of synthetic oligonucleotides produced in a conventional, automated, polynucleotide synthesizer followed by ligation with appropriate ligases. For example, overlapping, complementary DNA fragments comprising 15 bases may be synthesized semi manually using phosphoramidite chemistry, with end segments left unphosphorylated to prevent polymerization during ligation. One end of the synthetic DNA is left with a "sticky end" corresponding to the site of action of a particular restriction endonuclease, and the other end is left with an end corresponding to the site of action of another restriction endonuclease. Alternatively, this approach can be fully automated. The DNA encoding the protein may be created by synthesizing longer single strand fragments (e.g., 50-100 nucleotides long) in, for example, a Biosearch oligonucleotide synthesizer, and then ligating the fragments.

A particularly useful method of producing the BABS is to produce a synthetic DNA encoding a polypeptide comprising, e.g., human FRs, and intervening "dummy" CDRs, or amino acids having no function except to define suitably situated unique restriction sites. This synthetic DNA is then altered by DNA replacement, in which restriction and ligation is employed to insert synthetic oligonucleotides encoding CDRs defining a desired binding specificity in the proper location between the FRs. This approach allows one to change the binding specificty of the BABS at will, and also facilitates empirical refinement of the binding properties of the BABS.

This technique is dependent upon the ability to cleave a DNA corresponding in structure to a variable domain gene at specific sites flanking nucleotide sequences encoding CDRs. These restriction sites in some cases may be found in the native gene. Alternatively, non-native restriction sites may be engineered into the nucleotide sequence resulting in a synthetic gene with a different sequence of nucleotides than the native gene, but encoding the same variable region amino acids because of the degeneracy of the genetic code. The fragments resulting from endonuclease digestion, and comprising FR-encoding sequences, are then ligated to non-native CDR-encoding sequences to produce a synthetic variable domain gene with altered antigen binding specificity. The additional nucleotide sequences encoding a bioactive molecule or other effector sequence may then be linked to the gene sequences to produce a bifunctional protein.

The expression of these synthetic DNA's can be achieved in both prokaryotic and eucaryotic systems via transfection with an appropriate vector. In E. coli and other microbial hosts, the synthetic genes can be expressed as fusion protein which is subsequently cleaved. Expression in eucaryotes can be accomplished by the transfection of DNA sequences encoding CDR and FR region amino acids and the amino acids defining a second function into a myeloma or other type of cell line. By this strategy intact hybrid antibody molecules having hybrid Fv regions and various bioactive proteins including a biosynthetic binding site may be produced. For fusion protein expressed in bacteria, subsequent proteolytic cleavage of the expression enhancing leader sequence can be performed to yield free bi- or multifunctional protein, which can be renatured to obtain an intact biosynthetic, hybrid antibody binding site.

### 5.3.3. Process For Manufacturing Substantially Isotropic Microporous Membranes

The process for derivatizing hydrophobic polymer interfaces discussed above is especially applicable to the surface modification of microporous membranes. In the course of devising new ways of preparing membranes, the inventors have discovered a unique four-component dope composition which in combination with other aspects of the overall manufacturing process provides membranes with substantially isotropic microporous structures.

### 5.3.3.1. Dope Composition

This novel dope composition comprises a primary polymer component, a secondary polymer component and two solvents, the first of which is an effective solvent for both polymers (i.e., one in which both polymers readily dissolve), and the second an effective solvent for the secondary polymer component but a nonsolvent for the primary polymer component (i.e., one in which the primary polymer is poorly or sparingly soluble, or, preferably, substantially insoluble). It is the latter solvent component which conveys a certain degree of incompatibility or instability to the resulting dope mixture, and by judiciously adjusting the relative amounts of the various components, the critical parameters, such as the lower and upper critical solution temperatures (LCST and UCST, respectively) which characterize the dope composition, may be optimized to better fit the desired processing steps.

An important feature of this invention is selection of the polymer pair to be employed in the dope. Relatively good compatibility is needed to enable the manipulation of the phase boundary as a function of the nonsolvent content (e.g., glycerin) to the extent desired. Compatibility can be defined very generally in the following way: when two polymers can be co-dissolved in a common solvent (or mixture of solvents) in any ratio at 5-50% total solids to obtain an optically clear solution at a manageable temperature, such a solution is said to be compatible.

As in the case of the PES/PEO (primary/secondary polymer components, respectively) polymer pair, a solvent such as glycerin serves both as a solvent for one polymer while acting as a nonsolvent for the other. The polymer for which this liquid is an effective nonsolvent comprises the major or primary component of the final membrane desired (in this case, PES). The polymer which is soluble in both solvents should also possess hydrophilic character, such as water-soluble polymers, but is not limited to this group. However, one should keep in mind that when a water-soluble polymer is used, higher molecular weight forms are statistically more likely to be retained in the final membrane by chain entanglement than those of lower molecular weight when an aqueous quench is employed in manufacture. This entrapment, in itself, may be desirable because a certain degree of hydrophilicity and wettability is imparted to the otherwise hydrophobic membrane surface.

A broad range of hydrophilic polymer (e.g., PEO) molecular weights are useful in this invention, from molecular weights in the tens of thousands (e.g., PEG) up to millions in molecular weight). The preferred molecular weight for PEO is no less than 100,000.

An important advantage of the PES, polysulfone (PS), and other high glass transition/melt temperature polymers as the main component in these blends, is that their use results in membranes which can be autoclaved repeatedly without detrimentally altering membrane properties. Indeed, autoclaving can, in fact, increase tensile strength of PES/PEO fibers, presumably by enabling polymer relaxation to occur and thereby prevent the slow tightening or densification process observed with solution cast membranes over days, weeks, or even months. Such a densification process often leads to a reduced hydraulic permeability over time. Finally, PES/PEO fibers are sufficiently hydrophilic (by virtue of the presence of some PEO at the surfaces of the membrane) that wet/dry cycling can be done without wetting aids and with no change in performance.

Examples of other suitable polymer pairs which may be utilized in this invention include, but are not limited to: polysulfone (PS)/PEO; PES/Polyvinyl pyrrolidone (PVP) (particularly the high molecular weight forms, e.g., MW about 360,000 of PVP); PS/PVP (MW ~360,000); Polyvinylidene fluoride (PVDF)/PEO; PES/Epichlorohydrin copolymers of PEO; PES/Polyvinyl alcohol (PVA); Polyphenylene oxide (PPO)/Hydrophilized forms of polystyrene (including copolymers and sulfonylated polystyrene); poly(acrylonitrile) (PAN) and copolymers/hydrophilic acrylic polymers (including polyacrylamide), or PVP; PES/hydrophilized forms of PES (including sulfonated PES); and PS/hydrophilized forms of PS.

Suitable solvent/non-solvent pairs are numerous but are preferably within the definition of compatibility and selection of the polymer pair stated previously. Generally speaking, the class of heterocyclic or amine-containing solvents (including dimethylformamide, N-methyl pyrrolidone, dimethylacetamide, and piperidine) and polar aprotic solvents such as dimethyl sulfoxide and the like are excellent and preferred choices as first solvents for both polymers by virtue of their relatively high boiling points, polar character and water miscibility. Alternatives to glycerin as second solvents with both solvent power for the secondary polymer component and nonsolvent power for the major polymer component include: 2-methoxyethanol, formamide, ethylene glycol, various alcohols and the like.

For ease of processing it is preferred to use aqueous quench/wash baths, but this process need not be so limited. Similarly, the allowable ranges for the LCST and UCST phase boundaries are limited only by attainable temperature ranges based on available equipment and suitable quench media. In a process based on an aqueous quench/wash, the preferred ranges for phase boundaries are: LCST about 80°C and UCST about 50°C.

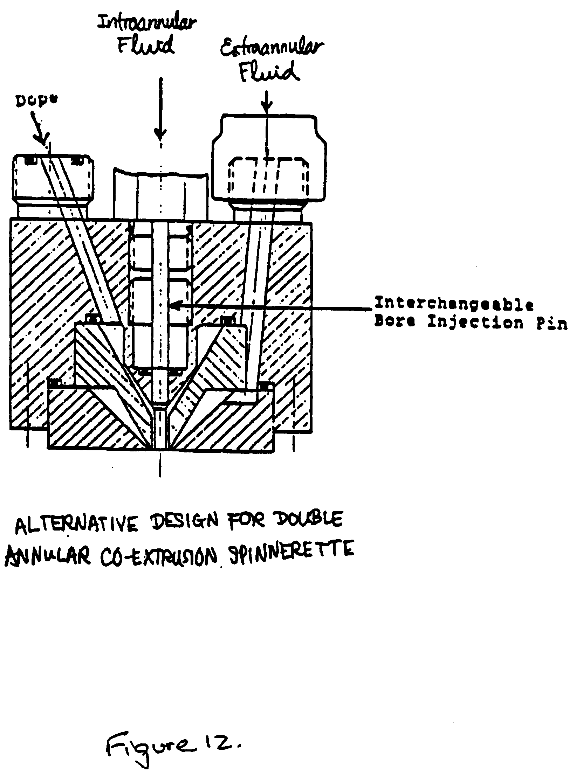

In a preferred embodiment of the invention, polyethersulfone (PES), a hydrophobic polymer, is selected as the primary polymer component of the dope composition. Poly(ethylene oxide) (PEO), $\underline{N}$-methylpyrrolidone (NMP), and glycerin make up the secondary polymer component, first, and second solvents, respectively. The cloud point phase diagram for a series of dopes comprised of 20 wt% PES and a mixture containing 7.5 wt% PEO in NMP containing varying amounts of glycerin, as a percent weight of the total mixture, is shown in FIG. 10. As shown, the range of temperatures, in which a single phase homogeneous composition is obtained, can be varied for a given PES/PEO ratio depending on the amount of nonsolvent present. Thus the single phase temperature range can be as wide as 100°C to as narrow as a few degrees. In most cases, the preferred range is about 30-40°C. For the four component dope composition, it has surprisingly been discovered that a temperature phase inversion occurs not only at temperatures below the UCST but also at high temperatures, above the LCST (See FIG. 10).

### 5.3.3.2. Flat Sheet Membranes

It has also been discovered that membranes with substantially isotropic porous structures (i.e., structures in which the pore diameters are within about an order of magnitude or so of each other) can be prepared and preserved by subjecting the homogeneous dope composition to an abrupt change in temperature, preferably at or above the LCST, and essentially simultaneously "freezing out" the precipitated structure by introducing a nonsolvent for at least the primary polymer component. This procedure is carried out in the case of flat sheet membranes, which do not form part of the invention, by immersing a liquid film of the dope composition in a nonsolvent quenching bath (e.g., water) maintained at a temperature above the LCST. Quenching the mixture above the LCST produces more open membrane structures with larger isotropic pores in the μm range. By contrast, anisotropic microporous or macrovoid-containing membranes are obtained from quench baths held below the LCST or UCST. The membrane pore sizes, besides being substantially isotropic, may thus be potentially controlled by selecting the temperature of the quench bath. Furthermore, the membranes produced by quenching above the LCST are substantially skinless having a very high density of pores in the exterior surface of the membrane.

In effect this invention has succeeded in harnessing a thermal phase inversion process initiated at a high temperature by an almost instantaneous change in the temperature of the entire dope composition which, in turn, is brought about by immersing the solution in a quenching bath. Not seeking to be limited by theory, it is believed that the resulting microphase separated binary polymer system has a substantially isotropic microstructure as a result of the uniform rapid transfer of heat. The microstructure is then "frozen" and preserved in the integral membrane by a secondary process, occurring simultaneously with the thermal phase inversion, involving the diffusion of the nonsolvent quenching medium. This combination of a high-temperature phase inversion and nonsolvent quench processes provides membranes which are substantially isotropic and which can be made relatively thick and self-supporting. The description substantially isotropic is meant to encompass perfectly isotropic pore size distributions as well as a distribution of pore size within about one order of magnitude as determined by porometry, by passage of latex spheres, or by examination using scanning electron microscopy (SEM). Methods for determination of particle size by porometry are documented in the "Operator's Handbook for Coulter Porometer", issue A, June 1986, Part 9903175 (issued by Coulter Electronics Limited, Northwell Dr., Juton, Beds., England).

While current techniques for preparing microporous hollow fibers are capable of producing membranes with surface pores ranging from tenths of a μm to several μm in diameter, such conventional membranes of the prior art typically retain particles more than an order of magnitude smaller than the surface pore size. As an example, the nominal 0.2 μm-rated hollow fiber commercially available from AG Technology (Needham, MA) has been found to substantially completely reject latex spheres as small as 0.03 μm; which is more than an order of magnitude smaller than the surface pore size of about 1 μm as revealed by SEM examination. Furthermore, SEM examination reveals that pores rapidly decrease in diameter to less than 0.1 μm below the lumen surface over a distance of a few μm. SEM studies of such a membrane after a 0.03 μm latex challenge test shows entrapment of latex particles within the finely porous region in the matrix below the lumen surface. A typical microporous hollow fiber of the present invention was determined by SEM to contain surface pores in the range of 1 μm. Latex sphere challenge tests, as a means for defining isotropy, show that latex particles as large as 0.25 μm passed freely across the membrane wall. Furthermore, SEM examination of the hollow fiber wall confirmed that pore size distribution across the entire membrane wall was substantially isotropic, with the smallest pores in the fiber wall typically being no smaller than about 0.3 μm.

It has surprisingly been discovered that a reverse pore-size distribution (i.e., large pores near membrane/solvent interface and smaller pores within membrane matrix) membrane can be prepared by a modified quench bath which contains in it a sufficient amount of strong solvent to swell the pores of the portion of the membrane in contact with the bath. After the desired pore sizes have been achieved, the strong solvent is diluted and eventually displaced with a nonsolvent wash composition. The resulting membranes can be made relatively thick and self-supporting in this fashion using the four-component dope described above. These membranes are useful in a variety of microfiltration applications particularly in the separation of blood cells from whole blood.

By employing the manufacturing process disclosed in the present invention, substantially isotropic self-supporting membrane structures of high solid content can be made. It is believed that initiating the phase inversion process at higher temperatures results in a less viscous system in which greater numbers of polymer component molecules migrate to their respective domains before the solidification or precipitation of the membrane. Such a migration process could be responsible for the larger pore structures observed for membranes prepared from quench baths kept above the LCST.

### 5.3.3.3. Improved Co-Extrusion Spinnerette And Production of Hollow Fibers

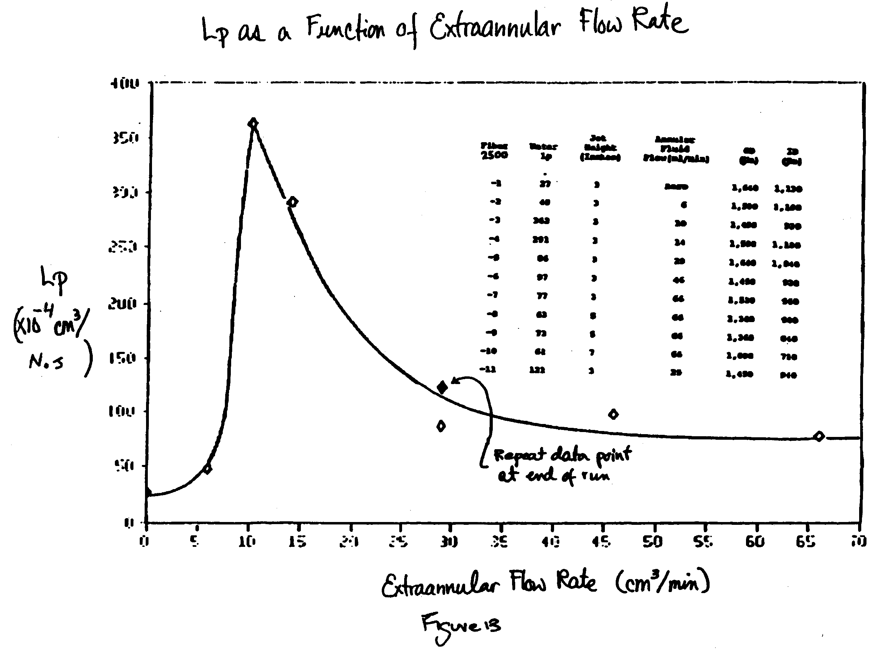

For the production of hollow fiber membranes having substantially isotropic microporous structures, manufacturing procedures more sophisticated than immersing a liquid film of dope composition into a quenching bath (i.e., in the production of flat sheets) are required. For this purpose an improved spinnerette assembly, a schematic diagram of which is shown in FIG. 11, is used.

The "co-extrusion" spinnerette assembly depicted in the figure is part of an overall manufacturing system which includes among other things, pots for mixing, stirring, and holding the dope composition; pipes, tubing, or feed lines to introduce and deliver reagents, solvents, dopes, or fluids; pumps; stirrers; baths; and heating units to control the temperature of these devices, including the spinnerette assembly. An important feature of the present spinnerette is that no more than three fluid entrance ports are necessary to achieve the desired flow distribution within the device. That is, there is one port for each of the flow paths. The flow of each fluid through each port can be independently manipulated providing greater flexibility and simplicity to the overall process control. Moreover, the flow rate of the respective fluids can affect the structural characteristics of the resulting fiber.

The co-extrusion spinnerette of FIG. 11 embodies the central feature of this invention, while providing a wide degree of versatility in the use thereof (consistent with the above teachings). As alluded to above, there are three fluid paths formed by four or five modular parts. These parts are listed below in the order in which they are labeled in FIG. 11.

1. The top portion provides containment of the dope at the top of the device and an entrance port for said dope;

2. This portion consists of a ring with a plurality of spokes radiating to the center where the spokes provide support for the hollow bore injection pin -- the bore or intraannular fluid for making a hollow fiber passes through one of these spokes to the bore injection pin;

3. The port for the extraannular fluid is contained in this portion, and in conjunction with portion 5, the extraannular space is formed;

4. An optional spiral device is provided, which is designed to overcome any flow distribution problems which may arise from high viscosity solutions entering the extraannular space from a single port;

5. The face-plate of the spinnerette contains the surface (pointing downward towards the quench bath), on which the double annular configuration of this invention is evident.

Numerous alternative embodiments of this invention are possible. One example is depicted in FIG. 12, where the central theme of two mutually concentric annuli configured around a hollow pin is maintained. In this case the design emphasizes ease of device manufacture, where mass production of identical spinnerettes can be accomplished most effectively. Furthermore, by making the bore injection or intraannular fluid pass through the device vertically, it enables the use of interchangeable pins. This feature provides additional cost savings as damaged pins can be replaced modularly. Also, fibers of different dimensions can be produced readily by changing pins and/or modifying the face-plate portion.

Using the present device, an intraannular or bore injection fluid, which may be a gas, a vapor, or a liquid, can be made to emerge from the hollow pin of the central bore. Examples of a preferred intraannular fluid include, but are not limited to, an inert gas, water, water vapor, a water-miscible organic solvent, an aqueous solution of a water-miscible organic solvent, an aqueous solution of a water-soluble polymer, or mixtures thereof. Surrounding this central bore are two concentric annuli. From the inner annulus is extruded the dope solution. The dope composition emerges as a hollow tube which is prevented from collapsing over itself by the presence of the stream of intraannular fluid. As this process occurs, an extraannular fluid can be forced to flow over the outside surface of the fiber by utilizing the second, outer annulus which circumvents the first. This extraannular fluid is preferably a solvent system which is similar or the same as the intraannular solution used. Preferred nonlimiting examples of an extraannular fluid include water, alcoholic solvent, a water-miscible organic solvent, an aqueous solution of a water-miscible organic solvent an aqueous solution of a water-soluble polymer, or mixtures thereof. It is especially preferred that both fluids be maintained at a temperature above the LCST of the dope composition and are both able to serve as essentially the quenching media for the phase separated polymer system. In this manner, the inner and outer surfaces of the hollow fiber may be exposed to the same environment resulting in a more isotropic distribution of pore sizes than what would normally result in a process which does not enjoy the benefit of the outer curtain of extraannular fluid.

Using such a configuration in the process for manufacturing the hollow fiber membranes of the present invention, the variations in pore size brought about by the ambient air residing between the exit ports of the spinnerette and the normal stationary quenching/washing bath, which air is a different medium than the intraannular fluid and which is usually at a different temperature, is essentially eliminated. Thus the distance between the extrusion assembly and the stationary bath is no longer an important factor and a much greater flexibility to the placement of process equipment is subsequently achieved.

Perhaps more significantly, since the phase separation, extrusion, and quenching can be achieved in a very short span of time by employing the intra- and extraannular fluid configuration, the overall microscopic process for producing the hollow fibers more closely resembles the sequence of events which takes place during the simple flat sheet manufacturing process. Consequently, this invention is able to use essentially the same dope compositions, quenching media, and temperature settings used in preparing flat sheets, to produce hollow fibers with microstructures and characteristics similar to that of the flat sheets. Using the methods of this invention, a worker striving to develop better hollow fiber membranes may elect to experiment with and produce flat sheets because of their ease in manufacturing, while being confident that the results can be translated easily to hollow fiber structures. The benefits realized in terms of time, cost of materials, manpower, and capital costs can be significant.

Another advantage of the present invention is that expensive or relatively toxic extraannular fluids can be used to good effect employing only minimum amounts of liquid. Cost savings are again realized not only in purchasing materials but also in the subsequent disposal of waste.

Also, the use of the spinnerette assembly of the present invention offers a major improvement in manufacturing technique in that it enables one to obtain the desirable effect of high solvent content in the extraannular fluid, if desired, followed by rapid removal of the solvent on entering an aqueous quench bath. Thus, previously unobtainable membrane structures and control over structural features is now possible by the methods of the invention.

Accordingly, the role of the air gap in controlling the solvent evaporation time is largely eliminated. Thus, the distance between the spinnerette face and the quench bath becomes relatively unimportant. This distance becomes an important part of the process only if the kinetics of membrane formation is sufficiently slow or if the composition of the extraannular fluid compromises the structure of the membrane itself.

Furthermore, although the intra- and extraannular fluids may serve both to initiate the thermal phase separation and to quench the resulting microporous structure, the stationary washing/quenching bath still serves to partially quench and preserve the membrane structure. As mentioned above, the strong solvent is also washed away from the membrane in the wash process along with other contaminants. Preferably, the bath temperature should also be kept above the LCST of the dope composition.

Phase boundaries may naturally serve to define process temperatures. Typically, a temperature of about 10°C above the LCST is employed in producing relatively isotropic microporous membranes with pores in the range of 1 mm in diameter. Dopes can be maintained in the single phase region of the phase diagram (e.g., at 60°C) before reaching the spinnerette in the extrusion process, or equally useful, the dope may be caused to phase separate either in the dope lines or dope pot. The point up-stream of the spinnerette at which thermal phase inversion occurs does not seem to matter greatly, an observation which is contrary to the general teachings of the membrane art which teaches that dopes should be maintained in the single phase at all times until it emerges from the spinnerette. According to conventional wisdom, phase separation in any part of the spinning apparatus should be avoided because it normally results in irreproducible and inferior membrane properties (e.g., defects, closed cell matrix structure, and the like). It has been found, however, that the important consideration is that the dope attains a temperature equal to or greater than the LCST before or very soon after contacting quench media. Thus in the case of flat sheet casting, polymer dopes are preferably extruded in the single phase and quenched in about 80°-90°C water. For hollow fibers, both the quench bath and spinnerette are preferably maintained at about 80°-90°C.

The hollow fibers which emerge from the quench bath are preferably further washed "on-line" in a series of Godet baths. A Godet bath consists of a pair of parallel drums partially submerged in a wash tank. The fibers are wrapped several times around these rotating drums, increasing the length of time that the fibers reside in the bath. Godet and wash bath temperatures are also important considerations with regard to membrane permeability and fine structure. For example, a commonly used washing temperature after the fiber has been taken off the spin line is about 60°C (for approximately one day). However, if washing is done at room temperature instead such fibers may exhibit reduced hydraulic permeability compared with fibers washed at 60°C. These reduced $L_p$ fibers, when subsequently washed at 60°C or above show equivalent permeability to fibers which are washing at 60°C immediately after being produced in the spin line.

In extruding preferred PES/PEO dope compositions a number of bore injection (intraannular) fluids can be employed to good effect. These include: water, water/solvent (e.g., NMP) mixtures, pure solvents (e.g., NMP), water soluble polymer solutions (e.g., PVA), gas (e.g., nitrogen), humidified gas, various non-solvents and liquids which are immiscible with components in the dope, according to one's ultimate goal. When making relatively isotropic microporous membranes with surface pores in the range of 1 $\mu$m, the preferred bore injection fluid is a water/NMP mixture.

Similarly, the extraannular fluid composition and flow rate can both be varied over a very wide range in order to manipulate the nature of the surface pores, or the degree of symmetry in the sub-structure. Again, for substantially isotropic structures, the intra- and extraannular fluids may be the same.

It is important to remember that one skilled in the membrane art can take full advantage of the methods and apparatus described herein to make various adjustments and combinations in spinning conditions (e.g., nonsolvent as the intraannular fluid while using strong solvent as the extraannular fluid, operating at various temperatures, etc.) to produce a wide variety of hollow fibers with pore sizes ranging in the μm scale to tens of angstroms in size.

### 5.3.4. Charge-Modified PES Membrane Surfaces

Commercial 2.5 cm polysulfone membrane discs (Tuffryn membrane filter, HT-200, Gelman Sciences, Ann Arbor, MI) are washed with cold water three times to remove the water washables, and three times more with isopropanol to remove the isopropanol extractables. The membranes are then washed overnight to remove acetonitrile extractables. The membranes are then activated with 10% EGDGE in 0.6 N NaOH for 4 hours to form pendant covalently bound epoxide groups. After washing the excess EGDGE with cold deionised water, some of the membranes are immersed in 2% HEC in 0.6 N NaOH, others in 2% carboxymethyl cellulose (CMC) (Cellulose Gum-CMC, Type 7LF, Hercules Inc., Wilmington, Del.) in 0.6 N NaOH, and still others in 2% poly(ethyleneimine) (PEI) (Molecular weight 70,000, Aldrich Chemicals, Milwaukee, Wisconsin). A control extracted Tuffryn membrane is also placed in deionised water. All the membranes are then placed in a water bath at 60°C for 16 hours to commence covalent grafting. The membranes are then washed with hot deionized water at 60°C in order to remove the unreacted water soluble polymers. The membranes are next tested for their ability to bind and elute human IgG using the standard protocol given in Example 6.4.2 of the copending U.S. Application Serial No. 07/258,406 application of Azad and Goffe entitled, "Process for the Covalent Surface Modification of Hydrophobic Polymers and Articles Made Therefrom," and incorporated in its entirety by reference herein, except for the following differences. The human IgG is dissolved in the phosphate buffered saline with Tween 80 which has been diluted 1:10. The washings are also carried out using the same diluted buffer. The elution protocol is the same as given in said Example 6.4.2 of the above-identified U.S. Application. The results of the elution are given in Table I.

TABLE I

| Human IgG Eluted from HEC, CMC and PEI Treated Tuffryn Commercial Polysulfone Membrane, and Controls | |
|---|---|
| Sample | mg IgG eluted/mL [a] membrane volume |
| 1. HEC coated | 0.21 |
| 2. CMC coated | 0.86 |
| 3. PEI coated | 1.27 |
| 4. Control, uncoated | 0.24 |
| Results average of two samples. | |

The results suggest that charge-modified membranes (entries 2 and 3 in Table I) are better able to bind human IgG than a simple hydrophilic (HEC) surface.

### 5.3.5. Preparation of Modules Containing Modified Hollow Fiber Membranes

PES/PEO hollow fiber membranes (Batch No. 2300-6) are made as described in Example 6.8. Approximately 100, 457,20 mm (18 inch) fiber membranes are placed in a two liter beaker. The membranes are then washed in the normal manner with hot water at 95°C for 16 hours, and then with 5N NaOH at 95°C for 16 hours in order to maximize the surface functional end groups. Samples from the NaOH treated fiber are saved for analysis. The remaining fibers are then activated with EGDGE and grafted with HEC once (1X HEC) as described above. After washing with hot water, the fibers are divided into two groups. The first group is washed in cold water, while the second group is washed in acetonitrile. The second group of fibers is then transferred to cold water. Both groups of fibers are then given a second grafting of HEC, washed in hot water to remove unreacted HEC, and finally washed in cold water and acetonitrile as described above. This procedure is repeated once more, in order to produce a thrice HEC grafted (3X HEC) PES fiber. The samples are washed again in water and acetonitrile. Samples are saved at each stage of treatment for the determination of hydroxyl group concentration and non-specific binding, as well as permeability and Protein A coupling/human IgG binding and elution. The results of hydroxyl group and bovine serum albumin non-specific binding are given in Table II.

TABLE II

| Total Hydroxyl Group Concentration (OH Conc.) and Non-Specific Binding (NSB) of PES/PEO Hollow Fibers (2300-6) after Various Surface Treatments | | | | |
|---|---|---|---|---|
| Sample | -OH Conc. | NSB | -OH Conc. | NSB |
| I. After NaOH Treatment | 2.3 | 1650 | | |
| II. HEC Coated | Water-Washed Only | | Acetonitrile-Washed | |
| 1. Once coated | 17.3 | 67 | 21.2 | 62 |
| 2. Twice coated | 24.6 | low* | 22.2 | low* |
| 3. Thrice coated | 28.1 | low* | 29.3 | low* |
| III. Control Membrane Hydrophilic Durapore (0.22 μm) | 103.2 | low | | |

* The NSB was very low and was difficult to assign a quantitative value. The -OH concentrations are expressed as umol - OH/mL membrane volume and the NSB values are in ug monomeric BSA/mL membrane volume.

The fibers (2300-6) have an initial permeability in the 900 x $10^{-4}$ cm$^3$/N.s (900 x $10^{-9}$ cm$^3$ /dyne-sec) range before coating the HEC. After grafting three times with HEC, the permeabilities are still in the 290 to 300 x $10^{-4}$ cm3/N.s (290 to 300 x $10^{-9}$ cm$^3$/dyne-sec) range. This result again shows the efficiency of the present invention in generally limiting the covalent grafting onto the surface layers, while not plugging the pores of the membrane.

The fibers which have been coated three times with HEC, with acetonitrile washings in between, are then FMP activated, in a beaker, and dried in air. A hollow polysulfone module is packed with 0.5 mL of the membranes. Recombinant Protein A is then coupled to the contents of the module. The module is then tested for the ability to t e up human IgG from the phosphate buffer solution containing IgG. After loading of human IgG and washing off the unbound IgG, the module elutes 4.0 mg of IgG, giving a membrane capacity of 8.0 mg IgG/mL membrane volume.

### 5.3.5.1. Immunoaffinity Purification of Factor VIII (FVIII)

Two hundred and sixty, 558,80 mm (22 inch) PES/PEO hollow fiber membranes (Batch No. 2400-5) prepared according to the procedure described in Example 7.6 of the copending U.S. Application serial No. 07/258,406 and with a hydraulic permeability of 500 x $10^{-4}$ cm3/N.s (500 x $10^{-9}$ cm$^3$/dyne-sec), are soaked in two liters of hot 95°C water for 16 hours. The fibers are then autoclaved with steam at 121°C for 15 min. They are then soaked in acetonitrile at room temperature for 16 hours, washed with cold water and treated with 0.6 N NaOH containing 10% EGDGE for 4 hours. After washing the excess EGDGE with cold deionized water, the fibers are soaked in 0.6 N NaOH containing 2% HEC at 75°C for 3 hours and then washed with hot deionized water at 55°C (to remove the unreacted HEC and excess NaOH). The fibers are then treated with FMP and air dried. The hydraulic permeability of the FMP-activated fibers is 138.$10^{-4}$ cm3/N.s (138 x 10-9 cm3/dyne-sec) with an average mean pore diameter of 0.30 mm as measured by a Coulter-Porometer. The fibers also have a hydroxyl concentration of 32.4 mmol/mL membrane. A hollow fiber module with an internal volume of 1.5 mL, is then made by packing several fibers treated as above into a polysulfone module. The final fiber membrane volume is 0.5 mL.

A NaHCO$_3$ buffer solution (pH 8.1, 15 mL) containing 0.3 mg of anti-FVIII antibody/mL is prepared by diluting anti-FVIII antibody (received as ascites fluid ESWF 7 from American Diagnostic, New York, NY, and purified using a Protein A column) with bicarbonate buffer (prepared according to Example 6.4.1 of copending U.S. Application Serial No. 07/258,406), concentrating the resulting solution by ultrafiltration, and diluting the concentrated antibody to the appropriate concentration. The antibody solution is then recirculated at room temperature for 16 hours through the fiber module using a peristaltic pump. The loosely bound antibody is removed by washing the module with sodium bicarbonate buffer. Unreacted FMP groups are extinguished using the procedure described for Protein A in Example 6.4.1 of the copending U.S. Application Serial No. 07/258,406, except for the use of the pump to recirculate solvents and reagents. The module containing covalently attached antibody is then tested for its ability to pick up FVIII as described below.

FVIII concentrate (1.3 U/mg protein, Hyland Laboratories, Inc., California) is diluted to 0.76 U/mL with 0.015 $\underline{M}$ citrate buffer (pH 7.0) containing 0.15 $\underline{M}$ NaC1. The diluted buffer solution is passed once through the fiber membrane module at a flow rate of 2 mL/min. A total of 22.9 mL (17.3 U) of buffer is passed through the module. The filtrate is saved for protein content analysis. The device is then washed with a buffer containing 0.015 $\underline{M}$ sodium citrate and 0.15

$\underline{M}$ NaC1 (pH 7.0) until the absorbence at 280 nm of the washings is negligible indicating that no more loosely bound protein is coming off the module (ca. 30 mL). The bound FVIII is then eluted with a buffered solution containing 1 $\underline{M}$ KI, 1 $\underline{M}$ lysine, 20 $\underline{mM}$ imidazole, and 5 $\underline{mM}$ CaCl$_2$ (pH 6.5). The filtrate and eluate are then assayed separately for FVIII:C activity using Stratchrom FVIII:C Anti-hemophilic Factor Chromogenic Assay (Diagnostica Stago, 6 ter, rue Denis Papin, 92600 Asnieres, France). The filtrate is found to contain 3.3 U, indicating that the 81% of the applied FVIII is retained by the module. The eluate fraction (3.8 mL) contained a total of 8.0 U of FVIII:C activity corresponding to an overall recovery of 46% FVIII:C activity. The balance of the FVIII initially applied is assumed to be in the buffer washings. Specific FVIII:C activities of starting material (1.3 U/mg protein) and eluted FVIII (150 U/mg protein) are determined based on FVIII activity and protein concentration as measured by the Lowry protein assay, and yielded a purification factor of 115. The above procedure is not optimized and could, doubtless, be improved by lowering the flow rate, recirculating the buffered protein solution, or changing the buffer constituents, for example.

It is understood that the invention described and claimed herein is not limited to the immunoaffinity purification of FVIII as the ligate. The isolation and purification of other ligates, especially those of biological significance, by methods similar to those described above are within the scope of this invention. Examples of ligates that may be purified by immunoaffinity and biospecific recognition include, but are not limited to, tissue plasminogen activator, human coagulation factor IX, hormones, interleukins, other human and mammalian proteins, and others described previously in Section 5.

5.3.6. Modification of Commercial Flat Sheet and Hollow Fiber Membranes

Commercial polysulfone flat sheet and hollow fiber membranes are modified as follows to demonstrate the general applicability of this invention.

The 0.2 μm polysulfone hollow fiber membrane (Model CFP-2-E-4, AG Technology Corp., Needham, Mass.) is removed from the microfiltration module, and washed for two weeks with isopropanol in order to remove isopropanol extractables. The commercial 0.45 μm flat sheet polysulfone membrane (HT-450 Tuffryn, 25 mm diameter, Product No. 66221, Gelman Sciences, Ann Arbor, Michigan) is first washed with water to remove water extractibles, and overnight with isopropanol to remove isopropanol extractables. A part of the membranes are also washed overnight for 16 hours to remove acetonitrile extractables.

Part of the membranes from each treatment is then grafted with HEC as described earlier after first treating with 10% EGDGE in 0.6 N NaOH for 16 hours. The samples are then washed with hot water and saved for hydroxyl group determination and BSA-NSB. Table XV of the copending U.S. Application of Azad and Goffe, gives the results of the -OH group concentration and BSA-NSB of these membranes at various stages of the surface treatment. All the measurements are carried out in one experimental matrix with the commercial control membrane.

5.4. Membrane Process and Apparatus Specification

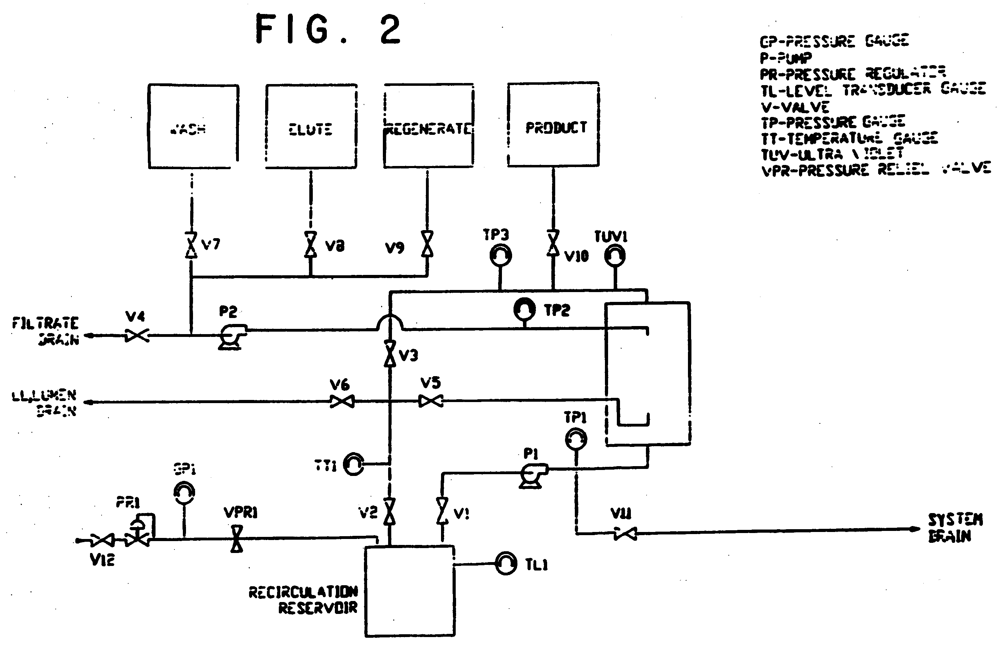

The apparatus can be configured at a scale required to achieve the operation steps. Four operation steps are preferred: (1) LOAD; (2) WASH; (3) ELUTE and (4) REGENERATE. The principles of scale-up will be described below. This will be followed by a detail description of the operating steps in conjunction with the schematic diagrams presented in FIGS. 1 and 2.

Scale-up of the membrane process is based on the constraints of mass transfer and pressure drop. The relationship of variables used for constraints on mass transfer is defined by equation 1. This expression is used to define the system scale or module size based on: 1) the knowledge of the volume of fluid to be processed per day, Q: 2) morphology of the porous membrane structure; 3) diffusivity of the target molecule; and 4) value of the Peclet number for favorable capture. Equation 1 becomes

$$V_0 = \frac{Q_L * t_D}{Pe} \qquad\qquad \text{Equation 2}$$

In this design equation, Pe can be specified as 0.01, $t_D$ is given as $L_D^2/D$, and $Q_L$ is the LOAD step flow rate based on Q. he result of this calculation is a value for the membrane void volume, $v_0$, where:

$$V_m = V_o/\text{Porosity fraction} \qquad\qquad \text{Equation 3}$$

$V_m$ is the volume occupied by the membrane. Given a membrane with defined wall thickness, internal diameter (or width) and length, the frontal surface area of the membrane required is defined.

Pressure drop constraints result from the need to provide uniform perfusion of the membrane down the length of the channel. To achieve this it is preferred to constrain the ratio of transmembrane to translumen pressure drop to less than 0.10. This constraint can further change dimension of the flow channel and/or membrane surface area.

Three scales of module apparatus can be defined with the design process outlined above. This does not preclude other process scale that can range over any volume that is to be processed in a required period of time.

Membrane module size can be defined as 1.5 mL, 30 mL or 150 mL, referring to the approximate interior volume in the module itself. Hollow fiber membrane typically occupies 1/3 of the total module volume. A 1.5 mL laboratory scale module apparatus according to this invention can process 5 Liters of feedstream per day, corresponding to a monoclonal antibody product yield in the 200 mG range. A 30 mL pilot scale membrane module apparatus according to this invention can process 100 liters of feedstream daily. The 150 mL process scale membrane apparatus according to this invention will process 500 liters daily according to this invention. Similar membrane process apparatus according to this invention will process 500 liters daily according to this invention. Similar membrane process apparatus can be designed to process 2,000 to 10,000 liters per day. It is within the scope of this invention that the interior volume of the module can range from 1 mL or lower to 10 liters or higher, preferably 1.5 mL to about 1000 mL.

The membrane process apparatus at any scale is designed to occupy a space smaller than required for column technology that would process equivalent volumes of feedstream. Process control and data aquisition are preferably automated. The operator interface is a power-on switch, start/stop buttons and computer interface that provides access to system control for changing operating conditions. The computer and/or microprocessor based controller actuates solenoid (pneumatic) valves and ramp pumps up to desired flow rates.

Events are timed open loop intervals or decisions based on any combination of absorbance UV value(s), pressure(s), conductivity, reservoir level(s) or temperature. The decision can be based on either magnitude or time based derivative and can be used to start and stop steps or switch valves.

Filtrate and recirculation flow rates are preferably controlled by peristaltic pumps. The pump speeds are preferably set on an operator interface but can be based on pressure feed back control.

The systems can be operated under sterile conditions using disposable tubing and pinch valves in the case of the smaller scale membrane process apparatus and Steam-In-Place technology in the case of the process scale membrane process system apparatus. The large scale process system is pressurized during all phases of the operation. This is achieved by adjusting pressure in the head space of the reservoirs. The system is operated under positive pressure to insure a net positive suction head for the filtrate pump.

Tanks with cooling jackets and recirculation loop preferably have temperature probes.

Tank levels are preferably monitored with capacitance probes.

Materials in contact with the fluid streams are preferably non-reactive corrosion resistant and biocompatible, such as polypropylene, silicone, polysulfone, polycarbonate, teflon, norphene, C-Flex (thermoplastic elastomer), and electropolished 316L stainless steel. Flexible tubing is connected by barbs to luers and fittings and used in the smaller scale system. The peristaltic pumps in both lab and large scale systems use flexible tubing. Tubing in the large scale system is preferably stainless steel.

In the 1.5 and 30 ml scale membrane affinity absorption systems (FIG. 1) the apparatus is preferably operated in the sequence which follows (wherein "V" is a valve means and "P" is a pump means):

Step 1. LOAD

Purpose of this step is to load the membrane structure with the target protein. Cross Flow Filtration is used in this step. The filtrate pump regulates flow rate and volumetric throughput.

A. Recirculation Flow - 50 to 100 ml/min is preferred for the 1.5 ml module, while 250 to 2000 ml/min is preferred for the 30 ml module.

P1 supplies flow to Affinity Modules
V4 is open
V8 directs flow to V9
V9 directs flow to Recirculation reservoir

B. Filtrate Flow - 2 to 20 ml/min for the 1.5 ml module is preferred; 40-400 ml/min for the 30 ml module.

P2 regulates flow through the membrane in the Affinity Module
V5 directs flow to V6
V6 directs flow to P2
V7 directs flow to Filtrate Drain

Step 2. WASH

Purpose of this step is to remove unwanted and unbound proteins from the Affinity Module and membrane.
P1 stops and V4 closes

A. Shell Flush

    P2 pumps buffer to Shell side of module
    V1 directs flow to V6
    V6 directs flow to P2
    V7 directs flow to Affinity modules
    V5 directs flow to Shell Drain

B. Lumen Wash

    V4 is open, V5 stops flow to drain
    P2 pumps buffer to Shell side of module
    V1 directs flow to V6
    V6 directs flow to P2
    V7 directs flow to Affinity modules
    V4 directs flow to V8
    V8 directs flow to Lumen Drain

Step 3. ELUTE

The purpose of this step is to provide for uniform elution of the target molecule from the membrane structure. In the Product Elution part of this step it is preferred to use Back Flushing.

A. Shell Flush - Same as above (in IIA), V2 provides buffer.
B. Product Elution - Same as Lumen Flush (in IIB) except:

    V8 directs flow to V9
    V9 directs flow to Product Reservoir

STEP 4. REGENERATE

The purpose of this step is to re-equilibrate the membrane to conditions suitable for loading.

A. Shell flush - same as wash, V3 provides buffer
B. Lumen wash - same as wash, V3 provides buffer

In a Large scale system (FIG. 2) the apparatus is preferably operated in the sequence which follows (similarly "V" is a valve means and "P" is a pump means):

STEP 1. LOAD

Purpose of this step is to load the membrane structure with the target protein. Cross Flow Filtration is used in this step. The filtrate pump is reversible and regulates flow and volumetric throughput. V1, V2, V3 and V4 are open. All other valves are closed.

Recirculation Flow - 5 to 10 1/min, preferred for one 150 ml membrane module

    P1 supplies flow to Affinity Modules

Filtrate Flow -0.2 to 2 1/min, typical.

    P2 Regulates flow through the membrane in the Affinity Module
    V4 directs flow to Filtrate Drain
    PR1 Supplies positive bias pressure to the system and assures net positive suction head to P2.

STEP 2. WASH

Purpose of this step is to remove unwanted and unbound proteins from the Affinity Module and membrane.
P1 stops and V1, V2, V3 and V4 closes

A. Shell Flush

P2 pumps buffer to Shell side of module
V5, V6 and V7 are opened
V5 directs flow to V6
V7 directs flow to P2
V6 directs flow to Drain

B. Lumen Wash

V5 is closed, V3 is opened
P2 pumps buffer to Shell side of module
V3 directs flow to V6
V6 directs flow to Drain

STEP 3. ELUTE

The purpose of this step is to provide for uniform elution of the target molecule from the membrane structure. In the product elution part of this step it is preferred to us Back Flushing is used.

A. Shell Flush - Same as above (in IIA), V8 provides buffer
B. Product Elution - Same as Lumen Flush (in IIB) except:

V3 is closed, V10 is open
V10 directs flow to Product Reservoir

STEP 4. REGENERATE

The purpose of this step is to provide for uniform regeneration of the membrane hollow fibers. After regeneration of the membrane hollow fibers, the cycle can be repeated.

A. Shell Flush - same as above (in 2A), V9 supplies buffer
B. Lumen Flush - same as above (2B) wash but again, V9 supplies the buffer

The examples above detail the use of crossflow filtration in the load step and membrane backflush in the wash, elute and regeneration steps. It is also within the scope of this invention to eliminate backflushing by flowing solutions in the wash, elute and regeneration steps entirely in the direction of the load step operation.

Additional preferred operational specifications specific to the large or process scale apparatus are described below. The process scale affinity apparatus should be self-draining and free of cracks or crevices. These specifications are scalable to either the 1.5 or 30 ml membrane process and apparatus.

The preferred performance specifications for the large scale apparatus are set forth in Table III.

TABLE III

| | |
|---|---|
| IgG Inlet Concentration | 0.05±0.02 mg/ml |
| IgG Outlet Concentration | 0.75±0.25 mg/ml |
| IgG Binding Capacity | 10±4 mg/ml MV |
| Total IgG Binding Capacity | 450±240 mg |
| IgG Purity | >80% |
| IgG Specificity | <1 mg serum protein/ml MV |
| Protein A Binding Capacity | 10 mg/ml MV |
| Total Protein A Binding | 450 mg |
| Cycles/Module | 75 |

The preferred operating flow rates, pressures and temperatures are specified in Table IV.

TABLE IV

| Pressures | Min | Max | Base Case |
|---|---|---|---|
| static operating | 1 | 10 | 0,34 bar (5 psig) |
| Transmembrane | 1 | 3 | 0,14 bar (2 psig) |
| Temperatures | | | |
| Product & Recycle Tanks | | 4° ± 2°C | |
| Inlet Buffers | | 4° ± 2°C | |
| Loop Gradient | | 2°C max | |
| Flow Rates | Min | Max | Base Case |
| Filtrate | 1 | 6 | 3.5 L/min |
| Recycle | 10 | 20 | 15 L/min |
| Fraction Perfused | 10% | 30% | 25% |
| Buffers | 1 | 5 | 3 L/min |

The volumes preferably utilized in the large scale apparatus are set forth below in Table V.

TABLE V

| | Min. | Max. | Base Case |
|---|---|---|---|
| Adsorption | 4 | 14 | 9 L |
| Wash | 1 | 5 | 3 L |
| Elution | 1 | 5 | 3 L |
| Regeneration | 1 | 5 | 3 L |
| Adsorption/8 hour shift | 80 | 560 | 360 L |
| Buffer/8 hour shift | 80 | 480 | 280 L |
| Adsorption/Batch | 300 | 1050 | 675 L |
| Adsorption/Week | 1500 | 9450 | 4725 L |

The large scale affinity apparatus module specifications are preferably as follows:

| | |
|---|---|
| Volume | - 150 mL |
| Number per system | - 4 |
| Material of Construction | - Polysulfone |
| Pressure | - 2,04 bar (30 psig) |
| Temperature Limit | - 126°C |
| connections | - 1-1/2 Triclamp |

The preferred fiber specifications for use in the affinity apparatus are as follows:

| Effective Length | 13±0.5cm |
|---|---|
| Total Length | 15±0.5cm |
| ID | 1000±5.0μm |
| OD | 1600±5μm |
| Porosity | 0.8 ± .05<br>- .35 |
| Pore Size | 0.6μm + 1.0<br>-0.3 |
| Membrane Volume/Fiber | 0.159±0.012mL |
| Fibers/Module | 285±15 |
| Total Membrane Volume/Module | 45±5ml |
| Surface Area/module | 1163±155cm$^2$ |
| Dynamic Pressure Drop | 0,14-0,48 bar (2-7 psig) |

The use of the affinity apparatus in carrying out an affinity purification procedure is illustrated by the following example.

6.0. Examples

6.1. Membrane Affinity Purification of Fibronectin From Blood Plasma

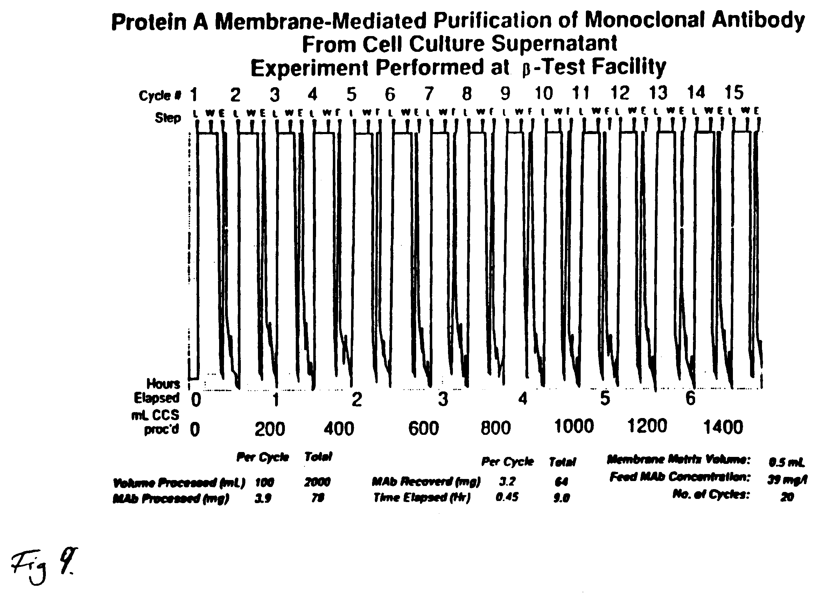

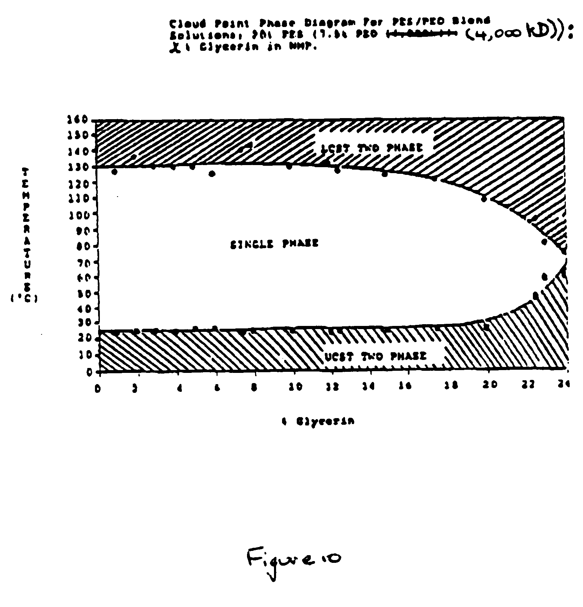

Purification of fibronectin (FN) from blood plasma was carried out in accordance with the general method described by Miekka, et al. (Thromb. Res. 27, 1-14, 1982) using flat sheet HPC-coated PES/PEO membranes containing immobilized porcine skin gelatin (Sigma Type I). Membrane disks were inserted in a plastic filter holder (2.5 cm diameter), which was placed in the experimental apparatus illustrated in Figure 6. Convection of plasma and buffer solutions through the membrane device was controlled by a peristaltic pump. Absorbance of effluents at 280 nm was monitored using a UV detector installed upstream of a fraction collector. Transmembrane pressure was measured using an on line pressure transducer. In a typical affinity membrane-based FN purification run (Figure 7), 4.6 mL of blood plasma was loaded onto a 0.34 mL membrane device. After washing the membrane with equilibration buffer and 1 M NaCl, FN was eluted by lowering the pH to 5.5. All steps were performed at a flow rate of 1.0 mL/min, corresponding to a membrane residence time of approximately 15 seconds. Analysis of filtrate fractions for FN using a commercial immunoturbidimetric assay (Boehringer Mannheim) indicated that essentially all of the FN was removed from the feed plasma. The amount of purified FN isolated was 0.34 mg, or 26% of the starting FN.

Samples of purified FN and FN-depleted plasma were analyzed by SDS-PAGE on 8-25% gradient gels (Pharmacia) visualized by silver staining (Figure 8). The FN band near 230,000 daltons evident in the starting plasma (b) and the FN standard (E) is nearly absent in the FN-depleted plasma (C). The low pH eluate (D) is nearly pure FN, containing trace amounts of serum albumin. The percent purity of the eluted product was determined by comparing the concentration of FN determined by immunoturbidimetry with the protein concentration determined by A280 (based on a value of 12.8 for the absorbence of a 1% FN solution). The value obtained for affinity membrane purified FN is 93%.

The effect of plasma loading rate on efficiency of FN uptake was examined using the experimental setup described above. Plasma (8.8 mL; 25.9 membrane volumes) was passed through the membrane device at flow rates of 0.3, 1.0, 3.0 and 6.0 mL/min. (corresponding to residence times of 51, 15, 5.1 and 2.6 seconds, respectively). At all of the flow rates tested, it was found that at least 6.0 mL (18 membrane volumes) of FN-depleted plasmas could be collected prior to emergence of appreciable quantities of unbound FN.

6.2. Immunoaffinity Purification of Factor VIII (F.VIII)

A 1.5 mL hollow fiber affinity module containing covalently attached monoclonal antibody against F.VIII concentrate diluted to 0.76 U/mL in 0.015 M citrate, 0.15 M citrate, 0.15 M NaCl pH 7.0 at a flow rate of 2 mL/min. After loading of 22.9 mL (17.3 U), the device was washed with 0.015 M citrate, 0.15 M NaCl pH 7.0 until the absorbence at 280 nm of the washings was negligible (30 mL). The bound F.VIII was then eluted with a buffered solution containing 1M KI, 1M lysine, 20 mM imidazole, 5 mM CaCl$_2$ pH 6.5. Pooled filtrates and eluates were assayed for F.VIII:C activity. The filtrates were found to contain 3.3U, indicating that the device had captured 80% of the applied F.VIII. The eluate fraction contained 8.0 U.F.VIII:C activity for an overall recovery of F.VIII activity of 46%. Specific F.VIII:C activities of starting material and eluted F.VIII were determined based on F.VIII activity and protein concentration as measured by the Lowry assay. Comparison of these values yielded a purification factor of 115. These data are summarized in Table VI, below:

TABLE VI

| Fraction | F.VIII Activity U/mL | Protein mg/mL | Spec Act mg/mL | Purif. factor |
|---|---|---|---|---|
| Starting material | 0.76 | 0.60 | 1.3 | -- |
| Eluate | 2.1 | 0.014 | 150 | 115 |

Commercial F.VIII concentrate was obtained from Hyland Laboratories, Inc. MAB against F.VIII complex was obtained from American Diagnostica. This monoclonal antibody was produced in ascites fluid and purified on Protein-A Sepharose gel; its designation is American Diagnostica ESVWF1, indicating that the monoclonal antibody recognizes the von Wildebrandt's portion of the Factor VIII complex.

6.3. Immunoaffinity Purification of F. VIII from Blood Plasma

A. 1.5 mL affinity module containing monoclonal antibody against F. VIII similar to the device described in the above example was loaded with untreated blood plasma at a flow rate of 2 mL/min. After loading 50.6 mL plasma (50.6 Units F. VIII:C activity), the device was washed and eluted as described above. Pooled filtrates and eluates were assayed for F. VIII:C activity as described above. The filtrates were found to contain 8.1 Units (16% of activity loaded), indicating that 84% of the applied F. VIII had been captured. The eluate fraction contained 16.9 Units (33% of activity loaded). Overall recovery of F. VIII:C activity was 49%. Specific F. VIII:C activities of the eluate fraction and the starting plasma were determined based on the F. VIII:C assay results and protein concentrations are measured by the Lowry assay. Comparison of initial and final specific activity values yields a purification factor of 56. These results are summarized below:

| Fraction | F.VIII Activity (U/ml) | Protein (mg/mL) | Specific Activity (U/mg) | Purif. factor |
|---|---|---|---|---|
| Starting plasma | 1.0 | 72 | 0.014 | -- |
| Eluate | 3.2 | 4.1 | 0.78 | 56 |

6.4. Protein A Membrane-Mediated Capture of Human IgG

The ability of affinity membranes to efficiently capture target proteins at loading rates corresponding to residence times of few seconds was assessed using Protein A as the affinity ligand and human IgG as the target protein. Flat sheet affinity membranes containing immobilized Protein A were inserted in a plastic filter holder (2.5 cm diameter) and placed in the experimental setup shown in Figure 6. The total membrane matrix volume contained in the device was 0.2 mL; the total static IgG capacity of the device was 0.76 mg (3.8 mg/mL membrane matrix volume).

The bind/elution cycle was composed of (i) passage of 11.6 mL of a solution of 0.05 mg/mL human IgG (Sigma) in phosphate buffered saline pH 8.0 (PBS) through the device at a preselected flow rate (corresponding to an IgG loading of 2.9 mg/mL membrane matrix volume--76% of the static capacity), (ii) washing of the device with PBS until the absorbance of the washings at 280 nm was negligible and (iii) elution of the captured IgG with 0.1 M citrate buffer pH 3.0. The efficiency of capture during the IgG loading step was assessed in the flow rate range from 0.25 to 5.0 mL/min. All other steps were performed at 0.5 mL/min. The IgG capture efficiency at each loading rate was assessed by determining the amount of IgG in the citrate eluate fractions using the Lowry protein assay. Table VII below indicates that at an IgG loading of 76% of the device static capacity and at flow rates as high as 5.0 mL/min (corresponding to a fluid residence time of 1.8 sec), at least 75% of the applied IgG was captured.

TABLE VII

| Run# | Loading Rate (matrix | | Fluid Res. Time (s) | IgG (mg/mL matrix) | Eluted % of applied |
|---|---|---|---|---|---|
| | (mL/min) | (vol/min) | | | |
| 1 | 0.25 | 1.3 | 36 | 2.6 | 90 |
| 2 | 0.50 | 2.5 | 18 | 2.6 | 90 |
| 3 | 2.5 | 12.5 | 3.6 | 2.4 | 80 |
| 4 | 5.0 | 25.0 | 1.8 | 2.1 | 75 |

*Protein A Membrane-Mediated IgG Binding and Elution Effect of IgG Loading Rate*

Matrix volume: 0.2 mL IgG applied per cycle 2.9 mg/mL matrix
Feed IgG conc.: 0.05 mg/mL Total IgG cap. (static): 3.8 mg/mL matrix

## 6.5. Protein A Mediated Capture of a Monoclonal Antibody From Cell Culture Supernatant

The ability of affinity membranes to efficiently capture target proteins from complex mixtures at high loading rates was assessed using Protein A as the affinity ligand and a monoclonal antibody as the target protein. In this case, the Protein A was immobilized in 1.5 mL hollow fiber module, which was then attached to the automated lab-scale affinity system described in Section 5. The feed solution was a serum-free cell culture supernatant containing 0.055 mg/mL of the desired monoclonal antibody along with contaminating proteins that were either produced by the cells or initially present in the media. Loading was performed in the crossflow mode with a feed flow rate of 50 mL/min and filtrate flow rates ranging from 2.6 to 16 mL/min (9.2 to 1.5 seconds). The amount of cell culture supernatant loaded per cycle corresponded to either 2.9±0.1 mg monoclonal antibody per mL matrix volume (about 30% of the static capacity of the device for the target monoclonal antibody) or 9.0±0.4 mg/mL matrix volume (about 30% of the static capacity). The effect of filtration rate and monoclonal antibody loading on capture efficiency was determined by collecting the filtrates generated during each cycle and assaying for the presence of the monoclonal antibody using the HPLC method outlined by Hammen et al. (BioChromatography 3, 54-59, 1988). Comparison of the filtrate monoclonal antibody concentration with that of the feed solution yielded the percent capture efficiency. The results obtained in Table VIII indicate that at device loadings as high as 100% of the device static capacity and at fluid residence as low as 1.5 sec, over 80% of the applied target protein was captured.

TABLE VIII

| Amount loaded (mg/mL matrix) | Flow rate (mL/min) | $t_c$ (s) | Capture efficiency (%) |
|---|---|---|---|
| 2.9±0.1 | 2.6 | 9.2 | 92.2 |
| | 5.3 | 4.5 | 91.9 |
| | 10.7 | 2.2 | 90.8 |
| | 16.0 | 1.5 | 89.6 |
| 9.0±0.4 | 2.6 | 9.2 | 85.9 |
| | 5.3 | 4.5 | 86.1 |
| | 10.7 | 2.2 | 84.0 |
| | 16.0 | 1.5 | 83.3 |

*Protein A Membrane-Mediated Capture of MAb from Cell Culture Supernatant*

### 6.6 Protein A Membrane Mediated Purification of Monoclonal Antibody From Cell Culture Supernatant

Purification of a target protein from a complex mixture in a rapid, multicyclical fashion was demonstrated using a 1.5 mL hollow fiber module containing immobilized Protein A attached to the automated lab-scale affinity system described in Section 5.1. The target protein was murine monoclonal antibody which was present in cell culture supernatant at a concentration of 0.039 mg/mL. Each bind/elution cycle comprised the following:

| | |
|---|---|
| LOAD: | Recirculation Flow--50 mL/min |
| | Filtrate Flow--12 mL/min |
| | Amount loaded per cycle--100 mL; 7.8 mg |
| | MAb/mL matrix |
| LUMEN WASH: | Buffer-PBS pH 8.0 |
| | Flow rate--20 mL/min |
| | Volume--40 mL |
| SHELL ELUTE: | Buffer-0.1m citrate pH 3.0 |
| | Flow rate--10 mL/min |
| | Volume--10 mL |
| LUMEN ELUTE: | Buffer--0.1m citrate pH 3.0 |
| | Flow rate--4.0 mL/min |
| | Volume--24 mL |
| SHELL REGEN: | Buffer--PBS pH 8.0 + 0.% Tween 80 |
| | Flow rate--10 mL/min |
| | Volume--10 mL |
| LUMEN REGEN: | Buffer--PBS pH 8.0 + 0.1% Tween 80 |
| | Flow rate--10 mL/min |
| | Volume--20 mL |

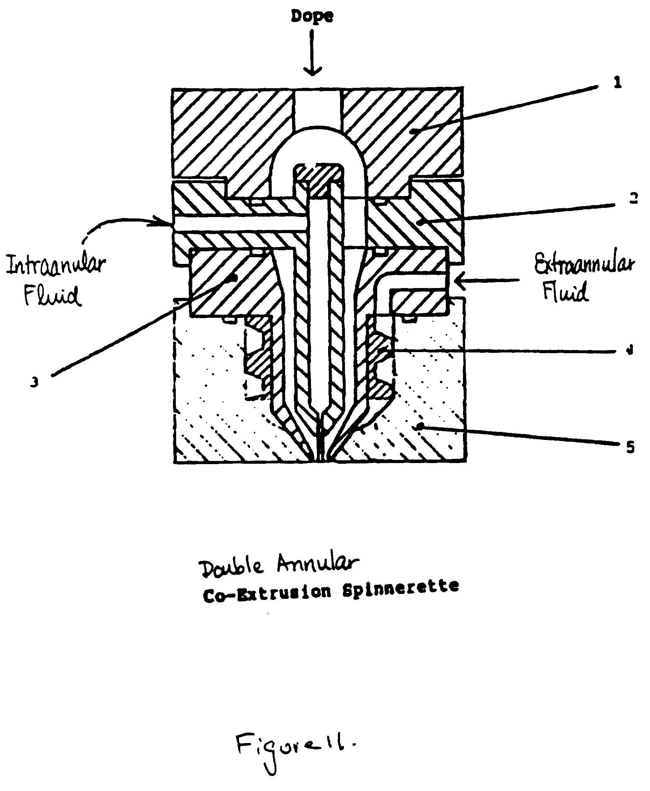

The time for each individual cycle was 27 minutes. A total of 20 cycles were performed in which 2000 mL of cell culture supernatant was processed (Figure 9). The amount of monoclonal antibody recovered was estimated by measuring the absorbance of the eluates at 280 nM and calculating the product concentration using $\varepsilon_{280} = 1.1$. Of the 78 mg monoclonal antibody initially present in the cell culture supernatant, 64 mg was recovered in a volume of 300 mL-indicating a product recovery of 82% and a concentration factor of 5.4. SDS-PAGE analysis of samples generated in this manner indicated that all contaminating proteins were removed from the purified product and that filtrates were essentially depleted of target monoclonal antibody.

### 6.7. Dope Preparation and Polymer Drying Procedure

The dope preparation procedure involves weighing and pretreating the two polymers employed in the blend. PES, which is also known as Victrex (by ICI America, grade 5200P, obtained in 15 kg bags), is dried in an oven at 150°C for 3 h then allowed to cool to 60°C for several hours more. The total time of heating in the oven is not less than about 24 h. PEO (Polyox 301, MW 4000000 D, by Union Carbide Corp., obtained in 63,42 kg (140 lb) drums) is pretreated in a vacuum oven at room temperature for about 24 h. Care is taken not to leave the pretreated polymers in the open air for extended periods before adding them to the mixer.

Both NMP (i.e., N-Pyrol, Cat. No. 1-3-72755/000 & 5-72, by GAF Chemicals Corp., obtained in 242,55 liter (55 gal) drums) and glycerin (by Baxter Scientific Product Group, Mallincrodt, catalog # 5092-4, Analytical Reagent) are used as received, but precautions are taken to minimize the uptake of atmospheric moisture by adding them to the mixer immediately after removal from the respective containers. Their containers should be closed tightly when they are not in use.

### Mixing Procedure

NMP (2812 g) and glycerin (1128 g) are pre-mixed in a 3,8 liter (1 gal) container before adding them to a Ross (model PVM2) mixer at room temperature. The Ross mixer is fitted with a source for purging with nitrogen. The inert atmosphere is maintained over all liquids until the PEO has been added. On applying pre-mixed NMP/glycerin to the Ross mixer two of the mixing blades are started: the anchor blade at 135 rpm, and the disperser blade at 3,500 rpm. PEO (360 g) is added while mixing at room temperature over the period of about one minute. A 500 gram portion of NMP is then added to make a total of 3312 gram NMP in the dope. At this point the disperser blade is switched off and Mokon heat exchange unit is set at 1200C. After 3 h of mixing, the PES (1200 g) is added over the space of 2-3 minutes, and the temperature is noted with the anchor blade maintained at 135 rpm. After an additional 18 h, a steady decrease in temperature is initiated by setting the Mokon at 60°C. Within about 1.5 h of making this temperature change, the dope usually attains a temperature of about 75 +5°C, at which time a vacuum is gradually applied to de-gas the mixture. Full vacuum is usually achieved within 15 min and is maintained for a further 5 min. The mixer is then switched off while continuing to de-gas. A vacuum is maintained for 1-2 min longer before introducing nitrogen to re-establish atmospheric pressure in the mixing vessel at 60°C.

This preparative procedure typically results in a dope viscosity of about 100 (±20) Pa.s (100,000 (+ 20,000) cps) at 60°C. However, occasional deviations from the norm occur which do not appear to result in any ill effects in membrane properties. Such a dope has phase boundaries at about 78°C (LCST) and about 57°C (UCST), as shown in Figure 10.

6.8. Hollow Fiber Spinning of Relatively Isotropic Microporous Membranes Primarily For Affinity Applications

A dope is prepared as outlined above and is found to have a viscosity at 60°C of 123 Pa.s (123,000 cps). This dope is extruded through the co-extrusion spinnerette schematically represented in Figure 11. Spinnerette temperature is maintained at 80°C throughout the duration of the experiment. Other fixed parameters preferably include:

- . dope pump speed --- about 70 rpm
- . quench bath temperature --- about 90°C
- . quench bath composition --- Deionized (DI) water
- . intraannular fluid composition --- about 70% NMP:30% DI water (v/v)
- . extraannular fluid composition --- about 70% NMP:30% DI water (v/v)
- . intraannular fluid flow rate --- about 30.2 (±1.2) ml/min.
- . first and second godet bath temperatures ---about 42.5 (±2.5) °C

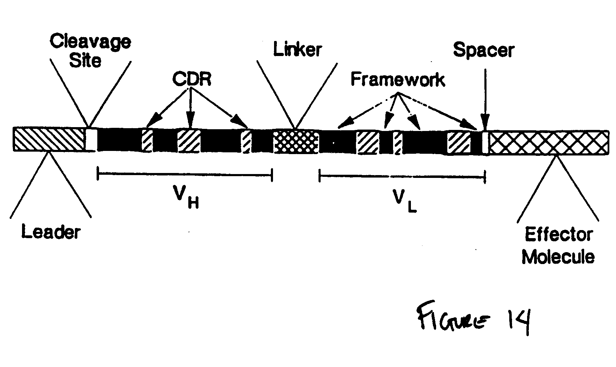

Other parameters which may also be varied in this experiment are: air gap or spinnerette height above the quench bath (which results in a change in fiber take-up rate or the rate of fiber production in linear feet per minute) and extraannular fluid flow rate. The latter is varied from zero to 66 mL/min with spinnerette heights ranging from 76,20-117,80 mm (3-7 inches). Note that the spin line contains only two godet baths. Results from this experiment are shown in Figure 13 and in the table of data inserted as part of the figure for fibers designated 2500-1 through 11. A very pronounced dependence of membrane hydraulic permeability (Lp) for DI water on extraannular fluid flow rate is evident. Fiber 2500-1, with zero outer annular fluid flow is equivalent to a fiber produced with a conventional tube-in-orifice spinnerette.

This dependence of the Lp on the extraannular fluid flow rate is reproducible using a portion of the same dope as that used in Figure 13 (See Table IX for fibers designated 2600-1 through 9).

TABLE IX

| Hydraulic Permeabilities of Hollow Fibers as a Function of Extraannular Flow | | | | | |
|---|---|---|---|---|---|
| Fiber Sample | LP (X10$^{-4}$ cm3/N.sec) | Extraannular Flow (mL/min) | Air Gap (inches) (1 inch = 2.54 cm) | ID ($\mu$m) | OD ($\mu$m) |
| 2600-1 | 13 | 0 | 2.5 | 1000 | 1600 |
| 2600-2 | 210 | 10 | 2.5 | 930 | 1520 |
| 2600-3 | 13 | 0 | 2.5 | 1040 | 1500 |
| 2600-4 | 296 | 10 | 2.5 | 1020 | 1610 |
| 2600-5 | 33 | 0 | 2.5 | 1040 | 1620 |
| 2600-6 | 337 | 10 | 2.5 | 950 | 1500 |
| 2600-7 | 23 | 0 | 2.5 | 1000 | 1580 |
| 2600-8 | 309 | 37 | 2.5 | 1000 | 1560 |
| 2600-9 | 9 | 0 | 3.5 | 1000 | 1600 |

Fiber sample 2600-6 is examined by electron microscopy and pores in the 1-3 pm are observed on the two surfaces. overall pore size distribution in the matrix of the approximately 300 Am fiber wall varies within the range of about 1-2 orders of magnitude, but the great majority of the pores are within 1 order of magnitude of each other in size. The results indicate that this membrane is an example of a substantially skinless relatively isotropic microporous membrane. By contrast, fiber 2600-5 (which is made minus the extraannular fluid) is a far more anisotropic microporous membrane structure.

Fiber 2600-6 and others from this batch are then autoclaved, hydrophilized by grafting a composite coating onto its entire internal and external surfaces, and successfully employed in affinity and bioseparation experiments. The 1.5 mL hollow fiber modules containing 0.5 mL membrane volume are covalently functionalized to attach Protein A ligand. The IgG capacity for such modules is determined to be 7 and 8 mg/mL (for modules #13 and #14 respectively), while non-specific binding of fetal calf serum proteins have a capacity of about 1 mg/mL. The non-specifically bound proteins are easily washed off the membrane surfaces due to the hydrophilicity. High loading capacities are achievable with this fiber because of the 300 micron wall thickness, while the high hydraulic permeability for DI water is retained both after hydrophilization and chemical activation. For example, modules #13 and 14 have Lp values of $314 \times 10^{-4}$ and $191 \times 10^{-4}$ cm3/N.sec ($314 \times 10^{-9}$ and $191 \times 10^{-9}$ cm3/dyne sec), respectively. After Protein A ligand is applied the Lp value is $149 \times 10^{-4}$ cm3/N.sec ($149 \times 10^{-9}$ cm3/dyne sec) for module #14.

A wide range of microfiltration and ultrafiltration applications can be addressed by these membranes (with or without further surface modification or hydrophilization), where the relatively low protein binding surfaces minimize fouling and plugging of the matrix. Of particular interest is the use of the relatively isotropic microporous fibers (e.g., fiber 2600-6) for cell separation. This separation of cells from accompanying liquid can be achieved at very high fluxes without catastrophic decay in hydraulic permeability, which is typically observed for commercially available hollow fibers. Some examples of such cell separation applications include: clarification of cell broth and conditioned media (where affinity binding and clarification may be combined to reduce the number of unit operations in protein purification), and separation of blood cells for medical applications.

The range of extraannular flow rate demonstrated in this example (i.e., FIG. 13), with the dope and extraannular fluid combinations spanning over five orders of magnitude difference in viscosity, is not possible with the modular spinnerette disclosed in U.S. Patent No. 4,493,629.

## 6.9. Effect of Wash Time and Temperature

Hollow fibers are produced with an extraannular fluid from a dope having a viscosity of 108 Pa.s (108,000 cps) at 60°C. Spin parameters employed in the experiment are detailed in Table X. After washing in water at about 60°C overnight the average hydraulic permeability for DI water (based on three test modules) was determined to be $348 \times 10^{-4}$ cm$^3$/N.s ($348 \times 10$-9 cc/dyne sec) for fiber sample 3000-1.

Similarly, another dope is prepared with a viscosity of 118 Pa.s (118,000 cps) at 60°C and hollow fibers 3100-1 through -5 are produced under substantially the same conditions as fiber 3000-1 above. All these fiber samples are produced under identical spinning conditions to provide large quantities of fibers with the same Lps. Some of these membranes (collection batch 3100-2 to -5) are employed in a series of washing experiments in which both washing time and temperature are varied. The data is presented in Table XI and reveals a trend of increasing Lp with increasing washing time, particularly at 20°C, and with increasing temperature. These numbers suggest that a convenient washing temperature and time for effective post-spin line washing could be about 60°C overnight.

## TABLE X

### Process Parameters for Hollow Fiber Membrane 3000-1

|  | Temperature (°C) | Dope Pot | Dope Line | Bore Line | Spin-erette | Quench Bath | Godet Bath 1 | Godet Bath 2 |
|---|---|---|---|---|---|---|---|---|
|  | 64 | 80.4 | 73.4 | 80.8 | 92 | 28 | 28 | |

| Flow Rate cm3/min) | Intraannular Fluid | Extraannular Fluid |
|---|---|---|
|  | 65 | 12 |

Take Up
Rate (mm/min)[a]: 5,18
(17 ft/min)

| Lp (X10$^{-4}$ cm3/N·sec) | Measurement 1 | 2 | 3 | Ave | std. dev. |
|---|---|---|---|---|---|
|  | 348 | 337 | 360 | 348 | 9 |

Also, fiber production rate

TABLE XI

| Results of Varying Wash Time and Temperatures After Taking the Fibers Off-Line | | |
|---|---|---|
| Wash Temp (°C) | Wash Time (h) | Lp (X10$^{-4}$ cm3/N.sec)[a] |
| 20 | 4-5 | 277 |
| | 18-19 | 327 |
| | 92 | 412 |
| | 115.5 | 431 |
| | 139 | 432 |
| 40 | 15 | 379 |
| | 24 | 463 |
| | 87 | 478 |
| 55 | 8 | 497 |
| | 23 | 463 |
| 75 | 8 | 494 |
| | 23 | 485 |
| 90 | 8 | 436 |
| | 23 | 439 |
| Average from 2-7 test modules | | |

## 6.10. Modification of Hollow Fiber Membranes

PES/PEO hollow fiber membranes are prepared as described above. Approximately 100 457,20 mm (18-inch) fiber membranes are placed in a two-liter beaker. The membranes are then washed with hot water at 950C for 16 h and then with 5 N NaOH at 95°C for 16 h in order to increase the number of available funtional end groups. The fibers are then allowed to react with an 0.1 N NaOH solution containing 10 wt% EGDGE at room temperature for 4 h. The fibers are then isolated and preferably washed with fresh cold water to remove unreacted EGDGE and excess base. Next, the samples are immersed in a 0.6 N NaOH solution containing 2 wt% hydroxyethyl cellulose (HEC, Natrosol 250 JR, Aqualon Company, Wilmington, Delaware, USA) and heated to 60°C for 16 h. Afterwards, the fibers are rinsed in 60°C water to remove unbound components. This material is referred to as 1X HEC material. Additional layers of HEC may be incorporated onto the fibers by repeating the EGDGE and HEC treatments to give, as desired, 2X HEC, 3X HEC, 4X HEC, etc. materials.

The 3X HEC material is selected (although another HEC-layered material can be chosen) for further modification. The fibers are placed in a beaker and treated with an excess of an acetonitrile solution of 2 wt% 2-fluoro-1-methylpyridinium p-toluenesolfonate (FMP, Aldrich Chemical company, Milwaukee, Wisconsin, USA) and 1 wt% triethylamine, at room temperature for About 15 min. The fibers are then washed with fresh acetontrile and air-dried.

A hollow polysulfone module with an internal volume of 1.5 mL is then packed with the FMP-activated fibers to give a final fiber membrane volume of 0.5 mL. Recombinant Protein A is then coupled to the contents of the module by recirculating a buffered solution of the protein through the module with the aid of a peristaltic pump. Any loosely-bound protein is washed off with sodium bicarbonate buffer and any unreacted FMP-activated groups are consumed by treating the fibers with 1 wt% mercaptoethanol in 30 $\underline{mM}$ sodium bicarbonate buffer. The module is then charged with a phosphate-buffered solution of human IgG. After washing off any loosely-bound material, the module is found to elute 4.0 mg of IgG, giving a membrane capacity of 8.0 mg IgG/ml membrane volume.

Similarly, an aqueous solution of anti-Factor VIII (anti-FVIII, American Diagnositics, New York, New York, USA) in sodium bicarbonate buffer is recirculated through a module packed with FMP-activated fibers, as above, for 16 h at room temperature. Unreacted FMP-activated groups are extinguished similarly. A total of 22.9 mL of a FVIII solution (0.76 U/mL) in 0.015 $\underline{M}$ citrate buffer (pH 7.0) containing 0.15 $\underline{M}$ NaCl is then passed through the module at a rate of 2 mL/min. The module is then washed with a buffer of 0.015 $\underline{M}$ citrate and 0.15 $\underline{M}$ NaCl until the absorbence at 280 nm of the washings is negligible, indicating the absence of protein in solution. The FVIII is then eluted with a suitable solution (e.g., an aqueous solution of 1 $\underline{M}$ potassium iodide, 1 $\underline{M}$ lysine, 20 $\underline{nM}$ imidazole, and 5 $\underline{mM}$ calcium chloride, pH 6.5). The eluate (3.8 mL) is found to contain 8.0 U of FVIII:C activity using a Stratchrom® FVIII:C Anti-Hemophilic Factor chromogenic Assay (Diagnostica Stage, 6 ter, rue Denis Papin, 92600 Asnieres, France), indicating a recovery of 46% based on the initial amount of FVIII loaded. Also, a purification factor of about 115 is achieved by this unoptimized procedure based on the specific activity of the initial FVIII solution.

### 6.11. Attachment of Anti-Factor IX Monoclonal Antibody to a 1.5 ml Module and Immunopurification of Factor IX

Approximately 2.0 mg of anti-Factor IX monoclonal antibody was attached to a 1.5 mL FMP-activated hollow fiber affinity membrane module. A semipurified preparation of Factor IX (FIX) obtained from processed plasma was prepared for affinity purification in a Hepes buffered diluent containing 30 millimolar Mg$^{++}$ to a calculated value of 10 Units of Factor IX per milliliter. A loading sample of 30 milliliters was applied to the anti-Factor IX module with a filtrate flow rate of 1.0 milliliter per minute. The loading volume was calculated to contain approximately 1.5 times the Factor IX that could be maximally bound to the module.

Factor IX was eluted from the module by applying a Hepes buffered aqueous solution without Mg$^{++}$ in a total volume of 12 milliliters. The eluted fractions were measured for total protein by optical density at 280 nm using an extinction coefficient for pure Factor IX of 1.3 for 1 mg per milliliter in aqueous solution. Experimental results revealed a recovery average of 0.36 mg total protein from the 1.5 ml module as measured by the extinction coefficient for Factor IX. (See Table XII for results)

TABLE XII

| EXPT # | FRACTION | AMT RECOV'D (mg) | AMT FACTOR IX AVAILABLE |
|---|---|---|---|
| 1 | ELUATE | 0.44 | 1.5 |
| 2 | ELUATE | 0.28 | 1.5 |

### 6.12. Synthesis of a Multifunctional Protein

In this example, a bifunctional protein was created by fusing a nucleic acid sequence encoding a biosynthetic digoxin binding site that mimics the antigenic determinant of the IgG2$_{\alpha,\lambda}$ monoclonal antibody 26-10 with a sequence encoding the FB fragment of protein A. The FB fragment, which functions as the second active region, was present as a leader in this construction. The native amino acids comprising the last 11 amino acids of the FB fragment bonded to an Asp-Pro dilute acid cleavage site was employed as a spacer. The FB binding domain of the FB fragment consists of the immediately preceding 43 amino acids which assume a helical configuration. The N-terminal portion of this domain can be covalently coupled to membrane surface groups that have been activated by introducing superior leaving groups using any of the reagents described in the specification.

### 6.12.1. Model Binding Site

The digoxin binding site of the IgG$_{2\alpha,\lambda}$ monoclonal antibody 26-10 has been analyzed by Mudgett-Hunter and colleagues (unpublished). The 26-10 V region sequences were determined from both amino acid sequencing and DNA sequencing of 26-10 H and L chain mRNA transcripts (D. Panka, J.N. & M.N.M., unpublished data). The 26-10 antibody exhibits a high digoxin binding affinity [$K_o = 5.4 \times 10^9$ M$^{-1}$] and has a well-defined specificity profile, providing a baseline for comparison with the biosynthetic binding sites mimicking its structure.

### 6.12.2. BABS Design

Crystallographically determined atomic coordinates for Fab fragments of MOPC-603 were obtained from the Brookhaven Data Bank. Inspection of the available three-dimensional structures of Fv regions within their parent Fab fragments indicated that the Euclidean distance between the C-terminus of the V$_H$ domain and the N-terminus of the V$_L$ domain is about 35 Å. Considering that the peptide unit length is approximately 3.8 Å, a 15 residue linker was selected to bridge this gap. The linker was designed so as to exhibit little propensity for secondary structure and not to interfere with domain folding. Thus, the 15 residue sequence (Gly-Gly-Gly-Gly-Ser)$_3$ was selected to connect the $V_H$ carboxyl- and $V_L$ amino-termini.

Binding studies with single chain binding sites having less than or greater than 15 residues demonstrate the importance of the prerequisite distance which must separate $V_H$ from $V_L$; for example, a (Gly$_4$-Ser)$_1$ linker does not demonstrate binding activity, and those with (Gly$_4$-Ser)$_5$ linkers exhibit low activity compared to those with (Gly$_4$-Ser)$_3$ linkers.

### 6.13. Gene Synthesis

Design of the 744 base sequence for the synthetic binding site gene was derived from the Fv protein sequence of 26-10 with the aid of a commercially available computer program which performs combined reverse translation and restriction site searches ("RV.exe" by Compugene, Inc.). The known amino acid sequences for $V_H$ and $V_L$ 26-10 polypeptides were entered, and all potential DNA sequences which encode those peptides and all potential restriction sites were analyzed by the program. In addition, the program selected codons preferred by E. coli.

Significant flexibility in $V_H$ and $V_L$ design is possible because the amino acid sequences are determined at the DNA level, and the manipulation of DNA can be accomplished easily.

For example, if a master framework gene that facilitates CDR exchange is desired, a synthetic DNA can be designed with the aid of a computer program like Compugene having the following generic structure:

$$R_1\text{-}FR_1\text{-}X_1\text{-}FR_2\text{-}X_2\text{-}FR_3\text{-}X_3\text{-}FR_4\text{-}R_2$$

where $R_1$ and $R_2$ are restricted ends which are to be ligated into a vector, and $X_1$, $X_2$, and $X_3$ are DNA sequences whose function is to provide convenient restriction sites for CDR insertion. The use of unique, 6-base restriction sites adjacent the FR borders allows synthetic nucleotide sequences encoding CDRs from a desired monoclonal to be inserted easily. A six base site is preferred, but where six base sites are not possible, four or five base sites are used. These sites, if not already unique, are rendered unique within the gene by eliminating other occurrences within the gene without altering necessary amino acid sequences. Preferred cleavage sites are those that, once cleaved, yield fragments with sticky ends just outside of the boundary of the CDR within the framework. However, such ideal sites are only occasionally possible because the FR-CDR boundary is not an absolute one, and because the amino acid sequence of the FR may not permit a restriction site. In these cases, flanking sites in the FR which are more distant from the predicted boundary are selected.

In the present example, the gene coding for $V_H$ was assembled from 46 chemically synthesized oligonucleotides, prepared on a Biosearch Model 8600 DNA synthesizer (San Rafael, CA). All polynucleotides synthesized were 15 bases long, except for terminal fragments (13 to 19 bases) that included cohesive cloning ends. Between 8 and 15 overlapping oligonucleotides were enzymatically ligated into double stranded DNA, cut at restriction sites suitable for cloning (NarI, XbaI, SalI, SacII, SacI), purified by PAGE on 8% gels, and cloned in pUC which was modified to contain additional cloning sites in the polylinker. The cloned segments were assembled stepwise into the complete gene mimicking $V_H$ by ligations in the pUC cloning vector.

The gene mimicking 26-10 $V_L$ was assembled from 12 long synthetic polynucleotides ranging in size from 33 to 88 base pairs, also prepared in automated DNA synthesizers (Model 6500, Biosearch, San Rafael, CA; Model 380A, Applied Biosystems, Foster City, CA). Five individual double stranded segments were made out of pairs of long synthetic oligonucleotides spanning six-base restriction sites in the gene (AatII, BstEII, PpnI, HindIII, BglII, and PstI). In one case, four long overlapping strands were combined and cloned. Gene fragments bounded by restriction sites for assembly that were absent from the pUC polylinker, such as AatII and BstEII, were flanked by EcoRI and BamHI ends to facilitate cloning.

The linker between $V_H$ and $V_L$, encoding (Gly-Gly-Gly-Gly-Ser)$_3$, was cloned from two long synthetic oligonucleotides, 54 and 62 bases long, spanning SacI and AatII sites, the latter followed by an EcoRI cloning end. The complete single chain binding site gene was assembled from the $V_H$, $V_L$, and linker genes to produce a construct, corresponding to aspartyl-prolyl-$V_H$-(linker)-$V_L$, flanked by EcoRI and PstI restriction sites.

The DNA sequence encoding the FB fragment of protein A was cloned into the EcoR1 site flanking the upsteam end of the BABS gene. The trp promoter-operator, starting from its SspI site, was assembled from 12 overlapping 15 base oligomers, and the MLE leader gene was assembled from 24 overlapping 15 base oligomers. These were cloned and assembled in pUC using the strategy of assembly sites flanked by cloning sites. The final expression plasmid was constructed in the pBR322 vector. Intermediate DNA fragments and assembled genes were sequenced by the dideoxy method. The completed fused gene set forth in Figure 15 was then expressed in E. coli.

### 6.14. Protein Expression

The high expression of these bifuntional proteins in E. coli results in their accumulation in refractile or inclusion bodies in the cell cytoplasm. The proteins can be harvested by disrupting the cells by sonication or by French press. In this example, proteins were harvested by sonication. After sonication, inclusion bodies were collected by centrifugation, and dissolved in 6 M guanidine hydrochloride (GuHCl), 0.2 M Tris, and 0.1 M 2-mercaptoethanol (BME), pH 8.2. The protein was denatured and reduced in the solvent overnight at room temperature. Size exclusion chromatography was used to purify fusion protein from the inclusion bodies. A Sepharose 4B column (1.5 X 80 cm) was run in a solvent of 6 M GuHCl and 0.01 M NaOAc, pH 4.75. The protein solution was applied to the column at room temperature in 0.5-1.0 ml amounts. Fractions were collected and precipitated with cold ethanol. These were run on SDS gels, and fractions rich in the recombinant protein (approximately 34,000 D) were pooled. This offers a simple first step for cleaning up inclusion body preparations without suffering significant proteolytic degradation.

For refolding, the protein was dialyzed against 100 ml of the same GuHCl-Tris-BME solution, and dialysate was diluted 11-fold over two days to 0.55 M GuHCl, 0.01 M Tris, and 0.01 M BME. The dialysis sacks were then transferred to 0.01 M NaCl, and the protein was dialyzed exhaustively before being assayed by RIA's for binding of $^{125}$I-labelled digoxin. The refolding procedure can be simplified by making a rapid dilution with water to reduce the GuHCl concentration to 1.1 M, and then dialyzing against phosphate buffered saline (0.15 M NaCl, 0.05 M potassium phosphate, pH 7, containing 0.03% $NaN_3$), so that it is free of any GuHCl within 12 hours. Products of both types of preparation showed binding activity.

This protein with an FB leader and a fused BABS is bifunctional; the BABS domain binds digoxin and the FB binds the Fc regions of immunoglobulins. To demonstrate this dual and simultaneous activity several radioimmunoassays were performed.

Properties of the binding site were probed by a modification of an assay developed by Mudgett-Hunter et al. (J. Immunol. (1982) 129:1165-1172; Molec. Immunol. (1985) 22:477-488), so that it could be run on microtiter plates as a solid phase sandwich assay. Binding data were collected using goat anti-murine Fab antisera (gAmFab) as the primary antibody that initially coats the wells of the plate. These are polyclonal antisera which recognize epitopes that appear to reside mostly on framework regions. The samples of interest are next added to the coated wells and incubated with the gAmFab, which binds species that exhibit appropriate antigenic sites. After washing away unbound protein, the wells are exposed to $^{125}$I-labelled (radioiodinated) digoxin conjugates, either as $^{125}$I-dig-BSA or $^{125}$I-dig-lysine.

The data are plotted in Figure 16A, which shows the results of a dilution curve experiment in which the parent 26-10 antibody was included as a control. The sites were probed with $^{125}$I-dig-BSA as described above, with a series of dilutions prepared from initial stock solutions, including both the slowly refolded (2) and fast diluted/quickly refolded (3) single chain proteins. The parallelism between all three dilution curves indicates that gAmFab binding regions on the BABS molecule are essentially the same as on the Fv of authentic 26-10 antibody, i.e., the surface epitopes appear to be the same for both proteins.

Experimental data on digoxin binding yielded binding constants in the range of $10^8$ to $10^9$ $M^{-1}$. The parent 26-10 antibody has an affinity of 5.4 X $10^9$ $M^{-1}$. Inhibition assays also indicate the binding of $^{125}$I-dig-lysine, and can be inhibited by unlabelled digoxin, digoxigenin, digitoxin, digitoxigenin, gitoxin, acetyl strophanthidin, and ouabain in a way largely parallel to the parent 26-10 Fab. This indicates that the specificity of the biosynthetic protein is substantially identical to the original monoclonal.

In a second type of assay, Digoxin-BSA is used to coat microtiter plates. Renatured FB-BABS is added to the coated plates so that only molecules that have a competent binding site can stick to the plate. $^{125}$I-labeled rabbit IgG (radioligand) is mixed with bound FB-BABS on the plates. Bound radioactivity reflects the interation of IgG with the FB domain of the BABS, and the specificity of this binding is demonstrated by its inhibition with increasing amounts of FB, Protein A, rabbit IgG, IgG2a, and IgG1, as shown in Figure 16B.

The following species were tested in order to demonstrate authentic binding: unlabelled rabbit IgG and IgG2a monoclonal antibody (which binds competiviely to the FB domain of the BABS); and protein A and FB (which bind competively to the radioligand). These species are found to completely inhibit radioligand binding, as expected. A monoclonal antibody of the IgG1 subclass binds poorly to the FB, as expected, inhibiting only about 34% of the radioligand from binding. These data indicate that the BABS domain and the FB domain have independent activity.

## Claims

1. An apparatus for carrying out affinity separation comprising:

 (a) a porous hollow fiber membrane in association with a ligand;
 (b) means for enclosing said porous hollow fiber membrane;
 (c) means for providing a first fluid in intimate contact with one side of said enclosed porous hollow fiber membrane; and
 (d) exit means for directing into a container any fluid present on the side of said enclosed porous hollow fiber membrane opposite that side to which said first fluid is first provided according to the means recited in element (c);

 characterized in that it further comprises

(e) a second exit means for directing said first fluid into a second container;

and in that at least part of said fluid leaving from means (d) has originated from said first fluid of means (c), and further in that said membrane has a substantially isotropic microporous structure in all directions throughout the membrane, with pores large enough to permit the convective flow of macromolecule-containing solutions across the membrane.

2. The apparatus of claim 1, characterized in that said membrane has a mean pore size of at least 0.20 micrometers.

3. The apparatus of claim 1 or 2 characterized in that said membrane is at least about 200 micrometers in thickness.

4. The apparatus of anyone of claims 1 to 3 characterized in that said membrane is a hydrophobic membrane having a derivatized interfacial surface.

5. The apparatus of claim 4 characterized in that said membrane interfacial surface is derivatized by a linker moiety attached to said surface and to a ligand having a plurality of functional groups, to provide a product membrane with derivatized interfacial characteristics.

6. The apparatus of anyone of claims 1 to 3 characterized in that said membrane comprises a) a hydrophobic polymer having functionalizable chain ends exposed at the polymer surface; b) a macromolecule or/and a ligand capable of altering the surface properties of said hydrophobic polymer; c) a linker moiety covalently bridging a chain end of said hydrophobic polymer to said macromolecule or/and said ligand.

7. The apparatus of claim 6 characterized in that said hydrophobic polymer is selected from the group consisting of polysulfones, polyethersulfones, polyimides, polyarylene oxide , polyurethanes, polyetheretherketones , polycarbonates, polyesters, polyvinylhalides , and polyvinylidene polyhalides , derivatives, blends, mixtures or copolymers thereof.

8. The apparatus of claim 6 or 7 characterized in that said linker moiety is a molecule having a base group and at least two functional groups selected from the group consisting of epoxide, carbonyl, carboxyl, amine, halide, acid chloride, acid anhydride, sulfonyl, sulfonyl chloride, carboxylic acid, hydroxyl, and stable combinations thereof.

9. The apparatus of claim 8 characterized in that said base group of the molecule of the linker moiety is a hydrocarbon molecule.

10. The apparatus of anyone of claims 6 to 9 characterized in that said linker moiety incorporates a functional group selected from the group consisting of silicon, aluminum, tin, boron, phosphorus, sulfur and nitrogen.

11. The apparatus of claim 6 characterized in that said linker moiety has a molecular weight less than about 2000.

12. The apparatus of claim 6 characterized in that said macromolecule is hydrophilic.

13. The apparatus of claim 6 characterized in that said macromolecule is hydrophobic.

14. The apparatus of claim 6 characterized in that said macromolecule is amphoteric.

15. The apparatus of claim 6 characterized in that said macromolecule has a functional group capable of bearing a charge.

16. The apparatus of claim 6 characterized in that said macromolecule has a nonionic functional group.

17. The apparatus of claim 6 characterized in that said macromolecule is a biosynthetic antibody binding site.

18. The apparatus of claim 6 characterized in that said macromolecule is selected from the group consisting of natural polymer, synthetic polymer, derivatives, blends, mixtures, and copolymers thereof, surfactant, carbohydrate, lectin, polypeptide, polysaccharide, liposome, protein, glycoprotein, oligonucleotide, synthetic dye, natural dye, polynucleotide, derivatives, and mixtures thereof, monoclonal antibody, polyclonal antibody, antigen, receptor, enzyme substrate, enzyme, carrier protein, coagulation factor, cofactor, inhibitor, hormone, immunoglobulin, histone, plasmid, derivatives, and mixtures thereof, and polysilane, polysiloxane, polysulfonate, polycarboxylate, hydroxyalkylcellulose, dextran, diethylaminoethyldextran, carboxymethylcellulose, polyethyleneimine , polycarboxymethylethylene imine , polyvinylalchohol , derivatives, blends, mixtures, and copolymers thereof.

19. The apparatus of claim 6 characterized in that said macromolecule has an average molecular weight of about 0.2 to about 5,000 kilodaltons.

20. The apparatus of claim 6 characterized in that said membrane polymer further comprises multiple layers of macromolecules, which layers are held together by covalent bonds.

21. The apparatus of claim 6 characterized in that said ligand is covalently bound to said macromolecule(s).

22. The apparatus of claim 6 characterized in that said ligand has an ionizable functional group.

23. The apparatus of claim 6 characterized in that said ligand is selected from the group consisting of long chain aliphatic hydrocarbon silane, branched aliphatic hydrocarbon silane, aromatic hydrocarbon silane, and mixtures thereof, surfactant, carbohydrate, lectin, polypeptide, monosaccharide, polysaccharide, liposome, protein, glycoprotein, oligonucleotide, synthetic dye, natural dye, polynucleotide, derivatives, and mixtures thereof, monoclonal antibody, polyclonal antibody, antigen, receptor, enzyme, substrate, enzyme, carrier protein, glycoprotein, coagulation factor, cofactor, inhibitor, hormone, immunoglobulin, histone, plasmid, derivatives, and mixtures thereof, and natural Protein A, recombinant Protein A, avidin, biotin, heparin, lectin, animal cell surface receptor, plant cell surface receptor, bacterial cell surface receptor, and single strand nucleic acid.

24. The apparatus of claim 6 characterized in that the ligand is a biosynthetic binding protein.

25. The apparatus of claim 6 characterized in that said ligand is a binding site capable of binding a molecule selected from the group consisting of immunoglobulin G, immunoglobulin M, immunoglobulin A, immunoglobulin E, tissue plasminogen activator, human interleukin protein, blood coagulation factor, human chorionic gonadotropin, thyrotropic hormone, carcinoembryonic antigen, a-fetoprotein, transforming growth factor, and interferon.

26. The apparatus of claim 6 characterized in that said membrane comprises (a) polyethersulfone as the primary hydrophobic polymer component having functionalizable phenolic chain ends; (b) hydroxyalkylcellulose having hydroxyl functional groups; and (c) a linker moiety selected from the group consisting of ethylene glycol diglycidyl ether, 1,4-butanediol diglycidyl ether, epichlorohydrin, and chloroacetic acid, which linker moiety is able to covalently bridge a phenolic chain end of said polyethersulfone with at least one hydroxyl group of said hydroxyalkylcellulose.

27. The apparatus of claims 1 and 2 characterized in that said membrane is derivatized with a ligand comprising a single chain multi-functional biosynthetic protein expressed from a single gene derived by recombinant DNA techniques, said protein comprising:

   - a biosynthetic antibody binding site capable of binding to a preselected antigenic determinant and comprising at least one protein domain, the amino acid sequence of said domain being homologous to at least a portion of the sequence of a variable region of an immunoglobulin molecule capable of binding said preselected antigenic determinant and peptide bonded to;
   - a polypeptide effector protein having at least one functional group capable of selective covalent binding to said membrane.

28. The apparatus of claim 27 characterized in that said binding site comprises at least two domains connected by peptide bonds to a polypeptide connector sequence.

29. The apparatus of claim 28 characterized in that said two domains mimic a $V_H$ and a $V_L$ from a natural immunoglobulin.

30. The apparatus of claim 28 characterized in that the amino acid sequence of each of said domains comprises a set of complementarily determining regions ("CDRs") interposed between a set of framework regions ("FRs"), each of which is respectively homologous with at least a portion of CDRs and FRs from a said variable region of an immunoglobulin molecule capable of binding said preselected antigenic determinant.

31. The apparatus of claim 28 characterized in that said polypeptide connector sequence comprises amino acids which together assume an unstructured polypeptide configuration spanning a distance of at least 40 Å in aqueous solution.

32. The apparatus of claims 1 and 2 characterized in that multiple strands of said hollow fiber membranes are present.

33. The apparatus of claims 1 and 2 characterized in that said apparatus further comprises a second means for providing a second fluid into said enclosure means on the opposite side of said membrane than said first fluid is provided.

34. The apparatus of claim 33 characterized in that said second means for providing is alternatively a reversible pump capable of withdrawing a fluid or regulating the exit of fluid from said enclosure means.

35. The apparatus of claim 34 characterized in that said exit means of element (d) is closed.

36. The apparatus of claims 1 and 2 characterized in that the static operating pressure of said apparatus ranges up to about 1,02 bars (15 psig).

37. The apparatus of claims 1 and 2 characterized in that the transmembrane pressure of said apparatus ranges up to about 0,68 bar (10 psig).

38. The apparatus of claims 1 and 2 characterized in that the temperature of said first and second fluids ranges from about 2°C to about 37°C.

39. The apparatus of claims 1 and 2 characterized in that said apparatus has a volume inside of said means for enclosing said membrane of from about 1 mL to about 10 L.

40. The apparatus of claims 1 and 2 characterized in that said means for enclosing said membrane is corrosion resistant.

41. The apparatus of claims 1 and 2 characterized in that said means for providing is a pump.

42. A method for carrying out affinity purification of a ligate in a membrane system comprising:

(a) providing a ligate-containing feed liquid to a porous hollow fiber membrane associated with a ligand to said ligate, said feed liquid being under a pressure sufficient to cause a portion of said feed liquid to flow convectively and tangentially across an exterior surface of said membrane and to cause the remainder of said fluid to flow into and through said porous membrane whereby said ligate present in said feed liquid binds to said ligand and is thereby separated from said feed liquid ;
(b) washing said membrane with a buffer solution;
(c) providing an elution solution to one side of said membrane under a pressure sufficient to cause said elution solution to flow into and through said membrane and to effect the disassociation of any ligate-ligand bonds formed in step (a), whereby any ligate bound to said ligand is eluted with said elution solution; and
(d) separating said ligate in a purified form from said elution solution;

said membrane having a substantially isotropic microporous structure in all directions throughout the membrane with pores large enough to permit the convective flow of macromolecule-containing solutions across the membrane.

43. The method of claim 42 characterized in that said elution solution is provided to the opposite side of said membrane than where the feed liquid is provided, under sufficient pressure to cause said solution to flow into and through said membrane.

44. The method of claim 42 or 43 characterized in that said buffer solution is provided to the opposite side of said membrane than where the feed liquid is provided, under sufficient pressure to cause said solution to flow into and through said membrane.

45. The method of anyone of claims 42 to 44 characterized in that said ligate is selected from the group consisting of IgG, Factor VIII, fibronectin, and Factor IX and said ligand is respectively selected from the group consisting of Protein A, a monoclonal antibody to Factor VIII, porcine gelatine, and a monoclonal antibody to Factor IX.

**Patentansprüche**

1. Eine Vorrichtung zur Durchführung eines Affinitätstrennverfahrens, bestehend aus:

   (a) einer porigen Hohlfasermembran gekoppelt mit einem Liganden;
   (b) Einrichtung zur Einfassung besagter poriger Hohlfasermembran;
   (c) Einrichtung zum Einleiten eines ersten Fluids und Herbelführen eines engen Kontakts zwischen demselben und einer Seite von besagter eingefaßter poriger Hohlfasermembran; und
   (d) Einrichtung zum Ableiten von solchen Fluids in ein Behältnis, die sich auf der Seite von besagter eingefaßter poriger Hohlfasermembran befinden, die der Seite gegenüber liegt, auf der zunächst besagtes erstes Fluid entsprechend der unter (c) genannten Einrichtung eingeleitet wird;

   dadurch gekennzeichnet, daß sie desweiteren

   (e) eine zweite Einrichtung zum Ableiten von besagtem ersten Fluid in ein zweites Behältnis beinhaltet;

   und daß zumindest ein Teil des über Einrichtung (d) austretenden Fluids aus besagtem über Einrichtung (c) eingeleiteten Fluid stammt und daß desweiteren besagte Membran durch und durch und in allen Richtungen eine im wesentlichen isotrope mikroporige Struktur aufweist, wobei die Poren groß genug sind, um die Konvektionsströmung von Makromoleküle enthaltenden Lösungen durch die Membran zu gestatten.

2. Die Vorrichtung gemäß Anspruch 1, dadurch gekennzeichnet, daß besagte Membran eine mittlere Porengröße von mindestens 0,20 μm aufweist.

3. Die Vorrichtung gemäß Anspruch 1 oder 2, dadurch gekennzeichnet, daß besagte Membran eine Dicke von mindestens 200 μm aufweist.

4. Die Vorrichtung gemäß einem der Ansprüche 1 bis 3, dadurch gekennzeichnet, daß besagte Membran eine hydrophobe Membran ist, die eine derivatisierte Grenzflächenoberfläche besitzt.

5. Die Vorrichtung gemäß Anspruch 4, dadurch gekennzeichnet, daß besagte Grenzflächenoberfläche der Membran durch eine Linker-Hälfte derivatisiert wird, die an besagte Oberfläche sowie an einen Liganden mit einer Vielzahl von funktionellen Gruppen ankoppelt, um eine Produktmembran mit derivatisierten Grenzflächenelgenschaften hervorzubringen.

6. Die Vorrichtung gemäß einem der Ansprüche 1 bis 3, dadurch gekennzeichnet, daß besagte Membran folgendes umfaßt: a) ein hydrophobes Polymer mit funktionalisierbaren Kettenenden, die an der Polymeroberfläche freiliegen; b) ein Makromolekül und/oder einen Liganden, daß/der in der Lage ist, die Oberflächenelgenschaften von besagtem hydrophoben Polymer zu verändern; c) eine Linker-Hälfte, die das Ende einer Kette von besagtem hydrophoben Polymer mit besagtem Makromolekül und/oder mit besagtem Liganden kovalent überbrückt.

7. Die Vorrichtung gemäß Anspruch 6, dadurch gekennzeichnet, daß besagtes hydrophobe Polymer ausgewählt wird aus der Gruppe bestehend aus Polysulfonen, Polyethersulfonen, Polyimiden, Polyarylenoxid, Polyurethanen, Polyetheretherketonen, Polycarbonaten, Polyestern, Polylvinylhalogeniden und Polyvinylidenpolyhalogeniden bzw. daraus gewonnenen Derivaten, Blends, Gemischen oder Copolymeren.

8. Die Vorrichtung gemäß Anspruch 6 oder 7, dadurch gekennzeichnet, daß besagte Linker-Hälfte ein Molekül ist, das eine Basisgruppe und mindestens zwei funktionelle Gruppen umfaßt, die aus der Gruppe bestehend aus Epoxid-, Carbonyl-, Carboxyl-, Amin-, Halogenid-, Säurechlorid-, Säureanhydrid-, Sulfonyl-, Sulfonylchlorid-, Carboxylsäure-, Hydroxylgruppen und stabilen Verbindungen derselben ausgewählt werden.

9. Die Vorrichtung gemäß Anspruch 8, dadurch gekennzeichnet, daß besagte Basisgruppe das Moleküls der Linker-Hälfte ein Kohlenwasserstoffmolekül ist.

10. Die Vorrichtung gemäß einem der Ansprüche 6 bis 9, dadurch gekennzeichnet, daß besagte Linker-Hälfte eine funktionelle Gruppe beinhaltet, die aus der Gruppe bestehend aus Silikon, Aluminium, Zinn, Bor, Phosphor, Schwefel und Stickstoff ausgewählt wird.

11. Die Vorrichtung gemäß Anspruch 6, dadurch gekennzeichnet, daß besagte Linker-Hälfte ein Molekulargewicht von weniger als ca. 2000 hat.

12. Die Vorrichtung gemäß Anspruch 6, dadurch gekennzeichnet, daß besagtes Makromolekül hydrophil ist.

13. Die Vorrichtung gemäß Anspruch 6, dadurch gekennzeichnet, daß besagtes Makromolekül hydrophob ist.

14. Die Vorrichtung gemäß Anspruch 6, dadurch gekennzeichnet, daß besagtes Makromolekül amphoter ist.

15. Die Vorrichtung gemäß Anspruch 6, dadurch gekennzeichnet, daß besagtes Makromolekül eine funktionelle Gruppe aufweist, die in der Lage ist, eine Ladung zu tragen.

16. Die Vorrichtung gemäß Anspruch 6, dadurch gekennzeichnet, daß besagtes Makromolekül eine nichtionische funktionelle Gruppe aufweist.

17. Die Vorrichtung gemäß Anspruch 6, dadurch gekennzeichnet, daß besagtes Makromolekül eine biosynthetische Antikörperbindungsstelle ist.

18. Die Vorrichtung gemäß Anspruch 6, dadurch gekennzeichnet, daß besagtes Makromolekül ausgewählt wird aus der Gruppe bestehend aus natürlichem Polymer, synthetischem Polymer, daraus gewonnenen Derivaten, Blends, Gemischen und Copolymeren, oberflächenaktivem Stoff (Surfactant), Kohlenhydrat, Lectin, Polypeptid, Polysaccharid, Liposom, Protein, Glycoprotein, Oligonucleotid, synthetischem Farbstoff, natürlichem Farbstoff, Polynucleotid, daraus gewonnenen Derivaten und Gemischen, monoklonalem Antikörper, polyklonalem Antikörper, Antigen, Rezeptor, Enzymsubstrat, Enzym, Trägerprotein, Koagulationsfaktor, Cofaktor, Inhibitor, Hormon, Immunglobulin, Histon, Plasmid, daraus gewonnenen Derivaten und Gemischen, sowie Polysilan, Polysiloxan, Polysulfonat, Polycarboxylat, Hydroxyalkylcellulose, Dextran, Diethylaminoethyldextran, Carboxymethylcellulose, Polyethylenimin, Polycarboxymethylethylenimin, Polyvinylalkohol und daraus gewonnenen Derivaten, Blends, Gemischen und Copolymeren.

19. Die Vorrichtung gemäß Anspruch 6, dadurch gekennzeichnet, daß besagtes Makromolekül ein durchschnittliches Molekulargewicht von ca. 0,2 bis ca. 5.000 Kilodalton hat.

20. Die Vorrichtung gemäß Anspruch 6, dadurch gekennzeichnet, daß besagtes Membranpolymer deswelteren mehrere Lagen von Makromolekülen beinhaltet, wobei diese Lagen durch kovalente Bindungen zusammengehalten werden.

21. Die Vorrichtung gemäß Anspruch 6, dadurch gekennzeichnet, daß besagter Ligand über eine kovalente Bindung mit besagtem (besagten) Makromolekül(en) verknüpft ist.

22. Die Vorrichtung gemäß Anspruch 6, dadurch gekennzeichnet, daß besagter Ligand eine ionisierbare funktionelle Gruppe umfaßt.

23. Die Vorrichtung gemäß Anspruch 6, dadurch gekennzeichnet, daß besagter Ligand ausgewählt wird aus der Gruppe bestehend aus langkettigem aliphatischem Kohlenwasserstoffsilan, verzweigtkettigem aliphatischem Kohlenwasserstoffsilan, aromatischem Kohlenwasserstoffsilan sowie Gemischen daraus, oberflächenaktivem Stoff (Surfactant), Kohlenhydrat, Lectin, Polypeptid, Monosaccharid, Polysaccharid, Liposom, Protein, Glycoprotein, Oligonucleotid, synthetischem Farbstoff, natürlichem Farbstoff, Polynucleotid, daraus gewonnenen Derivaten und Gemischen, monoklonalem Antikörper, polyklonalam Antikörper, Antigen, Rezeptor, Enzymsubstrat, Enzym, Trägerprotein, Glycoprotein, Koagulationsfaktor, Cofaktor, Inhibitor, Hormon, Immunglobulin, Histon, Plasmid, daraus gewonnenen Derivaten und Gemischen, sowie natürlichem Protein A, rekombinantem Protein A, Avidin, Botin, Heparin, Lectin, tierischem Zelloberflächenrezeptor, pflanzlichem Zelloberflächenrezeptor, bakteriellem Zelloberflächenrezeptor und einsträngiger Nukleinsäure.

24. Die Vorrichtung gemäß Anspruch 6, dadurch gekennzeichnet, daß der Ligand ein biosynthetisches Bindeprotein ist.

25. Die Vorrichtung gemäß Anspruch 6, dadurch gekennzeichnet, daß besagter Ligand eine Bindungsstelle ist, die ein Molekül aus der Gruppe bestehend aus Immunglobulin G, Immunglobulin M, Immunglobulin A, Immunglobulin E, Gewebeplasminogen-Aktivator, menschlichem Interleucinprotein, Blutkoagulationsfaktor, menschlichem gonadotropem Chorlon (HCG), Thyreotropinhormon (TTH), carcinoembryonalem Antigen, a-Fetoprotein, transformieren-

dem Wachstumsfaktor und Interferon binden kann.

26. Die Vorrichtung gemäß Anspruch 6, dadurch gekennzeichnet, daß besagte Membran folgendes umfaßt: (a) Polyethersulfon als erster hydrophober Polymerbestandteil mit funktionalisierbaren Phenolkettenenden; (b) Hydroxyalkylcellulose mit funktionellen Hydroxyl-Gruppen; und (c) eine Linker-Hälfte, ausgewählt aus der Gruppe bestehend aus Ethylenglycol-diglycidylether, 1,4-Butandioldiglycidylether, Epichlorohydrin und Chlorethensäure, wobei diese Linker-Hälfte in der Lage ist, das Ende einer Phenolkette des besagten Polyethersulfons mit mindestens einer Hydroxylgruppe von besagter Hydroxyalkylcellulose kovalent zu überbrücken.

27. Die Vorrichtung gemäß den Ansprüchen 1 und 2, dadurch gekennzeichnet, daß besagte Membran mit einem Liganden derivatisiert wird, der ein einkettiges multifunktionales biosythetisches Protein umfaßt, das von einem einzigen Gen, gewonnen durch rekombinante DNA-Techniken, ausgedrückt wird und das folgendes umfaßt:

- eine biosynthetische Antikörperbindungssteile, die eine vorbestimmte Antigen-Determinante an sich binden kann und die mindestens einen Proteinabschnitt aufweist, wobei die Aminosäuresequenz in besagtem Abschnitt mindestens mit einem Teil der Sequenz eines variablen Abschnitts eines Immunglobulin-Moleküls, das die Fähigkeit besitzt, besagte vorbestimmte Antigendeterminante nebst darangeknüpftem Peptid an sich zu binden, homolog ist;
- ein Polypeptid-Effektorprotein mit mindestens einer funktionallen Gruppe, die zur selektiven kovalenten Bindung an besagte Membran in der Lage ist.

28. Die Vorrichtung gemäß Anspruch 27, dadurch gekennzeichnet, daß besagte Bindungsstelle mindestens zwei Abschnitte aufweist, die über Peptidbindungen mit einer Polypeptidbindungssequenz verknüpft sind.

29. Die Vorrichtung gemäß Anspruch 28, dadurch gekennzeichnet, daß besagte zwei Abschnitte einem $V_H$ und einem $V_L$ eines natürlichen Immunglobulins ähnlich sehen.

30. Die Vorrichtung gemäß Anspruch 28, dadurch gekennzeichnet, daß die Aminosäuresequenz jeder der besagten Abschnitte einen Satz von komplementär-bestimmenden Bereichen ("CDRs - complementarily determining regions"), eingebettet in einen Satz von einrahmenden Bereichen ("FRs - framing regions"), umfaßt, von denen jeder mit mindestens einem Teil der CDRs bzw. FRs aus besagtem variablen Abschnitt eines Immunglobulin-Moleküls, das die Fähigkeit besitzt, besagte vorbestimmte Antigendeterminante zu binden, homolog ist.

31. Die Vorrichtung gemäß Anspruch 28, dadurch gekennzeichnet, daß besagte Polypeptidbindungssequenz Aminosäuren beinhaltet, die zusammen eine unstrukturierte Polypeptid-Konfiguration bilden, die sich über eine Strecke von mindestens 40 A in wäßriger Lösung erstreckt.

32. Die Vorrichtung gemäß den Ansprüchen 1 und 2, dadurch gekennzeichnet, daß eine Vielzahl von Strängen von besagten Hohlfasermembranen vorliegen.

33. Die Vorrichtung gemäß Ansprüchen 1 und 2, dadurch gekennzeichnet, daß sie desweiteren eine zweite Einrichtung zum Einleiten eines zweiten Fluids in besagte einfassende Einrichtung auf die der Einleitungsseite des ersten Fluids gegenüberliegenden Seite von besagter Membran aufweist.

34. Die Vorrichtung nach Anspruch 33 dadurch gekennzeichnet, daß die besagte zweite Einrichtung zum Einleiten wahlweise eine reversible Pumpe ist, die in der Lage ist, ein Fluid abzuleiten oder den Ausgang von Fluid aus besagter einfassender Einrichtung zu regulieren.

35. Die Vorrichtung gemäß Anspruch 34, dadurch gekennzeichnet, daß besagte unter (d) genannte Ablaßeinrichtung geschlossen ist.

36. Die Vorrichtung gemäß den Ansprüchen 1 und 2, dadurch gekennzeichnet, daß der statische Betriebsdruck besagter Vorrichtung bis zu ca. 1,02 bar (15 psig) reicht.

37. Die Vorrichtung gemäß den Ansprüchen 1 und 2, dadurch gekennzeichnet, daß der Transmembrandruck besagter Vorrichtung bis zu ca. 0,68 bar (10 psig) reicht.

38. Die Vorrichtung gemäß den Ansprüchen 1 und 2, dadurch gekennzeichnet, daß die Temperatur des besagten ersten Fluids und des besagten zweiten Fluids zwischen ca. 2°C und ca. 37°C liegt.

**39.** Die Vorrichtung gemäß den Ansprüchen 1 und 2, dadurch gekennzeichnet, daß sie einen von besagter Einrichtung zur Einfassung der Membran eingeschlossenen Raum mit einem Fassungsvermögen von ca. 1 ml bis zu ca. 10 l aufweist.

**40.** Die Vorrichtung gemäß den Ansprüchen 1 und 2, dadurch gekennzeichnet, daß besagte Einrichtung zur Einfassung besagter Membran korrosionsbeständig ist.

**41.** Die Vorrichtung gemäß den Ansprüchen 1 und 2, dadurch gekennzeichnet, daß besagte Einleitungseinrichtung eine Pumpe ist.

**42.** Ein Verfahren zur Reinigung/Trennung eines Ligats in einem Membransystem mittels Affinität, gekennzeichnet durch die nachgenannten Verfahrensschritte:

> (a) Einleiten einer ligathaltigen Einsatzflüssigkeit auf eine porige Hohlfasermembran, die mit einem Liganden für besagtes Ligat gekoppelt ist, wobei besagte Einsatzflüssigkeit unter einem Druck steht, der ausreicht, um einen Teil der besagten Einsatzflüssigkeit zu veranlassen, konvektiv und tangential über eine Außenseite besagter Membran zu strömen und um den Rest der Flüssigkeit zu veranlassen, in besagte porige Membran einzuströmen und durch diese hindurchzuströmen, wobei sich das in besagter Einsatzflüssigkeit enthaltene Ligat an besagten Liganden anlagert und dadurch aus besagter Einsatzflüssigkeit isoliert wird;
> (b) Abwaschen besagter Membran mit einer Pufferlösung;
> (c) Einleiten einer Elutionslösung auf eine Seite besagter Membran unter einem Druck, der ausreichend hoch ist, um besagte Elutionslösung zu veranlassen, in besagte Membran einzuströmen und durch diese hindurchzuströmen und die Dissozlierung sämtlicher in Schritt (a) gebildeter Ligat-Ligand-Verbindungen zu bewirken, wobei jedes an besagten Liganden gebundene Ligat mittels besagter Elutionslösung eluiert wird; und
> (d) Isolieren von besagtem Ligat in gereinigter Form aus besagter Elutionslösung;

> wobei besagte Membran durch und durch und in alle Richtungen eine im wesentlichen isotrope feinporige Struktur aufweist mit Poren, die groß genug sind, um die Konvektionsströmung von Makromoleküle enthaltenden Lösungen durch die Membran zu ermöglichen.

**43.** Das Verfahren gemäß Anspruch 42, dadurch gekennzeichnet, daß besagte Elutionslösung auf der der Einleitungsseite der Einsatzflüssigkeit gegenüberliegenden Seite von besagter Membran eingeleitet wird, wobei die Einspeisung unter ausreichend hohem Druck erfolgt, um besagte Lösung zu veranlassen, in die Membran einzuströmen und durch diese hindurchzuströmen.

**44.** Das Verfahren gemäß Anspruch 42 oder 43, dadurch gekennzeichnet, daß besagte Pufferlösung auf der der Einleitungsseite der Einsatzflüssigkeit gegenüberliegenden Seite von besagter Membran eingeleitet wird, wobei die Einspeisung unter ausreichend hohem Druck erfolgt, um besagte Lösung zu veranlassen, in die Membran einzuströmen und durch diese hindurchzuströmen.

**45.** Das Verfahren gemäß einem der Ansprüche 42 bis 44, dadurch gekennzeichnet, daß besagtes Ligat aus der Gruppe bestehend aus IgG, Faktor VIII, Fibronectin und Faktor IX und besagter Ligand aus der Gruppe bestehend aus Protein A, einem monokonalem Antikörper zu Faktor VIII, Gelatine vom Schwein und einem monoklonalem Antikörper zu Faktor IX ausgewählt wird.

**Revendications**

**1.** Un appareil permettant de mettre en oeuvre une séparation par affinité comprenant :

> a) une membrane poreuse formée de fibres creuses associées à un ligand;
> b) des moyens pour enclore ladite membrane poreuse formée de fibres creuses;
> c) des moyens pour amener un premier fluide au contact intime d'une face de ladite membrane poreuse, formée de fibres creuses, ainsi enclose; et
> d) des moyens de sortie pour envoyer vers un récipient tout fluide présent d'un côté de ladite membrane poreuse, formée de fibres creuses, enclose opposé au côté sur lequel le premier fluide est dans un premier temps amené par les moyens mentionnés en c);

> caractérisé en ce qu'il comprend en outre:

e) des seconds moyens de sortie pour diriger ledit premier fluide dans un second récipient,

et en ce qu'au moins une partie dudit fluide quittant les moyens d) provient dudit premier fluide amené par les moyens c), et ce que en outre ladite membrane présente une structure microporeuse sensiblement isotrope selon toutes les directions sur l'ensemble de la membrane, et comporte des pores suffisamment larges pour permettre l'écoulement convectif, à travers la membrane, des solutions contenant les macromolécules.

2. L'appareil selon la revendication 1, caractérisé en ce que ladite membrane présente une dimension moyenne de pores d'au moins 0,20 $\mu$m.

3. L'appareil selon la revendication 1 ou 2, caractérisé en ce que ladite membrane a une épaisseur d'au moins environ 200 $\mu$m.

4. L'appareil selon l'une quelconque des revendications 1 à 3, caractérisé en ce que ladite membrane est une membrane hydrophobe comportant une surface interfaciale substituée.

5. L'appareil selon la revendication 4, caractérisé en ce que ladite surface interfaciale de la membrane est substituée par un élément de liaison lié à ladite surface et à un ligand comportant une pluralité de groupes fonctionnels, de façon à obtenir une membrane présentant des caractéristiques de surfaces interfaciales substituées.

6. L'appareil selon l'une quelconque des revendications 1 à 3, caractérisé en ce que ladite membrane comprend :

a) un polymère hydrophobe comportant exposées à la surface du polymère des extrémités de chaînes pouvant être fonctionnalisées;
b) une macromolécule et/ou un ligand capable de modifier les propriétés de surface dudit polymère hydrophobe;
c) un élément de liaison pontant par liaison covalente une extrémité de chaîne dudit polymère hydrophobe à ladite macromolécule et/ou audit ligand.

7. L'appareil selon la revendication 6, caractérisé en ce que ledit polymère hydrophobe est sélectionné dans le groupe consistant en polysulfones, polyéthersulfones, polyimides, polyoxyde d'arylène, polyuréthane, polyétheréthercétones, polycarbonates, polyesters, polyhalogénures de vinyle et polyhalogénures de polyvinylidène, leurs dérivés, leurs mélanges et les copolymères en dérivant.

8. L'appareil selon la revendication 6 ou 7, caractérisé en ce que l'élément de liaison est une molécule comportant un groupe de base et au moins deux groupes fonctionnels sélectionnés dans le groupe consistant en époxyde, carbonyle, carboxyle, amine, halogénure, chlorure d'acide, anhydride d'acide, sulfonyle, chlorure de sulfonyle, acide carboxylique, hydroxyle, et les combinaisons stables de ces groupes.

9. L'appareil selon la revendication 8, caractérisé en ce que ledit groupe de base de la molécule de l'élément de liaison est une molécule hydrocarbonée.

10. L'appareil selon l'une quelconque des revendications 6 à 9, caractérisé en ce que ledit élément de liaison comporte un groupe fonctionnel sélectionné dans le groupe consistant en silicium, aluminium, étain, bore, phosphore, soufre et azote.

11. L'appareil selon la revendication 6, caractérisé en ce que ledit élément de liaison présente un poids moléculaire inférieur à environ 2000.

12. L'appareil selon la revendication 6, caractérisé en ce que ladite macromolécule est hydrophile.

13. L'appareil selon la revendication 6, caractérisé en ce que ladite macromolécule est hydrophobe.

14. L'appareil selon la revendication 6, caractérisé en ce que ladite macromolécule est amphotère.

15. L'appareil selon la revendication 6, caractérisé en ce que ladite macromolécule comporte un groupe fonctionnel capable de porter une charge.

16. L'appareil selon la revendication 6, caractérisé en ce que ladite macromolécule comporte un groupe fonctionnel non-ionique.

17. L'appareil selon la revendication 6, caractérisé en ce que ladite macromolécule correspond à un site de liaison biosynthétique pour un anticorps.

18. L'appareil selon la revendication 6, caractérisé en ce que ladite macromolécule est sélectionnée dans le groupe consistant en polymères naturels, polymères de synthèse, dérivés, mélanges, et les copolymères en dérivant; tensioactif, hydrate de carbone, lectine, polypeptide, polysaccharide, liposome, protéine, glycoprotéine, oligonucléotide, colorant de synthèse, colorant naturel, polynucléotide, leurs dérivés et leurs mélanges; anticorps monoclonal, anticorps polyclonal, antigène, récepteur, substrat d'une enzyme, enzyme, protéine de transport, facteur de coagulation, co-facteur, inhibiteur, hormone, immunoglobuline, histone, plasmide, leurs dérivés et leurs mélanges; polysilane, polysiloxane, polysulfonate, polycarboxylate, hydroxyalkylcellulose, dextrane, diéthylaminoéthyldextrane, carboxyméthylcellulose, polyéthylène imine, polycarboxyméthyléthylène imine, polyalcool vinylique, leurs dérivés, leurs mélanges, et les copolymères en dérivant.

19. L'appareil selon la revendication 6, caractérisé en ce que ladite macromolécule présente un poids moléculaire moyen d'environ 0,2 à environ 5000 kilodaltons.

20. L'appareil selon la revendication 6, caractérisé en ce que ledit polymère de membrane comprend de plus des couches multiples de macromolécules, lesdites couches étant maintenues entre elles par des liaisons covalentes.

21. L'appareil selon la revendication 6, caractérisé en ce que ledit ligand est lié par liaison covalente à la ou lesdites macromolécule(s).

22. L'appareil selon la revendication 6, caractérisé en ce que ledit ligand comporte un groupe fonctionnel ionisable.

23. L'appareil selon la revendication 6, caractérisé en ce que ledit ligand est sélectionné dans le groupe consistant en silane hydrocarboné aliphatique à chaîne longue, silane hydrocarboné aliphatique ramifié, silane hydrocarboné aromatique et leurs mélanges; tensioactif, hydrate de carbone, lectine, polypeptide, monosaccharide, polysaccharide, liposome, protéine et glycoprotéine, oligonucléotide, colorant de synthèse, colorant naturel, polynucléotide, leurs dérivés et leurs mélanges; anticorps monoclonal, anticorps polyclonal, antigène, récepteur, enzyme, substrat d'une enzyme, protéine de transport, glycoprotéine, facteur de coagulation, cofacteur, inhibiteur, hormone, immunoglobuline, histone, plasmide, leurs dérivés et leurs mélanges, ainsi que la protéine A naturelle, la protéine A recombinante, l'avidine, la biotine, l'héparine, la lectine, les récepteurs de surface des cellules animales, les récepteurs de surface des cellules végétales, les récepteurs de surface des cellules bactériennes et les acides nucléiques en brins simples.

24. L'appareil selon la revendication 6, caractérisé en ce que le ligand est une protéine de liaison biosynthétique.

25. L'appareil selon la revendication 6, caractérisé en ce que ledit ligand est un site de liaison capable de lier une molécule sélectionnée dans le groupe consistant en immunoglobuline G, imunoglobuline M, imunoglobuline A, imunoglobuline E, activateur de plasminogène tissulaire, interleukine humaine, facteur de coagulation sanguin, gonadotropine chorionique humaine, thyrotropine, l'antigène carcinoembryonique, a-fétoprotéine, le facteur de croissance modifiant et l'interféron.

26. L'appareil selon la revendication 6, caractérisé en ce que ladite membrane comprend :

   a) en tant que composant polymère hydrophobe principal une polyéthersulfone comportant des extrémités de chaînes phénoliques fonctionnalisables;
   b) de l'hydroxyalkylcellulose comportant des groupes fonctionnels hydroxyles; et
   c) un élément de liaison sélectionné dans le groupe consistant en éther diglycidylique d'éthylène glycol, éther diglycidylique de 1,4-butanediol, épichlorohydrine et acide chloroacétique, lequel élément de liaison est capable de ponter par liaison covalente l'extrémité de la chaîne phénolique de ladite polyéthersulfone avec au moins un groupe hydroxyle de ladite hydroxyalkylcellulose.

27. L'appareil selon les revendications 1 et 2, caractérisé en ce que ladite membrane est substituée par un ligand comprenant une protéine biosynthétique multifonctionnelle comportant une chaîne unique qui a été exprimée à partir d'un gène unique par des techniques d'ADN recombinant, ladite protéine comprenant:

- un site de liaison biosynthétique pour un anticorps capable de lier à un déterminant antigénique présélectionné et comprenant au moins un domaine protéinique, la séquence d'aminoacide dudit domaine protéique étant homologue à au moins une partie de la séquence de la région variable d'une molécule d'immunoglobuline capable de lier ledit déterminant antigénique présélectionné et le peptide qui y est lié;
- une protéine effectrice polypeptidique comportant au moins un groupe fonctionnel capable de se lier sélectivement de façon covalente à ladite membrane.

28. L'appareil selon la revendication 27, caractérisé en ce que ledit site de liaison comprend au moins deux domaines connectés par des agents peptidiques à un raccord de séquence polypeptidique.

29. L'appareil selon la revendication 28, caractérisé en ce que les deux domaines miment un domaine $V_H$ et un domaine $V_L$ d'une immunoglobuline naturelle.

30. L'appareil selon la revendication 28, caractérisé en ce que la séquence aminoacide de chacun desdits domaines comprend une série de régions déterminantes complémentaires ("CDRs") interposées entre une série de régions cadres ("FRs"), chacune desquelles étant respectivement homologue à au moins une partie des régions CDRs et FRs provenant de ladite région variable d'une molécule d'immunoglobuline capable de se lier audit déterminant antigénique présélectionné.

31. L'appareil selon la revendication 28, caractérisé en ce que ladite séquence polypeptidique de raccord comprend des acides aminés qui forment ensemble une configuration polypeptidique non structurée recouvrant une distance d'au moins 40 Å en solution aqueuse.

32. L'appareil selon les revendications 1 et 2, caractérisé en ce que de multiples brins de ladite membrane en fibres creuses sont présentes.

33. L'appareil selon les revendications 1 et 2 caractérisé en ce que ledit appareil comprend de plus des seconds moyens pour fournir un second fluide dans lesdits moyens pour enclore au niveau du côté de ladite membrane opposé à celui où le premier fluide est fourni.

34. L'appareil selon la revendication 33 caractérisé en ce que lesdits seconds moyens sont alternativement une pompe réversible capable de soutirer un fluide ou de réguler la sortie du fluide desdits moyens pour enclore.

35. L'appareil selon la revendication 34, caractérisé en ce que les moyens de sortie de l'élément (d) sont clos.

36. L'appareil selon les revendications 1 et 2, caractérisé en ce que la pression statique de fonctionnement dudit appareil peut atteindre environ 1,02 bars (15 psig).

37. L'appareil selon les revendications 1 et 2, caractérisé en ce que la pression transmembranaire dudit appareil peut atteindre environ 0,68 bars (10 psig).

38. L'appareil selon les revendications 1 et 2, caractérisé en ce que la température desdits premier et second fluides est comprise entre environ 2°C et environs 37°C.

39. L'appareil selon les revendications 1 et 2, caractérisé en ce que ledit appareil comporte un volume à l'intérieur desdits moyens pour enclore permettant de contenir ladite membrane d'environ compris entre 1 ml et environ 10 l.

40. L'appareil selon les revendications 1 et 2, caractérisé en ce que lesdits moyens pour enclore ladite membrane sont résistants à la corrosion

41. L'appareil selon les revendications 1 et 2, caractérisé en ce que lesdits moyens d'alimentation consistent en une pompe.

42. Un procédé pour mettre en oeuvre une purification par affinité d'une molécule dans un système de membrane comprenant :

(a) la mise en contact d'une charge liquide contenant la molécule avec une membrane poreuse formée de fibres creuses liées à un ligand à ladite molécule, ladite charge liquide étant sous une pression suffisante pour qu'une partie de ladite charge liquide s'écoule de façon convective et tangentiellement à travers une surface extérieure de ladite membrane de façon que le reste dudit fluide s'écoule dans et au travers de ladite membrane poreuse et que ladite molécule présente dans ladite charge liquide se lie audit ligand et se sépare ainsi de ladite charge liquide;

(b) le lavage de ladite membrane avec une solution tampon;

(c) la mise en contact d'une solution d'élution avec une face de ladite membrane sous une pression suffisante pour que ladite solution d'élution s'écoule dans et au travers de ladite membrane et produise la dissociation de toutes les liaisons ligand-molécule qui se sont formées dans l'étape (a) de façon à ce que toute molécule liée audit ligand soit éluée par la solution d'élution; et

(d) la séparation de ladite molécule sous une forme purifiée à partir de ladite solution d'élution;

ladite membrane présentant une structure microporeuse sensiblement isotrope selon toutes les directions sur l'ensemble de la membrane avec des pores suffisamment larges pour permettre l'écoulement convectif, à travers la membrane, des solutions contenant les macromolécules.

43. Le procédé selon la revendication 42, caractérisé en ce que ladite solution d'élution est amenée du coté de ladite membrane opposé à celui auquel la charge liquide est amenée, sous une pression suffisante pour permettre à ladite solution de passer dans et au travers de ladite membrane.

44. Le procédé selon la revendication 42 ou 43, caractérisé en ce que ladite solution tampon est amenée du coté de ladite membrane opposé à celui auquel la charge liquide est amenée, sous une pression suffisante pour permettre à ladite solution de passer dans et au travers de ladite membrane.

45. Le procédé selon l'une quelconque des revendications 42 à 44, caractérisé en ce que ladite molécule est sélectionnée dans un groupe consistant en IgG, facteur VIII, fibronectine, et facteur IX, et en ce que ledit ligand est respectivement sélectionné dans le groupe consistant en protéine A, un anticorps monoclonal dirigé contre le facteur VIII, la gélatine de porc et un anticorps monoclonal dirigé contre le facteur IX.

FIGURE 1

WASH BUFFER

ELUTE BUFFER

REGENERATE BUFFER

V1  V2  V3

V4  UV DETECTER  RETENTATE PRESSURE

FILTRATE

V5

UV DETECTER  AFFINITY MODULES

V6

SHELL DRAIN

BUFFER/ FILTRATE PUMP

FILTRATE DRAIN

FEED PRESSURE

FEED PUMP

V7

LUMEN DRAIN

V8  V9

SUPERNATANT RESERVOIR

RECIRCULATION RESERVOIR

PRODUCT RESERVOIR

OPTIONAL COMPONENTS

# FIG. 2

GP-PRESSURE GAUGE
P-PUMP
PR-PRESSURE REGULATOR
TL-LEVEL TRANSDUCER GAUGE
V-VALVE
TP-PRESSURE GAUGE
TT-TEMPERATURE GAUGE
TUV-ULTRA VIOLET
VPR-PRESSURE RELIEF VALVE

EP 0 483 143 B2

Figure 3. Hollow-Fiber Membrane Affinity Separation Steps

1:
LOAD
**** - filtrate on shell side/recirculated to feed stream    can be
      to reclaim unbound Product, if necessary

BROTH
RECYCLE!

2:
Wash - conducted from shell side to efficiently remove
       potential product contaminants from the interstices
       of the fibers.

STEP#1

STEP#2

3:
Elute - conducted from the shell side to prevent product
        losses due to dead volume effects.

STEP#1

STEP#2

4.
REGENERATE - Operated in the regular cross-flow filtration
             mode.

FIGURE 4.

Figure 5 Translumenal:Transmembrane Pressure Drop
vs. Effective Fiber Length.

Effective Length (cm)

| | ID | WALL THICKNESS |
|---|---|---|
| ——— | 1000 | x 50 µm |
| — — — — | 1000 | x 100 µm |
| — — | 1000 | x 200 µm |
| —·—· | 1000 | x 300 µm |
| __ __ .. | 1000 | x 400 µm |

**Figure 6**     Experimental setup for affinity membrane mediated purification of FN from blood plasma.

EP 0 483 143 B2

Figure 7 Purification of FN from blood plasma by membrane-mediated affinity process. Plasma was passed through a 0.34 cm³ membrane disk at 1.0 mL/min. (o) Absorbance at 280 nm; (o) concentration of FN.

Fig 8.

# Protein A Membrane-Mediated Purification of Monoclonal Antibody
## From Cell Culture Supernatant
## Experiment Performed at β-Test Facility

Fig 9.

| | Per Cycle | Total | | Per Cycle | Total | | |
|---|---|---|---|---|---|---|---|
| Volume Processed (mL) | 100 | 2000 | MAb Recoverd (mg) | 3.2 | 64 | Membrane Matrix Volume: | 0.5 mL |
| MAb Processed (mg) | 3.9 | 78 | Time Elapsed (Hr) | 0.45 | 9.0 | Feed MAb Concentration: | 39 mg/l |
| | | | | | | No. of Cycles: | 20 |

Cloud Point Phase Diagram For PES/PEO Blend
Solutions; 9% PES (7.5% PEO ~~~~~~ (4,000 kD)):
X % Glycerin in NMP.

Figure 10

Dope

1

2

Intraannular Fluid

Extraannular Fluid

3

4

5

Double Annular
Co-Extrusion Spinnerette

Figure 11.

ALTERNATIVE DESIGN FOR DOUBLE ANNULAR CO-EXTRUSION SPINNERETTE

Figure 12.

Lp as a Function of Extraannular Flow Rate

Figure 13

EP 0 483 143 B2

Cleavage
Site

CDR

Linker

Framework

Spacer

$V_H$

$V_L$

Leader

Effector
Molecule

FIGURE 14

```
        10        20        30        40        50        60        70
GAATTCATGGCTGACAACAAATTCAACAAGGAACAGCAGAACGCGTTCTACGAGATCTTGCACCTGCCGAACCTG
 E  F  M  A  D  N  K  F  N  K  E  Q  Q  N  A  F  Y  E  I  L  H  L  P  N  L
EcoRI                             MluI         BglII     BspMI+
                                  XmnI
          FB →

        85        95       105       115       125       135       145
AACGAAGAGCAGCGTAACGGCTTCATCCAAAGCTTGAAAGACGACCCGTCTCAGAGCGCTAACCTGCTGGCAGAG
 N  E  E  Q  R  N  G  F  I  Q  S  L  K  D  D  P  S  Q  S  A  N  L  L  A  E
                       HindIII                              BspMI+
                                                           Eco47III

        160       170       180       190       200       210       220
GCCAAGAAACTGAACGACGCTCAGGCGCCGAAGAGTGATCCCGAAGTTCAACTGCAGCAGTCTGGTCCTGAATTG
 A  K  K  L  N  D  A  Q  A  P  K  S  D  P  E  V  Q  L  Q  Q  S  G  P  E  L
                              NarI                  PstI
      ├── SPACER ───── ─────────── ──→ VH

        235       245       255       265       275       285       295
GTTAAACCTGGCGCCTCTGTGCGCATGTCCTGCAAATCCTCTGGGTACATTTTCACCGACTTCTACATGAATTGG
 V  K  P  G  A  S  V  R  M  S  C  K  S  S  G  Y  I  F  T  D  F  Y  M  N  W
      NarI         FspI

        310       320       330       340       350       360       370
GTTCGCCAGTCTCATGGTAAGTCTCTAGACTACATCGGGTACATTTCCCCATACTCTGGGGTTACCGGCTACAAC
 V  R  Q  S  H  G  K  S  L  D  Y  I  G  Y  I  S  P  Y  S  G  V  T  G  Y  N
      BstXI             XbaI                     PflMI         BstEII

        385       395       405       415       425       435       445
CAGAAGTTTAAAGGTAAGGCGACCCTTACTGTCGACAAATCTTCCTCAACTGCTTACATGGAGCTGCGTTCTTTG
 Q  K  F  K  G  K  A  T  L  T  V  D  K  S  S  S  T  A  Y  M  E  L  R  S  L
      DraI                       SalI

        460       470       480       490       500       510       520
ACCTCTGAGGACTCCGCGGTATACTATTGCGCGGGCTCCTCTGGTAACAAATGGGCCATGGATTATTGGGGTCAT
 T  S  E  D  S  A  V  Y  Y  C  A  G  S  S  G  N  K  W  A  M  D  Y  W  G  H
            SacII                                          NcoI

        535       545       555       565       575       585       595
GGTGCTAGCGTTACTGTGAGCTCTGGTGGCGGTGGGTCGGGCGGTGGTGGCTCGGGTGGCGGCGGATCCGACGTC
 G  A  S  V  T  V  S  S  G  G  G  G  S  G  G  G  G  S  G  G  G  G  S  D  V
NheI              SacI
                        ├── CONNECTOR REGION ─────── BamHI,AatII →VL

        610       620       630       640       650       660       670
GTTGTTACCCAGACTCCGCTGTCTCTGCCGGTTTCTCTGGGTGACCAGGCTTCTATTTCTTGCCGCTCTTCCCAG
 V  V  T  Q  T  P  L  S  L  P  V  S  L  G  D  Q  A  S  I  S  C  R  S  S  Q
                           BstEII                              PflM

        685       695       705       715       725       735       745
TCTCTGGTCCATTCTAATGGTAACACTTACCTGAACTGGTACCTGCAAAAGGCTGGTCAGTCTCCGAAGCTTCTG
I S  L  V  H  S  N  G  N  T  Y  L  N  W  Y  L  Q  K  A  G  Q  S  P  K  L  L
      BstXI                           BspMI+                    HindIII
                                      KpnI

        760       770       780       790       800       810       820
ATCTACAAAGTCTCTAACCGCTTCTCTGGTGTCCCGGATCGTTTCTCTGGTTCTGGTTCTGGTACTGACTTCACC
 I  Y  K  V  S  N  R  F  S  G  V  P  D  R  F  S  G  S  G  S  G  T  D  F  T

        835       845       855       865       875       885       895
CTGAAGATCTCTCGTGTCGAGGCCGAAGACCTGGGTATCTACTTCTGCTCTCAGACTACTCATGTACCGCCGACT
 L  K  I  S  R  V  E  A  E  D  L  G  I  Y  F  C  S  Q  T  T  H  V  P  P  T
      BglII

        910       920       930       940
TTTGGTGGTGGCACCAAGCTCGAGATTAAACGTTAACTGCAG
 F  G  G  G  T  K  L  E  I  K  R  *
                 XhoI          HpaI PstI
```

FIGURE 15

65

FIGURE 16A

Rabbit IgG
Protein A
FB
Mouse IgG2a
Mouse IgG1

FIGURE 16B